(19) Europäisches Patentamt
European Patent Office
Office européen des brevets

(11) EP 4 124 361 B1

(12) FASCICULE DE BREVET EUROPEEN

(45) Date de publication et mention de la délivrance du brevet:
**31.01.2024 Bulletin 2024/05**

(21) Numéro de dépôt: **21306070.0**

(22) Date de dépôt: **30.07.2021**

(51) Classification Internationale des Brevets (IPC):
***A61P 35/00*** *(2006.01)* ***A61K 31/439*** *(2006.01)*
***C07D 471/08*** *(2006.01)* ***C07D 471/18*** *(2006.01)*

(52) Classification Coopérative des Brevets (CPC):
**C07D 471/08; A61P 35/00; C07D 471/18**

(54) **LIGANDS MACROCYCLIQUES AVEC GROUPEMENT(S) PICOLINATE(S), LEURS COMPLEXES AINSI QUE LEURS UTILISATIONS MEDICALES**

MAKROZYKLISCHE LIGANDEN MIT PICOLINATGRUPPE(N), IHRE KOMPLEXVERBINDUNGEN UND MEDIZINISCHE VERWENDUNGEN

MACROCYCLIC LIGANDS WITH PICOLINATE GROUP(S), COMPLEXES THEREOF AND MEDICAL USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**01.02.2023 Bulletin 2023/05**

(73) Titulaire: **Guerbet**
**93420 Villepinte (FR)**

(72) Inventeurs:
- **ROUSSEAUX, Olivier**
  **60300 SENLIS (FR)**
- **PAULY - BATARD, William**
  **77410 MESSY (FR)**
- **FOUGERE, Olivier**
  **60128 MORTEFONTAINE (FR)**
- **CATOEN, Sarah**
  **93190 LIVRY GARGAN (FR)**

(74) Mandataire: **Lavoix**
**2, place d'Estienne d'Orves**
**75441 Paris Cedex 09 (FR)**

(56) Documents cités:
**WO-A1-2017/109217 WO-A1-2019/002505**
**WO-A1-2019/185901**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Processed by Luminess, 75001 PARIS (FR)

## Description

La présente invention concerne de nouveaux ligands macrocycliques ainsi que leurs complexes, notamment radioactifs, et leurs utilisations en imagerie médicale et/ou en thérapie, notamment en radiologie interventionnelle.

La présente invention concerne également un nouveau procédé de préparation des ligands tels que selon l'invention, ainsi que leurs intermédiaires de préparation.

Le besoin de traitements ciblés et personnalisés en oncologie conduit à développer des stratégies thérapeutiques nouvelles basées sur des outils de détection précoces associés à des traitements vectorisés plus spécifiques et plus efficaces.

La radiologie interventionnelle est un axe très prometteur de la médecine individualisée. Elle permet de combiner dans une même séquence un diagnostic précis de la lésion ou tumeur et/ou son traitement instantané, guidé et contrôlé par l'image. Elle est décrite comme une chirurgie minimalement invasive et peut être de ce fait réalisée en ambulatoire, ce qui permet d'économiser de nombreuses et onéreuses journées d'hospitalisation pour une efficacité souvent comparable à la chirurgie conventionnelle. La radiologie interventionnelle peut donc représenter une alternative ou un complément au traitement chirurgical conventionnel.

La radiologie interventionnelle permet d'accéder à une lésion ou tumeur située à l'intérieur de l'organisme pour effectuer un acte diagnostique (prélèvement par exemple) ou thérapeutique. L'imagerie par fluoroscopie, échographie, scanner ou IRM permet un repérage, un guidage et un contrôle optimal du geste médical.

Il existe donc un besoin pour de nouvelles molécules utilisables en imagerie médicale et/ou en thérapie, en particulier en radiologie interventionnelle. Plus particulièrement, il existe un besoin pour des ligands permettant de complexer des éléments chimiques, en particulier des métaux, de façon à obtenir des complexes utilisables en imagerie médicale et/ou en thérapie, en particulier en radiologie interventionnelle.

De tels ligands doivent notamment être stables dans le sérum humain et complexer de façon suffisamment forte les métaux pour que ceux-ci atteignent leur cible et ne diffusent pas dans d'autres organes ou tissus sensibles comme les os, les poumons et les reins.

La présente invention a pour but de fournir de nouveaux ligands permettant de complexer des éléments chimiques, en particulier des radioéléments.

La présente invention a également pour but de fournir de nouveaux complexes, en particulier des complexes radioactifs.

La présente invention a pour but de fournir des ligands et/ou des complexes particulièrement utiles en imagerie médicale et/ou en thérapie, notamment dans le traitement des cancers.

La présente invention a également pour but de fournir une composition pharmaceutique comprenant des complexes permettant l'imagerie médicale, le ciblage et/ou le traitement de cancers.

La présente invention a pour but de fournir un nouveau procédé de préparation de ces ligands.

En partant des travaux décrits dans WO 2017/109217 et dans Le Fur et al. (Inorganic Chemistry Volume : 57 Issue : 4 Pages : 2051-2063 2018), les inventeurs ont développé de nouveaux ligands de grande affinité pour certains métaux notamment les terres rares, des complexes très stables et présentant des inerties cinétiques importantes. De plus, ces complexes peuvent être facilement radiomarqués et présentent alors un rendement radiochimique et une pureté radiochimique satisfaisants. Ces complexes peuvent également être incorporés dans une huile iodée de façon stable et avec une bonne reproductibilité, et présentent ainsi un profil de biodistribution permettant leur usage dans le traitement du cancer.

La présente invention concerne un composé de formule générale (I) suivante :

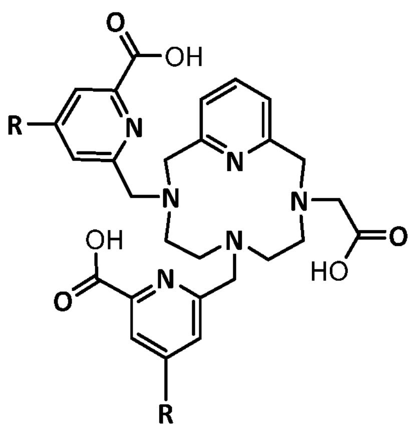

(I)

dans laquelle R est un groupement de formule (II) suivante:

-CΞC-Ph-L1-(CH2)n-L2 (II)

avec

- L1 représentant Ph ou -CΞC- ou CH2,
- L2 représentant H ou Ph ou alkylphényle,
- n compris entre 4 et 12,

ou l'un de ses sels pharmaceutiquement acceptables.

Selon un mode de réalisation préféré, la présente invention concerne un composé de formule générale (I) dans laquelle le groupement R est choisi parmi :

- le groupe R dans lequel L1=$CH_2$, n=7 et L2 =H, ce qui correspond à la structure suivante :

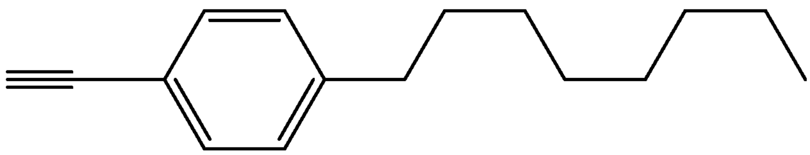

- le groupe R dans lequel L1= Ph, n=8 et L2 =H, ce qui correspond à la structure suivante :

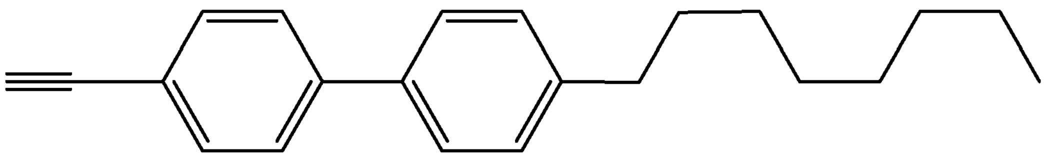

- le groupe R dans lequel L1= -CΞC-, n=8 et L2= Ph, ce qui correspond à la structure suivante :

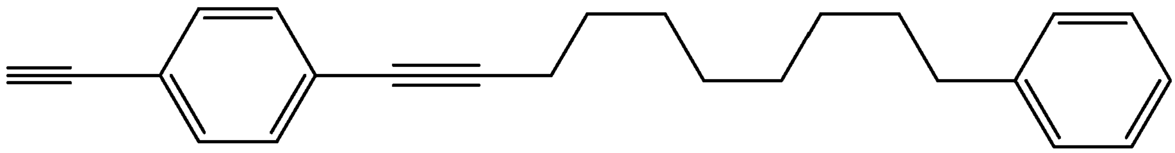

et l'un de ses sels pharmaceutiquement acceptables.

Les inventeurs ont mis au point de nouveaux complexes ligands-métal (complexes appelés également chélates) à partir du macrocycle pyclène (3,6,9,15-tétraazabicyclo[9.3.1]pentadeca-1(15),11,13-triène), diversement substitué par des groupements acétate et/ou picolinate (acide méthylène-6-pyridine-2-carboxylique). Le macrocycle pyclène est de formule suivante :

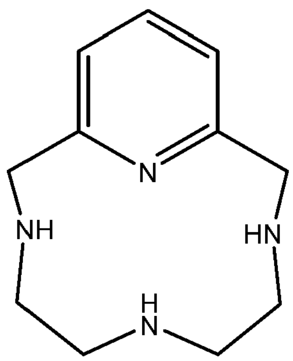

Pyclène

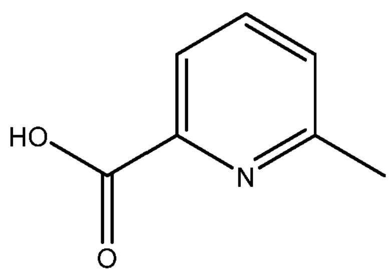

Picolinate

Comme les complexes décrits dans WO 2017/109217, les complexes selon l'invention présentent une bonne stabilité thermodynamique ainsi qu'une bonne inertie cinétique. Ils peuvent être aussi solubilisés dans une huile iodée telle que le Lipiodol®, une huile iodée fabriquée et commercialisée par la société Guerbet et qui est constituée par des esters éthyliques d'acides gras iodés de l'huile d'oeillette. Ainsi, les complexes selon l'invention solubilisés dans une huile iodée telle que le Lipiodol® peuvent être vectorisés notamment vers le foie et peuvent permettre de visualiser et/ou traiter les cancers, par exemple les cancers du foie.

Ces complexes présentent également un bon rendement d'extraction dans une huile iodée telle que le Lipiodol®. Ils présentent en particulier une bonne incorporation de la radioactivité (rendement radiochimique) dans une huile iodée telle que le Lipiodol® et une bonne stabilité de la solution radioactive du Lipiodol® lors de tests *in vitro.*

En particulier, la combinaison des propriétés de vectorisation du Lipiodol®, d'efficacité thérapeutique des radioéléments, et la bonne tolérance de ces produits permettent de proposer un traitement thérapeutique des cancers sûr et plus facile à mettre en oeuvre.

La vectorisation des complexes selon l'invention par une huile iodée telle que le Lipiodol® permet notamment d'éviter une mauvaise délivrance des complexes et diminue ainsi le risque d'effets indésirables dans les organes sains, en particulier le foie sain ou dans les organes extra-hépatiques, et permet d'atteindre la dose de radioactivité efficace dans la tumeur.

Plus particulièrement, cette vectorisation facilite le travail du radiologue interventionnel au moment de l'injection des complexes selon l'invention. Par exemple, lors d'une injection intra-artérielle suivie sous fluoroscopie, le geste du radiologue sera plus précis et plus sûr en permettant un ajustement de la vitesse de délivrance des complexes en fonction de la capture par la tumeur des complexes selon l'invention.

**Définitions**

On entend par « ligand », un composé capable de complexer un élément chimique tel qu'un métal, de préférence un radioélément. Selon un mode de réalisation, les ligands au sens de l'invention sont sous forme anionique et peuvent complexer des radioéléments sous forme cationique, par exemple des cations métalliques au degré d'oxydation (III). Selon la présente invention, les composés de formule (I) sont des ligands.

On entend par « radioélément », tout radioisotope connu d'un élément chimique, qu'il soit naturel ou produit artificiellement. Selon un mode de réalisation, le radioélément est choisi parmi les radioisotopes de l'yttrium, de l'actinium, du cuivre, du gallium, de l'indium, du scandium et des lanthanides. Par « lanthanides » sont désignés les atomes choisis parmi le groupe constitué de : La, Ce, Pr, Nd, Pm, Sm, Eu, Gd, Tb, Dy, Ho, Er, Tm, Yb et Lu.

On entend par « alkylphényle », un radical alkyle linéaire ou ramifié, de préférence comprenant de 1 à 20 atomes de carbone, de préférence de 1 à 8 atomes de carbone, lié à un phényle. De préférence, l'alkylphényle est un radical octylphényle.

On entend par « complexe », l'association d'un ligand tel que défini ci-dessus avec un élément chimique, de préférence un radioélément tel que défini ci-dessus. Le terme « complexe » étant synonyme de « chélate ».

La « stabilité thermodynamique » représente l'affinité du ligand pour un élément donné, en particulier un métal donné. Il s'agit de la constante d'équilibre de la réaction suivante

$$\text{Métal} + \text{Ligand} \rightleftarrows \text{Complexe}$$

dont les expressions mathématiques sont les suivantes :

Constante de dissociation

$$Kd = \frac{[Métal] \times [Ligand]}{[Complexe]}$$

Constante d'association

$$Ka = \frac{[Complexe]}{[Métal] \times [Ligand]}$$

Les valeurs sont en général exprimées sous forme de logarithme décimal logka ou -log de Kd. Selon un mode de réalisation, les complexes selon l'invention sont de forte affinité. Selon un mode de réalisation, les complexes selon l'invention ont une constante thermodynamique d'équilibre au moins égale à 16 (LogKa au moins égal à 16).

Les complexes formés selon la réaction d'équilibre décrite ci-dessus sont susceptibles de se dissocier sous

l'action de différents facteurs (pH, présence de métaux ou ligands compétiteurs). Cette dissociation peut avoir des conséquences importantes dans le cadre de l'utilisation des complexes en médecine humaine car elle entraine une libération du métal dans l'organisme. Afin de limiter ce risque, des complexes à dissociation lente sont recherchés, c'est-à-dire des complexes ayant une bonne inertie cinétique. L'inertie cinétique peut être déterminée par des tests de dissociation en milieu acide. Ces expériences conduisent à la détermination pour chaque complexe d'un temps de demi-vie ($T_{1/2}$) dans des conditions définies.

Dans le contexte de l'invention, le terme « traiter », « traitement » ou « traitement thérapeutique » signifie inverser, soulager, inhiber la progression de, du trouble ou l'affection auquel ce terme est applicable, ou un ou plusieurs symptômes d'un tel trouble.

Le terme « imagerie médicale » désigne les moyens d'acquisition et de restitution d'images du corps humain ou animal à partir de différents phénomènes physiques tels que l'absorption ou l'émission de photons (visible, infrarouge, rayons X, rayons gamma) la résonance magnétique nucléaire, la réflexion d'ondes ultrasons ou la radioactivité. Selon un mode de réalisation, le terme « imagerie médicale » se réfère à l'imagerie par rayons X, l'IRM (Imagerie par Résonance Magnétique), la tomographie d'émission monophotonique (TEMP ou SPECT pour « Single Photon Emission Computed Tomography »), la tomoscin-tigraphie par émission de positons (TEP) et la luminescence. De préférence, la méthode d'imagerie médicale est l'imagerie par rayons X. SPECT si le complexe selon l'invention comprend un émetteur gamma et TEP si le complexe selon l'invention comprend un émetteur béta+.

Le terme « Lipiodol®» se réfère à une huile iodée et de manière préférentielle à la spécialité pharmaceutique Lipiodol®, solution injectable fabriquée et commercialisée par Guerbet et constituée par des esters éthyliques des acides gras iodés de l'huile d'oeillette. Lipiodol® est un produit notamment utilisé pour la visualisation, la localisation et/ou la vectorisation au cours de la chimioembolisation transartérielle du carcinome hépa-tocellulaire au stade intermédiaire, chez l'adulte ainsi que pour le diagnostic par voie artérielle hépatique sélective de l'extension hépatique des lésions malignes hépatiques ou non.

Par acide organique (ou fonction acide organique), on entend un composé organique (ou une fonction organique) présentant des propriétés acides, c'est-à-dire capable de libérer un cation $H^+$, ou $H_3O^+$ en milieux aqueux. Parmi les acides organiques, on peut citer les acides carboxyliques, les acides sulfoniques, les phosphates et les phosphonates. De préférence, les fonctions acides organiques selon l'invention sont des groupements carboxyles. De telles fonctions acides sont salifiables et peuvent se présenter sous leur forme basique. En particulier, ces fonctions acides se présentent sous forme de sels pharmaceutiquement acceptables, tels que définis ci-dessous ; par exemple, sous forme de sel de sodium ou de méglumine (1-Deoxy-1-(methylamino)-D-glucitol ou *N*-Methyl-D-glucamine).

**Les huiles iodées**

On entend par le terme « acide gras » désigner des acides carboxyliques aliphatiques saturés ou insaturés présentant une chaîne carbonée d'au moins 4 atomes de carbone. Les acides gras naturels possèdent une chaîne carbonée de 4 à 28 atomes de carbone (généralement un nombre pair). On parle d'« acide gras à longue chaîne » pour une longueur de 14 à 22 carbones et à très longue chaîne s'il y a plus de 22 carbones. On parle au contraire d'« acide gras à courte chaîne » pour une longueur de 4 à 10 carbones, notamment 6 à 10 atomes de carbone, en particulier 8 ou 10 atomes de carbone. L'homme du métier connaît la nomenclature associée et en particulier utilise :

- Ci-Cp pour désigner une fourchette d'acides gras en Ci à Cp,
- Ci+Cp, le total des acides gras en Ci et des acides gras en Cp,

Par exemple :

- les acides gras de 14 à 18 atomes de carbone s'écrivent « acides gras en C14-C18 »,
- le total des acides gras en C16 et des acides gras en C18 s'écrit C16 +C18,
- pour un acide gras saturé, un homme du métier utilisera la nomenclature suivante Ci : 0, où i est le nombre d'atomes de carbone de l'acide gras. L'acide palmitique sera par exemple désigné par la nomenclature (C16 :0),
- pour un acide gras insaturé, un homme du métier utilisera la nomenclature suivante Ci : x n-N où N sera la position de la double liaison dans l'acide gras insaturé en partant du carbone opposé au groupe acide, i est le nombre d'atomes de carbone de l'acide gras, x est le nombre de double liaisons (insaturations) de cet acide gras. L'acide oléique, sera par exemple désigné par la nomenclature (C18 :1 n-9).

De façon avantageuse, l'huile iodée selon l'invention comprend ou est constituée de dérivés d'acides gras iodés, préférentiellement d'esters éthyliques d'acides gras iodés, plus préférentiellement d'esters éthyliques d'acides gras iodés d'huile d'oeillette, d'huile d'olive, d'huile de graines de colza, d'huile d'arachide, d'huile de graines de soja ou d'huile de noix, encore plus préférentiellement d'esters éthyliques d'acides gras iodés d'huile d'oeillette ou d'huile

d'olive. Plus préférentiellement, l'huile iodée selon l'invention comprend ou est constituée d'esters éthyliques d'acides gras iodés d'huile d'oeillette (aussi appelée pavot noir ou *Papaver somniferum var. nigrum*). L'huile d'oeillette, aussi appelée huile de graines de pavot ou huile de graines d'oeillette, contient préférentiellement plus de 80% d'acides gras insaturés (en particulier de l'acide linoléique (C18 :2 n-6) et de l'acide oléique (C18 :1 n-9)) dont au moins 70% d'acide linoléique et au moins 10% d'acide oléique. L'huile iodée est obtenue à partir de la complète iodation d'une huile telle que l'huile d'oeillette dans des conditions permettant une liaison d'un atome d'iode pour chaque double liaison des acides gras insaturés (Wolff et al. 2001, Medicine 80, 20-36) suivie d'une trans-estérification.

L'huile iodée selon l'invention contient préférentiellement de 29 à 53% (m/m), plus préférentiellement 37% à 39% (m/m) d'iode.

Comme exemples d'huiles iodées peuvent être cités le Lipiodol®, le Brassiodol® (issu de l'huile de graines de colza (*Brassica compestis*), le Yodiol® (issu de l'huile d'arachide), l'Oriodol® (issu de l'huile d'oeillette mais sous forme de triglycérides d'acides gras), le Duroliopaque® (issu de l'huile d'olive).

Préférentiellement, l'huile iodée est Lipiodol®, une huile iodée utilisée comme produit de contraste et dans certaines procédures de radiologie interventionnelle. Cette huile est un mélange d'esters éthyliques d'acides gras iodés et non-iodés d'huile de graines d'oeillette. Elle consiste majoritairement (en particulier, plus de 84%) en un mélange d'esters éthyliques d'acides gras iodés à longue-chaine (en particulier des acides gras en C18) issus de l'huile de graines d'oeillette, préférentiellement en un mélange de monoiodostéarate d'éthyle et de diiodostéarate d'éthyle. L'huile iodée peut aussi être une huile à base d'ester éthylique monoiodé d'acide stéarique (C18 :0) issu de l'huile d'olive. Un produit de ce type, s'appelant Duroliopaque® était commercialisé il y a quelques années.

Les caractéristiques principales du Lipiodol® sont les suivantes :

| **Composés** | **Proportions dans le mélange d'acide gras** |
|---|---|
| Palmitate d'éthyle (Ethyl C16 :0) | 4,6 à 6,7% (m/m), préférentiellement 4,8% (m/m) |
| Stéarate d'éthyle (Ethyl C18 :0) | 0,8 à 1,9% (m/m), préférentiellement 1,2% (m/m) |
| Monoiodostéarate d'éthyle | 11,3 à 15,3% (m/m), préférentiellement 13,4% (m/m) |
| Diiodostéarate d'éthyle | 73,5 à 82,8% (m/m), préférentiellement 78,5% (m/m) |

| **Autres caractéristiques du Lipiodol® :** | |
|---|---|
| Iode | 37% à 39% (m/m) (soit 480 mg/ml) |
| Viscosité<br>à 37°C<br>à 20°C | <br>25 mPa.s<br>50 mPa.s |
| Densité | 1,268 - 1,290 g/cm$^3$ à 20°C, préférentiellement 1,28 |

**Composés de formule générale (I)**

Selon un mode de réalisation, les composés de formule générale (I) sont sous forme de sel, de préférence sous forme de sel pharmaceutiquement acceptable.

Par « sel pharmaceutiquement acceptable », on désigne notamment des sels permettant de conserver les propriétés et l'efficacité biologique des composés selon l'invention. Des exemples de sels pharmaceutiquement acceptables se trouvent dans Berge, et al. ((1977) J. Pharm. Sd, vol. 66, 1). Par exemple, les composés de formule générale (I) sont sous forme de sel de sodium ou de méglumine (1-Deoxy-1-(methylamino)-D-glucitol ou N-Methyl-D-glucamine).

L'invention concerne également les solvates tels que les hydrates des composés de formule (I).

Selon un mode de réalisation, le composé de formule (I) est choisi parmi le groupe constitué des composés suivants :

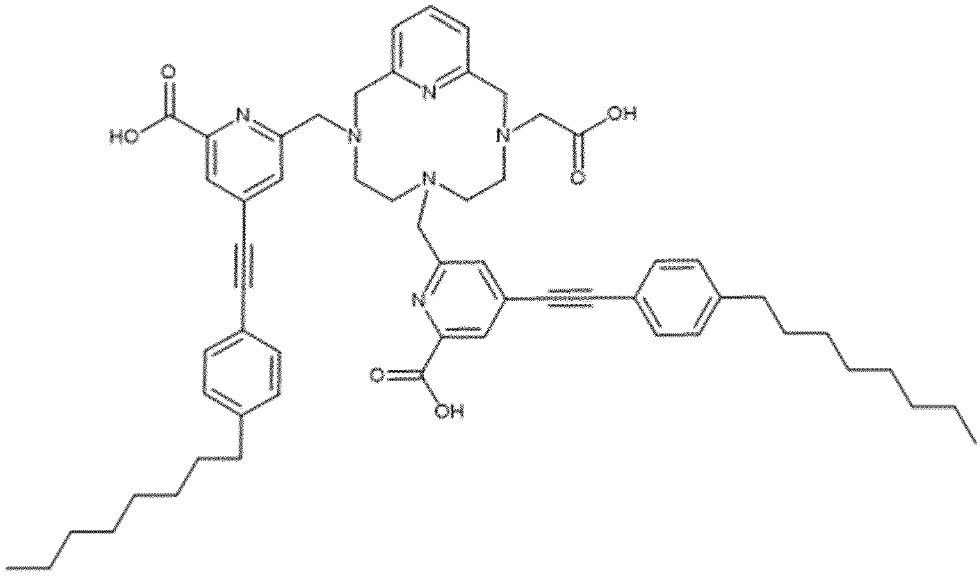

(ce composé est celui de l'exemple 11a et a pour nomenclature 6,6'-((9-(carboxymethyl)-3,6,9-triaza-1(2,6)-pyridinacyclodecaphane-3,6-diyl)bis(methylene))bis(4-((4-octylphenyl)ethynyl)picolinic acid))

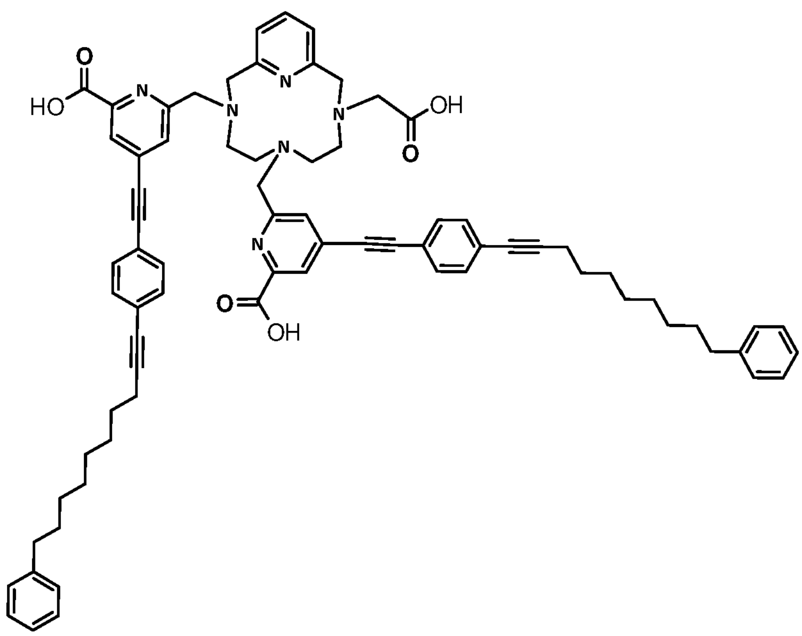

(ce composé est celui de l'exemple 11b et a pour nomenclature 6,6'-((9-(carboxymethyl)-3,6,9-triaza-1(2,6)-pyridinacyclodecaphane-3,6-diyl)bis(methylene))bis(4-((4-(10-phenyldec-1-yn-1-yl)phenyl)ethynyl)picolinic acid))

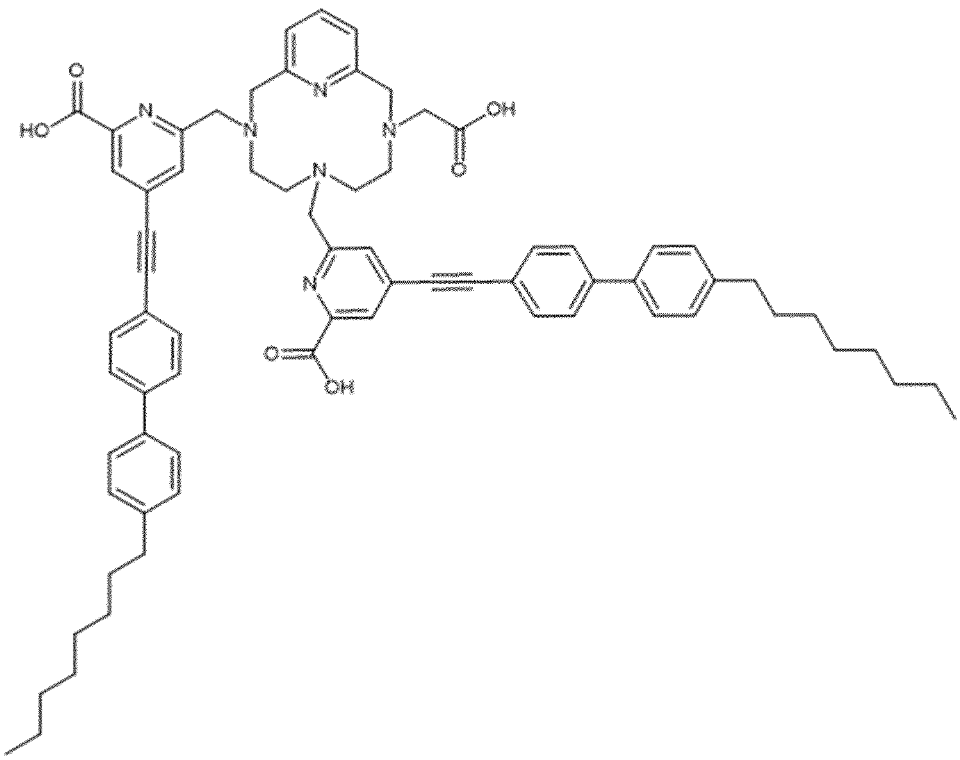

(ce composé est celui de l'exemple 11c et a pour nomenclature 6,6'-((9-(carboxymethyl)-3,6,9-triaza-1(2,6)-pyridinacyclodecaphane-3,6-diyl)bis(methylene))bis(4-((4'-octyl-[1,1'-biphenyl]-4-yl)ethynyl)picolinic acid)
et l'un de leurs sels pharmaceutiquement acceptables.

Selon un mode de réalisation particulier, le composé de formule (I) est le composé suivant :

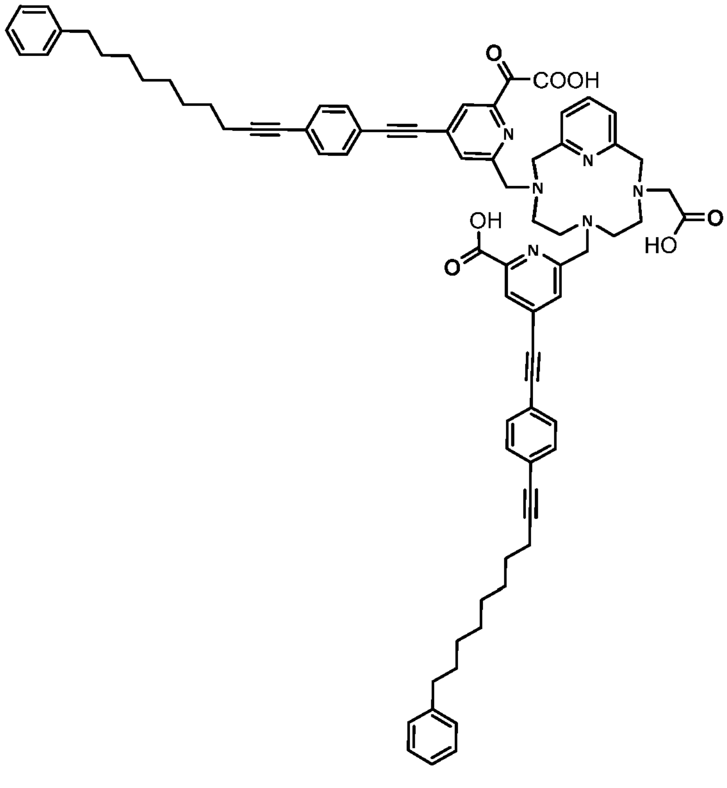

ou l'un de ses sels pharmaceutiquement acceptables.

**Complexes**

L'invention concerne également un complexe d'un composé de formule (I) ou d'un de ses sels pharmaceutiquement acceptables, tel que défini ci-dessus, avec un élément chimique M, de préférence un métal.

Selon un mode de réalisation, les composés de formule générale (I) sont sous forme de complexe neutre avec les cations aux degrés d'oxydation III.

Selon un mode de réalisation, l'élément chimique M est un cation métallique choisi parmi le groupe constitué de cuivre (II), de gallium (III), d'indium (III), de scandium (III), d'yttrium (III), de samarium (III), de terbium (III), d'holmium (III), de lutétium (III), d'actinium (III), de manganèse, de préférence l'yttrium, le lutétium, le terbium, l'indium, le gallium, le cuivre et l'actinium. De manière encore plus préférentielle, l'élément chimique M est un cation métallique choisi parmi le groupe constitué de l'yttrium (III), du lutétium (III), du cuivre (II) et de l'actinium (III).

De préférence, M est un radioélément choisi parmi les isotopes radioactifs de l'yttrium, du lutétium, du terbium, de l'indium, du gallium, du cuivre, de l'actinium et du manganèse. Selon un mode de réalisation particulier, l'élément chimique M est un radioélément choisi parmi le groupe constitué de $^{44}$Sc(III), $^{47}$Sc(III), $^{111}$In(III), $^{152}$Tb(III), $^{155}$Tb(III), $^{149}$Tb(III), $^{161}$Tb(III), $^{64}$Cu(III),$^{61}$Cu(III), $^{67}$Cu(II) ,$^{68}$Ga(III), $^{90}$Y(III), $^{153}$Sm(III), $^{166}$Ho(III), $^{177}$Lu(III), $^{52}$Mn et $^{225}$Ac(III), de préférence $^{90}$Y(III), $^{177}$Lu(III), $^{67}$Cu(II), $^{225}$Ac(III), $^{111}$In(III), $^{152}$Tb(III), $^{155}$Tb(III), $^{149}$Tb(III), $^{161}$Tb(III) et $^{68}$Ga(III).

Selon un mode de réalisation, ledit complexe est de formule générale (III) suivante :

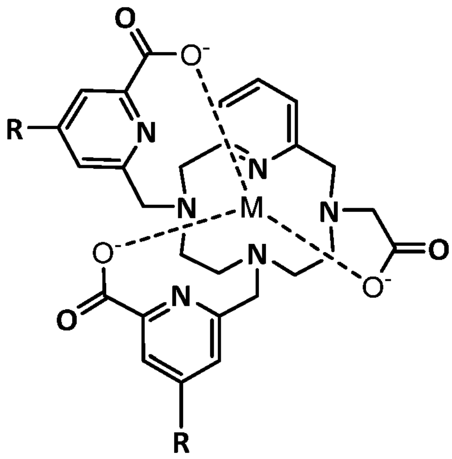

(III),

dans laquelle le groupement R et M sont tels que définis ci-dessus.

La synthèse de tels complexes est illustrée en exemples 12 et 13.

Selon un mode de réalisation particulier, le complexe de formule (III) est choisi parmi le groupe constitué des complexes suivants :

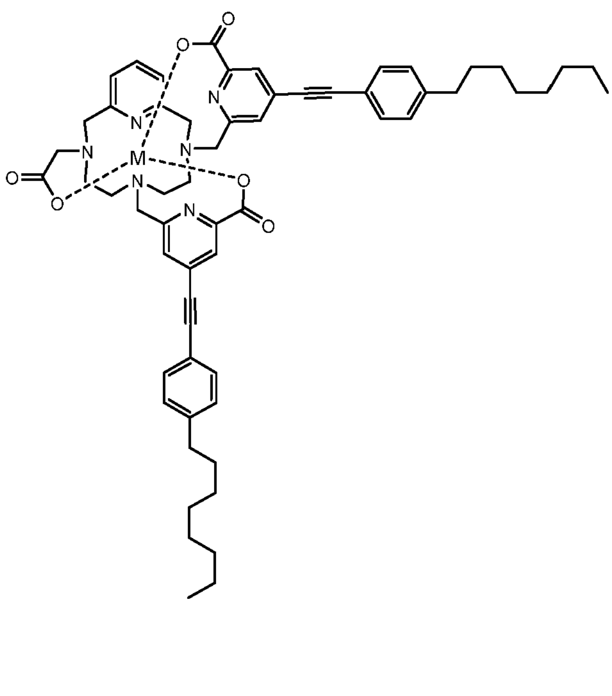

(ce complexe de formule (III) est formé avec le composé de l'exemple 11a)

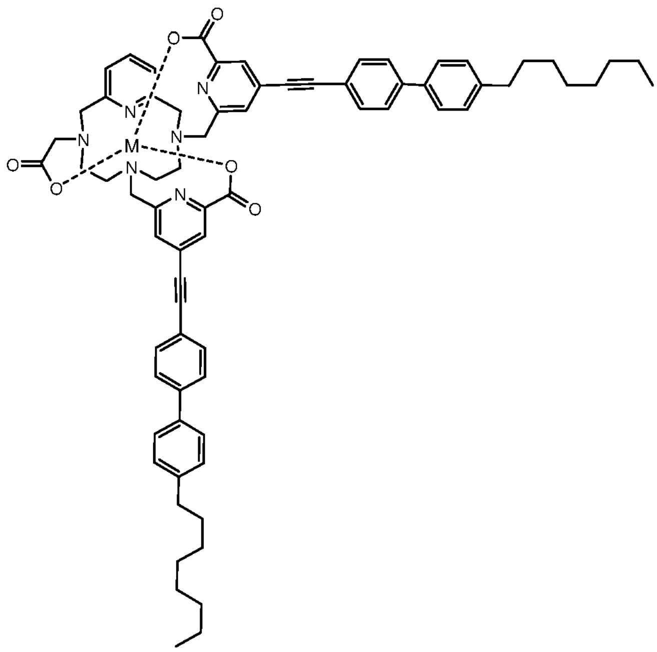

(ce complexe de formule (III) est formé avec le composé de l'exemple 11c) et

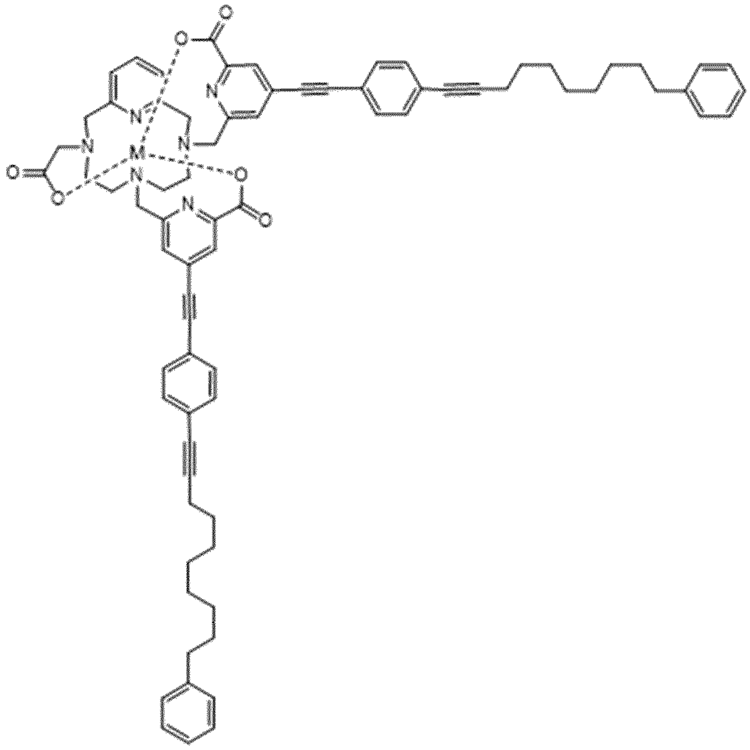

(ce complexe de formule (III) est formé avec le composé de l'exemple 11b).

**Composition pharmaceutique**

L'invention concerne également une composition pharmaceutique comprenant un composé de formule (I) tel que défini ci-dessus ou un complexe de formule (III) tel que défini ci-dessus, et éventuellement un ou plusieurs excipient(s) pharmaceutiquement acceptable(s). La composition pharmaceutique peut comprendre un composé de formule (I) tel

que défini ci-dessus ou un complexe de formule (III) tel que défini ci-dessus, dans un milieu pharmaceutiquement acceptable. A titre d'exemples, on peut citer comme excipients les radioprotecteurs ou les antioxydants. On entend par radiolyse une réaction chimique provoquée par un rayonnement ionisant et qui est susceptible d'initier ou d'accélérer des mécanismes de dégradation du radiopharmaceutique. Un radioprotecteur possède la propriété de bloquer ou limiter ces phénomènes de dégradation radiochimiques.

La composition pharmaceutique peut comprendre une phase huileuse, notamment une huile iodée. Selon un mode de réalisation particulier, la composition pharmaceutique comprend en outre des esters éthyliques d'acides gras iodés de l'huile d'oeillette.

Selon un mode de réalisation, la composition pharmaceutique selon l'invention comprend au moins un excipient pharmaceutiquement acceptable. Selon un autre mode de réalisation, la composition pharmaceutique selon l'invention ne comprend pas d'excipient.

Selon un mode de réalisation, la composition pharmaceutique selon l'invention est constituée d'une huile iodée et de composés ou de complexes selon l'invention. Typiquement, la composition pharmaceutique selon l'invention est constituée de Lipiodol® et de composés ou de complexes selon l'invention. Le Lipiodol® est constitué d'esters éthyliques d'acides gras iodés de l'huile d'oeillette. De préférence, la composition pharmaceutique selon l'invention est constituée de Lipiodol® et du composé de l'exemple 11b, ou bien de Lipiodol® et du complexe de l'exemple 13a (i.e. complexe d'yttrium 90 du composé de l'exemple 11b) ou 13c (i.e. complexe de lutétium 177 du composé de l'exemple 11b).

De préférence, la composition pharmaceutique selon l'invention est radio-opaque, et donc visible par radiographie aux rayons X.

Selon un mode de réalisation particulier, la composition pharmaceutique est une composition injectable. Selon un mode de réalisation, la composition pharmaceutique selon l'invention est administrée par injection hépatique intra-artérielle.

L'invention concerne un complexe ou une composition pharmaceutique tels que définis ci-dessus, pour son utilisation dans le traitement des cancers, en particulier des cancers du foie.

L'invention concerne également un complexe ou une composition pharmaceutique tels que définis ci-dessus, pour son utilisation en imagerie médicale.

L'invention concerne l'utilisation d'un complexe tel que défini ci-dessus pour la préparation d'un médicament pour le traitement de cancers.

L'invention concerne également l'utilisation d'un complexe ou d'une composition pharmaceutique tels que définis ci-dessus en imagerie médicale.

L'invention concerne une méthode de traitement thérapeutique d'un patient atteint d'un cancer, comprenant l'administration audit patient d'un complexe ou d'une composition pharmaceutique tels que définis ci-dessus. En particulier ladite méthode de traitement ne comprend pas d'étape de traitement chirurgical.

L'invention concerne également une méthode d'imagerie médicale d'une tumeur comprenant :

- une étape d'administration à un patient atteint d'un cancer d'un complexe ou d'une composition pharmaceutique selon l'invention ; et
- une étape de détection de la tumeur par une méthode d'imagerie médicale.

Par « cancer », on entend une prolifération cellulaire anormale (également appelée tumeur) au sein d'un tissu normal de l'organisme. Ces cellules cancéreuses dérivent toutes d'un même clone, cellule initiatrice du cancer qui a acquis certaines caractéristiques lui permettant de se diviser indéfiniment. Au cours de l'évolution de la tumeur, certaines cellules cancéreuses peuvent migrer hors de leur lieu de production et former des métastases.

Parmi les cancers, on peut notamment citer les cancers du foie, en particulier les cancers primitifs du foie, de préférence les hépatocarcinomes. Selon un mode de réalisation particulier, on peut citer parmi les cancers, l'hépatocarcinome, l'hémangioendothéliome épithélioïde, le cholangiocarcinome, les tumeurs neuro-endocrines et les métastases d'autres cancers telles que les métastases du cancer colorectal.

Selon un mode de réalisation particulier, le cancer est un carcinome hépatocellulaire au stade intermédiaire, chez l'adulte.

### Procédé de préparation des composés de formule générale (I) et radiomarquage

L'invention concerne également un procédé de préparation pour les composés de formule générale (I) selon l'invention.

Dans ce procédé de préparation, les étapes de déprotection sont connues de l'homme du métier et correspondent à des réactions classiques d'hydrolyse d'un amide. Les étapes de fonctionnalisation sont également connues de l'homme du métier et correspondent à des réactions d'alkylation classiques (cf. Loic Bellouard J CHEM S Perkin 1, (23), 1999, pp. 3499-3505).

Ce procédé de préparation est avantageusement basé sur la réaction d'un diester de l'acide oxalique sur le pyclène qui permet de bloquer deux atomes d'azote du pyclène (N-6 et N-9) afin de pouvoir intervenir sélectivement sur le troisième atome (N-3) laissé libre. Après fonctionnalisation de l'azote en position -3, la déprotection du groupe oxalamide conduit à un pyclène substitué en position -3 de manière contrôlée, selon le schéma 1 suivant :

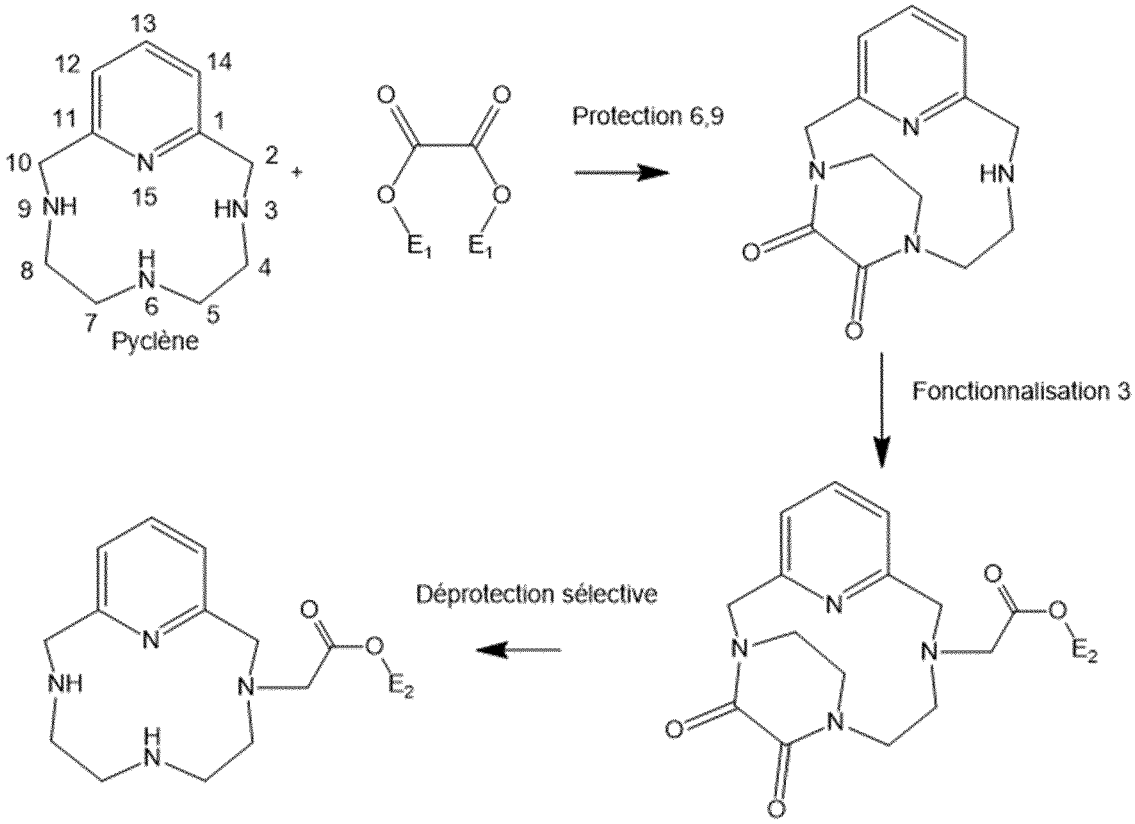


Schéma 1

Selon un mode de réalisation, l'étape de protection est réalisée en présence de méthanol.

L'invention concerne un procédé de préparation des composés de formule générale (I) comprenant une étape de fonctionnalisation d'un composé de formule générale (IX) suivante :

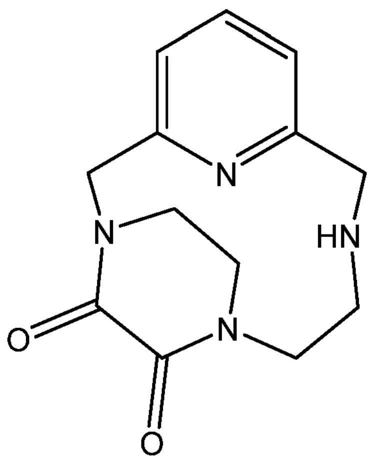

(IX)

pour former un composé de formule générale (X) suivante :

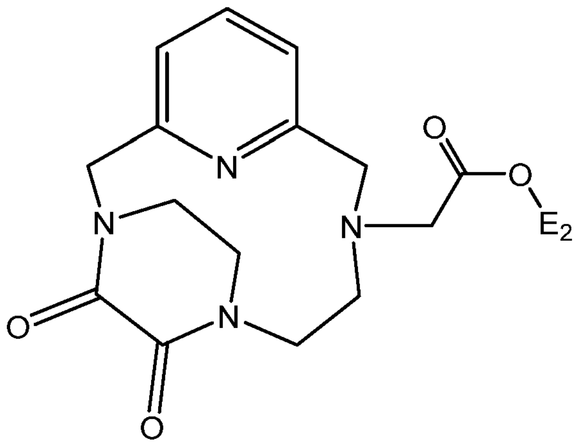

(X),

dans laquelle E2 est un groupe protecteur alkyle en C1-C4 pouvant par exemple être choisi dans le groupe constitué de méthyl, éthyle, isopropyle et terbutyle.

Ledit procédé de préparation comprend en outre :

- une étape de déprotection du composé de formule générale (X), pour obtenir un composé de formule générale (XI) suivante :

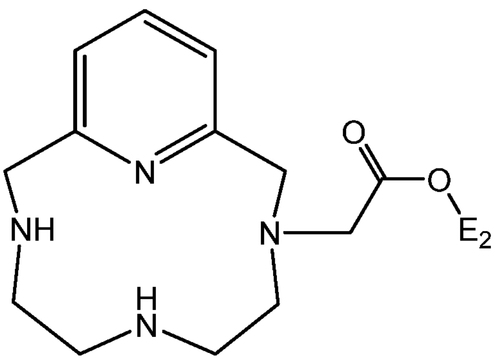

(XI)

et

- une étape de fonctionnalisation du composé de formule générale (XI) selon le schéma 2, pour obtenir un composé de formule générale (I) tel que défini ci-dessus :

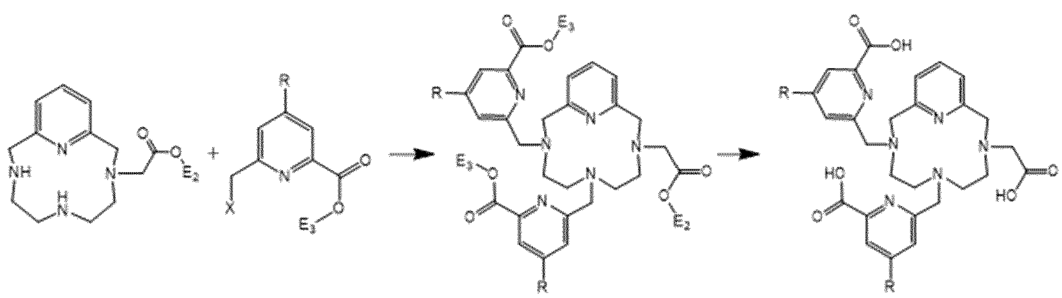

(XI) (XII) (XIII) (I)

Schéma 2

E1, E2, E3 étant des groupes protecteurs alkyle en C1-C4 pouvant par exemple être indépendamment choisis dans le groupe constitué de méthyl, éthyle, isopropyle et terbutyle.

La demande décrit également un composé de formule générale (X) suivante :

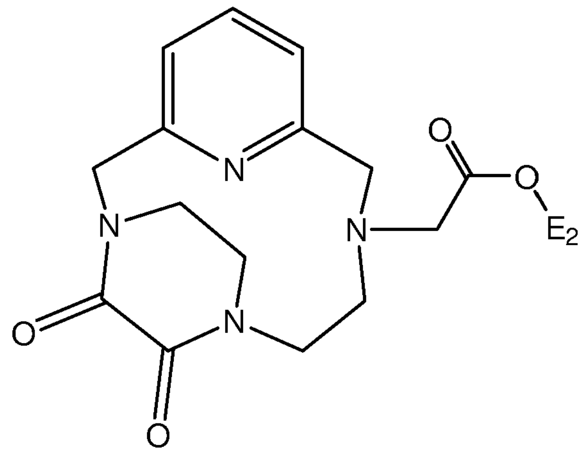

(X),

dans laquelle E2 est un groupe protecteur alkyle en C1-C4 pouvant par exemple être choisi dans le groupe constitué de méthyl, éthyle, isopropyle et terbutyle.

Selon un mode de réalisation particulier du procédé de préparation des composés de formule (I), la préparation d'un intermédiaire picolinate substitué est réalisée via un dérivé bromé en position -4, ce qui permet par une réaction de couplage avec un alcyne, catalysée au palladium (réaction dite de Sonogashira, Comprehensive Chirality, Volume 4, Pages18-32, 2012), d'installer le résidu choisi, selon le schéma 3 ci-dessous:

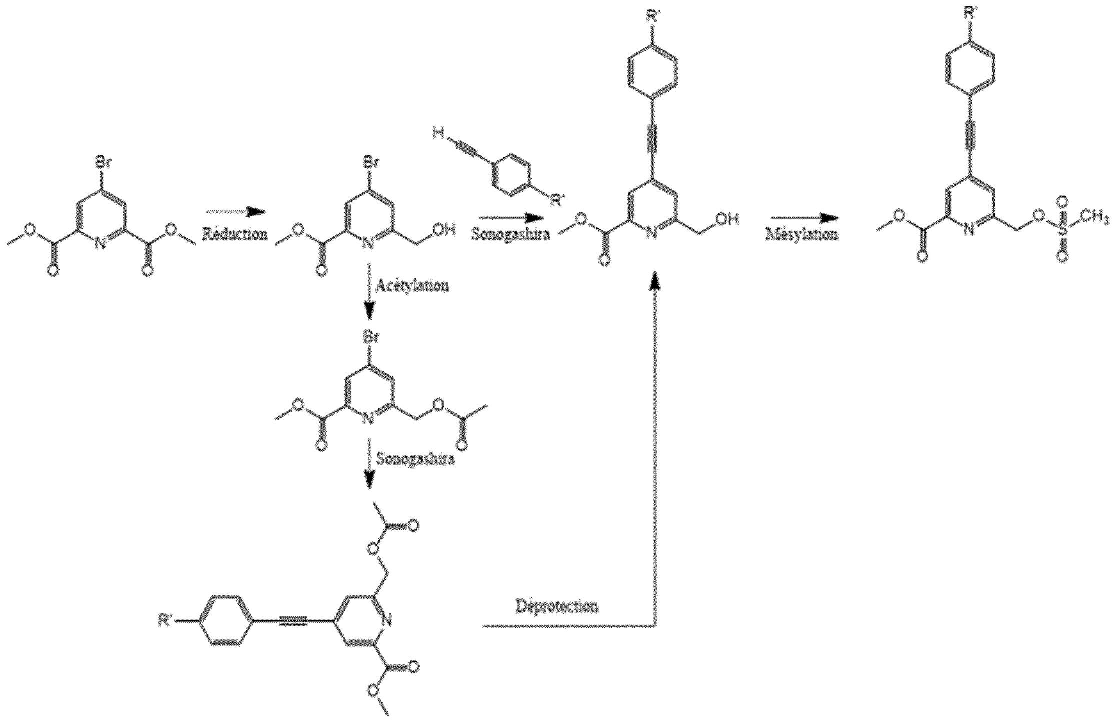


Schéma 3

Avec R' étant un groupement de formule suivante -L1-(CH2)n-L2, où

- L1 représente Ph ou -CΞC- ou CH2,
- L2 représente H ou Ph ou alkylphényle, et
- n compris entre 4 et 12.

De préférence, R' est l'un des radicaux suivants :

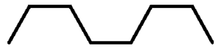

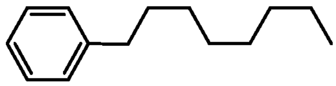

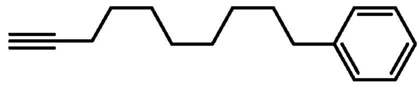

L'invention concerne également un procédé de radiomarquage des composés de formule générale (I). Ledit procédé de radiomarquage est de préférence réalisé à un pH compris entre 5 et 9, de préférence entre 5 et 7, de préférence 5,2 afin de permettre la complexation. Selon un mode de réalisation particulier, ledit radiomarquage est réalisé en présence de tampon acétate pour ajuster le pH et permettre ainsi la complexation. Selon un mode de réalisation, le radiomarquage est réalisé en présence d'eau ou d'alcool tel que l'éthanol, ou leurs mélanges.

Le radiomarquage est réalisé à une température comprise entre 60°C et 100°C, de préférence entre 80°C et 100°C, de manière plus préférentielle 80°C.

Les résultats de radiomarquage sont présentés dans le tableau 1 ci-dessous. La pureté radiochimique du complexe selon l'invention après complexation à l'yttrium 90 est exprimée en pourcentage de la radioactivité totale engagée. Le pourcentage d'extraction du complexe radiomarqué dans le Lipiodol® représente la fraction de la radioactivité totale qui est extraite dans la phase huileuse.

**Tableau 1**

| Ligand | Complexe 90Y | RCP % | Lipiodol® Extraction% |
|---|---|---|---|
| **Exemple 11a** | Exemple 15 | 99,7 | 92,9 |
| **Exemple 11c** | Exemple 14 | 92,7 | 89,5 |
| **Exemple 11b** | Exemple 13a | 98,1 | 90,0 |
| Ligand $1.10^{-3}$ M<br>RCP : pureté radiochimique | | | |

Ces données démontrent l'efficacité du radio marquage des ligands selon l'invention et que les complexes radios marqués selon l'invention ont une affinité élevée pour la phase huileuse.

A l'issue des opérations de radio-marquage, les complexes radiomarqués incorporés à la phase huileuse sont engagés dans un test de stabilité dans le sérum humain. Pour cela, les solutions huileuses sont incubées à 37°C sous agitation modérée en présence de sérum humain.

La répartition de la radioactivité dans la phase huileuse et dans le sérum est mesurée à intervalle régulier afin de déterminer la fraction de radioactivité qui diffuse vers le sérum en fonction du temps. Des courbes d'extraction sont ainsi établies et permettent d'évaluer le comportement des différents produits et de les comparer. Ces tests sont décrits à l'exemple 16.

## Description des figures

Figure 1 : Relargage d'yttrium 90 ou d'indium 111 pour les composés des exemples 13a et 13b respectivement dans la phase aqueuse (sérum physiologique).

Figure 2 : Relargage d'yttrium 90 dans le sérum humain (phase aqueuse) pour les composés des exemples 13a, 14 et 15.

Figure 3 : Relargage d'yttrium 90 dans le sérum humain (phase aqueuse) pour le composé comparateur 2.

Figure 4 : Relargage d'yttrium 90 dans le sérum humain (phase aqueuse) pour les composés selon l'invention des exemples 13a, 14 et 15 ; et pour les composés comparateurs 3 à 5.

Figure 5 : Evaluation du ratio de la dose captée par la tumeur par rapport au foie sain pour le composé de l'exemple 13a.

Figure 6 : Résultats de biodistribution du composé de l'exemple 13a comparé au composé de l'exemple 13b,

Pourcentage de dose injectée par organe.
Figure 7 : Relargage de lutétium 177 dans le sérum humain et dans le sérum physiologique pour le composé selon l'invention de l'exemple 13c.

Les exemples suivants sont décrits à titre illustratifs de la présente invention.

## EXEMPLES

### Matériels et méthodes

**Radiomarquage des ligands, synthèse des complexes chaud** :

Le chlorure d'yttrium-90 est acheté chez PerkinElmer Life Sciences, l'indium-111 chez Curium. Le lutétium-177 nca (no carrier added) est fournis par ITM. Les radioactivités mises en jeu dans ces exemples sont comprises entre 28 pCi et 8,51 mCi pour l'yttrium (1,04 - 314,87 MBq) et 4.71 mCi (174 MBq) pour l'indium, 673 MBq pour le lutétium.
Les produits (solvants HPLC, tampons...) sont utilisés tels quels, sans purification supplémentaire. Excepté si précisé autrement, le ligand est solubilisé dans l'éthanol.
Les expériences ont été réalisées dans des flacons en verre borosilicaté sertis. Les flacons ont été chauffés dans un bloc chauffant Bioblock permettant de chauffer jusqu'à 6 flacons. Lorsqu'une agitation était nécessaire, un vortex Lab Dancer S40 (VWR) était utilisé. Les centrifugations ont été réalisées avec une centrifugeuse MF 20-R (Awel).
Les radioactivités étaient mesurées dans un activimètre CRC-127R (Capintec), dont la calibration était réalisée chaque matin.
Les contrôles qualité ont été réalisés par chromatographie sur couche mince (CCM) sur papier Whatman 1, avec comme éluant un mélange $MeOH/NEt_3$ 0.1 %. Les Puretés Radio-Chimiques sont déterminées à l'aide d'un phosphoimageur Cyclone (Perkin Elmer), à l'aide du logiciel Optiquant.
Les analyses par chromatographie en phase liquide à haute performance (HPLC) décrite selon la méthode 10 sont réalisées sur une chaîne HPLC Dionex Ultimate 3000 équipée d'un détecteur à barrette de diodes et d'un détecteur radiochromatographique fLumo (Berthold), pilotée par le logiciel Chromeleon.
Dans les méthodes de synthèse décrites ci-dessous, les produits commerciaux ainsi que les solvants proviennent essentiellement des sociétés Sigma-Aldrich®, Merck et VWR®. La température ambiante est comprise entre 20°C et 25°C. Les évaporations des solvants sont réalisées sous pression réduite, à l'aide d'un évaporateur Buchi R-210, à des températures d'environ 40°C.
Les purifications par flash chromatographie sont effectuées en utilisant des cartouches de silices en gel irrégulières ou en oxyde d'aluminium neutre de la marque Buchi (40g, 80g, 120g, 220g ou 440g) avec les appareils suivants :

- CombiFlash NextGen 300+ de Teledyne ISCO® équipé d'un détecteur UV de 200 à 400nm ou UV-visible de 200 à 800nm

- Reveleris X2 de Buchi équipé d'un détecteur UV ou UV-Vis (200 à 850nm) et d'un détecteur évaporatif à diffusion de lumière (DEDL).

Les purifications sur colonnes HPLC préparatives sont faites sur la PuriFlash F4250 de chez Interchim® équipée d'un détecteur UV de 200 à 600nm et d'un DEDL selon la méthode 11.
Les analyses et le suivi réactionnel sont faits par CCM dans une cuve saturée par les vapeurs du solvant d'élution. Le support utilisé est de la silice en gel sur plaque de verre de granulométrie 60Â avec indicateur fluorescent F254 ou de l'oxyde d'aluminium basique sur plaque de verre ou neutre sur plaque d'aluminium de granulométrie 60Â avec indicateur fluorescent F254 de la marque Merck®. On définit le rapport frontal (Rf) des composés par le calcul suivant :

$$Rf = \frac{\textit{Distance ligne de dépôt-composé}}{\textit{Distance ligne de dépôt-front de solvant}}$$

Les analyses et le suivi réactionnel sont également faits par chromatographie en phase liquide à haute performance sur une chaine Agilent de la série1200 équipé d'un détecteur UV ou UV/Vis G1315D DAD SL et traités avec le logiciel EMPOWER® ou sur une chaine Shimadzu LCMS-2020 équipée d'un détecteur UV ou UV/Vis SPD-M30A et d'un spectromètre de masse quadripolaire puis traités avec le logiciel Labsolution. L'échantillon introduit à partir du

chromatographe en phase liquide est ensuite pulvérisé et ionisé sous pression atmosphérique par l'électrospray (ESI) sous forme positive (ES+) ou négative (ES-). Des infusions sont également réalisées sur HPLC Ultimate 3000 RS de chez ThermoFisher® avec des injections de 5 μL/min et une détection en masse par trappe ionique amaZon X de chez Bruker®. Les résultats sont exprimés par le rapport masse/charge (m/z).

**Méthodes d'analyse et de suivi réactionnel**

Différentes méthodes d'analyse et de suivi réactionnel ont été utilisées pour chacun des composés. Elles sont décrites ci-dessous et seront précisées pour chaque synthèse.

Méthode 1 : HPLC (Chromatographie en phase liquide à haute performance)

Instrument : Agilent HP1200 ; Colonne : Waters : Xbride Amide 3,5 μm ; 4.6x150 mm ; éluant A : Acétonitrile, éluant B : Tampon formiate 10mM pH=3,3 ; débit 1.0mUmin; Température : 25°C ; injection : 10μL; Longueur d'onde 254nm; gradient :

| Temps (minutes) | %A | %B |
|---|---|---|
| **0** | 95 | 5 |
| **15** | 60 | 40 |
| **18** | 95 | 5 |
| **20** | 95 | 5 |

Méthode 2 : LCMS (Chromatographie en phase liquide à haute performance/ Masse)

Instrument : LC/MS Shimadzu ; Colonne : Waters : Kinextex C8 100x2,1 mm 1,7 μm ; éluant A : eau/acide trifluoroacétique (TFA) 0,05 %, éluant B : Acétonitrile; débit 0.5mL/min; Température : 30°C ; injection : 1μL; Longueur d'onde 210nm; gradient :

| Temps (minutes) | %A | %B |
|---|---|---|
| 0.01 | 50 | 50 |
| 5.00 | 5 | 95 |
| 10.00 | 5 | 95 |
| 10.01 | 50 | 50 |
| 15.00 | 50 | 50 |

Méthode 3 : LCMS

Instrument : LC/MS Shimadzu ; Colonne : ThermoFisher : Hypersil Gold 50 × 2.1mm 1.9μm ; éluant A : eau/acide formique 0,1 % (v/v), éluant B : Acétonitrile; débit 0.5mL/min; Température : 60°C ; injection : 1μL; Longueur d'onde 260nm; gradient :

| Temps en min | % solution A | % solution B | Débit (ml/min) |
|---|---|---|---|
| 0 | 75 | 25 | 0.5 |
| 1 | 75 | 25 | 0.5 |
| 11 | 0 | 100 | 0.5 |
| 13 | 0 | 100 | 0.5 |
| 14 | 75 | 25 | 0.5 |
| 20 | 75 | 25 | 0.5 |

Méthode 4 : LCMS

Instrument : LC/MS Shimadzu ; Colonne : ThermoFisher : Symmetry C18 150 × 4.6mm 5μm; éluant A : eau/acide trifluroacétique 0,05 % (v/v), éluant B : Acétonitrile; débit 1mL/min; Température : 25°C ; injection : 1μL; Longueur d'onde 260nm; gradient :

| Temps en min | % solution A | % solution B | Débit (ml/min) |
|---|---|---|---|
| 0 | 98 | 2 | 1 |
| 12 | 0 | 100 | 1 |
| 20 | 0 | 100 | 1 |
| 25 | 98 | 2 | 1 |
| 30 | 98 | 2 | 1 |

Méthode 5 : LCMS

Instrument : LC/MS Shimadzu ; Colonne : Waters : Kinextex C8 100x2,1 mm 1,7 μm ; éluant A : eau/acide formique 0,3% (v/v) ; éluant B : Acétonitrile ; débit 0.5mL/min; Température : 30°C ; injection : 1μL; Longueur d'onde 274nm; gradient :

| Temps (minutes) | %A | %B |
|---|---|---|
| 0.01 | 80 | 20 |
| 0.5 | 80 | 20 |
| 5 | 50 | 50 |
| 5.5 | 50 | 50 |
| 6.0 | 80 | 20 |
| 8.0 | 80 | 20 |

Méthode 6 : LCMS

Instrument : LC/MS Shimadzu ; Colonne : Kinextex C8 100x2,1 mm 1,7 μm ; éluant A : eau/acide formique 0,3% (v/v), éluant B : Acétonitrile ; débit 0.5mL/min ; Température : 30°C ; injection : 1μL ; Longueur d'onde 274nm; gradient :

| Temps (minutes) | %A | %B |
|---|---|---|
| 0.01 | 40 | 60 |
| 0.5 | 40 | 60 |
| 8.0 | 25 | 75 |
| 10.0 | 10 | 90 |
| 10.5 | 10 | 90 |
| 11.000 | 40 | 60 |
| 13.00 | 40 | 60 |

Méthode 7 : LCMS

Instrument : LC/MS Shimadzu ; Colonne : Accucore C30 150×2,1mm 2,6μm ; éluant A : eau/acide formique 0,3% (v/v), éluant B : Acétonitrile ; débit 0,6mL/min ; Température : 40°C ; injection : 1μL ; Longueur d'onde 320nm ; gradient :

| Temps en minutes | % A | % B |
|---|---|---|
| 0.01 | 50 | 50 |
| 5.00 | 5 | 95 |
| 10.00 | 5 | 95 |
| 10.01 | 50 | 50 |
| 15.00 | 50 | 50 |

Méthode 8 : LCMS

Instrument: LC/MS Shimadzu; Colonne: Hypersil GOLD Thermo Scientific 150x3m 3μm ; éluant A : eau/acide formique 0,1% (v/v) ; éluant B : Acétonitrile/acide formique 0,1% (v/v) ; débit 1mL/min ; Température : 60°C ; injection : 1μL ; Longueur d'onde 320nm ; gradient :

| Temps en minutes | % A | % B |
|---|---|---|
| 0.01 | 50 | 50 |
| 11.00 | 0 | 100 |
| 13.00 | 0 | 100 |
| 16.00 | 50 | 50 |
| 20.00 | 50 | 50 |

Méthode 9 : LCMS

Instrument : LC/MS Ultimate 3000 RS/ amaZon X ; Colonne : Waters, Symmetry C18 50*2.1 mm 3.5 μm ; éluant A : eau/acide trifluroacétique 0,05% (v/v) ; éluant B : Acétonitrile ; débit 0.208mL/min ; Température : 60°C ; injection : 1μL; gradient :

| Temps en minutes | % A | % B |
|---|---|---|
| 0.01 | 98 | 2 |
| 4.00 | 0 | 100 |
| 6.70 | 0 | 100 |
| 14.00 | 98 | 2 |
| 20.70 | 98 | 2 |

Méthode 10 : HPLC

Instrument : HPLC Dionex Ultimate 3000 ; Colonne : ThermoFisher, Accucore C18 100 × 3 mm, 2,6 μm ; éluant A : eau ; éluant B : Acétonitrile ; débit 0.4mL/min ; Température : 25°C ; gradient :

| Temps en minutes | % A | % B |
|---|---|---|
| 0 | 100 | 0 |
| 3.00 | 100 | 0 |
| 20.00 | 10 | 90 |
| 25.00 | 10 | 90 |
| 26.00 | 100 | 0 |

(suite)

| Temps en minutes | % A | % B |
|---|---|---|
| 30.00 | 100 | 0 |

Méthode 11 : HPLC Prep (purification sur colonne préparative)

Instrument : PuriFlash F4250 ; Colonne : Waters, Symmetry C18 150*30mm 5 µm; éluant A : eau/acide trifluroacétique 0,05% (v/v) ; éluant B : Acétonitrile ; débit 40mL/min; Température : ambiante ; injection : 2mL

**EXEMPLE 1** : **synthèse du macrocycle monoalkylé methyl 2-(3,6,9-triaza-1(2,6)-pyridinacyclodecaphane-3-yl)acetate**

Exemple 1a: Synthèse du 5-aza-1(1,4)-piperazina-3(2,6)-pyridinacycloheptaphane-12,13-dione

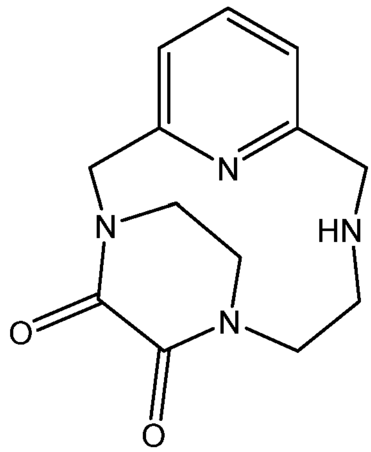

Chemical Formula: $C_{13}H_{16}N_4O_2$
Molecular Weight: 260,30

Le pyclen base (Inorganic Chemistry,Volume: 36, Issue: 14, Pages: 2992-3000; 10g, 0.047mol) est dissout dans 400mL de méthanol puis on ajoute en agitant une solution de diéthyloxalate (Sigma Aldrich, 1,01 équiv) dissout dans 200mL de méthanol sous une atmosphère inerte pendant 20 min. L'agitation du mélange se poursuit à température ambiante pendant 3 heures. Le solvant est ensuite évaporé sous vide.

Obtention d'un solide blanc m=12,7g
Rendement = quantitatif
Support : Oxide d'aluminium basique
Eluant : dichlorométhane/méthanol (9 :1)
Rf = 0,66
Méthode HPLC : 1
Tr (temps de rétention) = 9,0 min

Exemple 1b : synthèse du methyl 2-($1^2$,$1^3$-dioxo-5-aza-1(1,4)-piperazina-3(2,6)-pyridinacy-cloheptaphane-5-yl)acetate

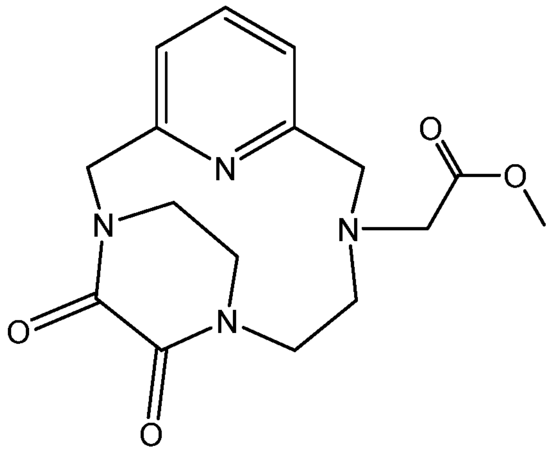

Chemical Formula: $C_{16}H_{20}N_4O_4$
Molecular Weight: 332,36

L'intermédiaire obtenu à l'exemple 1a (10.3g, 0.040 mol) est mis en suspension dans 260mL d'acétonitrile et 1,55 équivalent de $K_2CO_3$ sont ajoutés à la suspension. Le mélange ainsi formé est agité sous atmosphère inerte pendant 15 minutes. 1,01équiv de bromoacétate de méthyl (Aldrich ; référence : 147910-100G) mis en solution dans 260mL d'acétonitrile sont ajoutés au goutte à goutte sous atmosphère inerte pendant 30 minutes et laissée sous agitation pendant 3 heures. Le solvant est ensuite évaporé sous vide pour obtenir une huile.

L'huile brute est dissoute dans 970ml d'acétate d'éthyle et les sels sont extraits avec 35ml d'eau. La phase organique est séchée sur $Na_2SO_4$, filtrée, puis le solvant est évaporé.

On obtient un solide blanc m = 13,5g.

Rendement = quantitatif
Support : Oxide d'aluminium basique
Eluant : dichlorométhane/méthanol (9 :1)
Rf = 0,85
Méthode HPLC : méthode 1
Tr = 2,7 min

Exemple 1b' : synthèse du t-butyl-2-($1^2$,$1^3$-dioxo-5-aza-1(1,4)-piperazina-3(2,6)-pyridinacy-cloheptaphane-5-yl)acetate

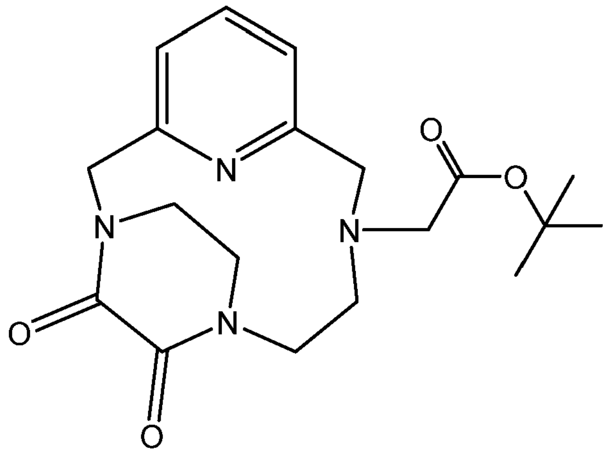

La synthèse se fait selon le mode opératoire décrit à l'exemple 1b en utilisant le bromacé-tate de t-butyle (124230-10G, Aldrich) à la place du bromoacétate de méthyl dans le procédé décrit à l'exemple 1b.

On obtient ainsi une huile jaune.

m = 710 mg.
Rendement = 99%.

Exemple 1c : synthèse du methyl 2-(3,6,9-triaza-1(2,6)-pyridinacyclodecaphane-3-yl)acetate

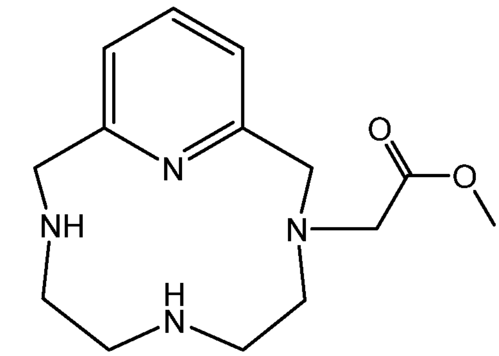

Chemical Formula: $C_{14}H_{22}N_4O_2$
Molecular Weight: 278,36

Une solution de 13,3g de l'intermédiaire décrit à l'exemple 1b et 11 mL d'acide sulfurique 98% dilués dans 265 mL de méthanol sont chauffés au reflux pendant 18H. La solution est ensuite refroidie à température ambiante avant d'ajouter 138 ml de résine Amberlyst® A21 (Aldrich ; référence 216410-1KG) puis laissée sous agitation pendant 30 minutes. La solution est filtrée et la résine est rincée au méthanol.

L'huile est lavée trois fois avec 50 ml d'éther éthylique pour éliminer le diméthyloxalate.

L'huile est solubilisée dans du dichlorométhane, séchée sur $MgSO_4$, puis filtrée et Le solvant est évaporé.

Le produit brut est purifié sur chromatographie flash en utilisant une cartouche d'oxyde d'aluminium neutre 440g avec un gradient en dichlorométhane/méthanol.

Obtention d'un solide blanc m=4,81g
Rendement = 43%
Support : Oxide d'aluminium neutre
Eluant : dichlorométhane/méthanol (9 :1)
Rf = 0,46
Méthode HPLC : 1
Tr = 6,6min

Les exemples 2, 3 et 4 suivants présentent la synthèse des alcynes.

**EXEMPLE 2** : **synthèse du 1-ethynyl-4-(10-phenyldec-1-yn-1-yl)benzene**

Exemple 2a : synthèse du dec-9-yn-1-ylbenzene

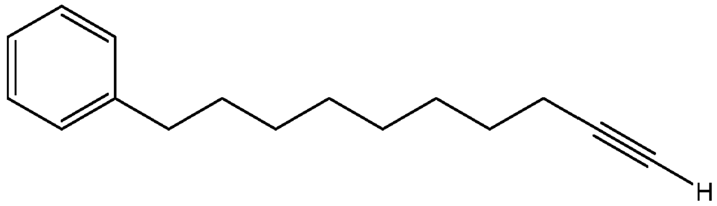

Chemical Formula: $C_{16}H_{22}$
Molecular Weight: 214,35

Le complexe de lithium acetylenediamine (Aldrich, reférénce 186155, 1444mg 2 équiv.) est dilué dans 66mL d'un mélange pentane/DMSO [7 :3]. La solution est agitée et dégazée deux fois à l'azote puis refroidie à 0°C. Le 1-bromo-8-phenyloctane (Interchim, pureté 95% ; référence OR8184 ; 2000mg ; 7,06mmol) en solution dans 7mL du mélange DMSO/$Et_2O$ [1 :1] est ajouté puis le milieu réactionnel est agité fortement à température ambiante pendant 22 heures. La solution est refroidie à 0°C puis 160mL de solution saturée en $NH_4Cl$ sont ajoutés avec précaution. Le produit est extrait avec 2x160mL de diéthyle éther, séché sur sulfate de sodium ($Na_2SO_4$), filtré et concentré à sec. L'huile jaune brute est purifiée par chromatographie flash avec une cartouche de silice (40g, Buchi ; référence 14000024) avec un gradient en heptane/dichlorométhane.

Obtention d'un liquide incolore

Rendement = 90%
Support : Silice
Eluant : Heptane
Rf = 0,34
Méthode LCMS : 2
Tr = 3,9min
$^1H$ RMN (300 MHz, $CDCl_3$) : $\delta$ 7.29 (m, 2H, phényl), 7.19 (m, 3H, phényl), 2.62 (t, $J$= 7.5Hz, 2H, R-$CH_2$-phényl), 2.20 (td, $J$= 6.9Hz & $J$= 2.6Hz, 2H, R-$CH_2$-alcyne), 1.95(t, $J$= 2.7Hz 1H, -C=CH), 1.67-1.35 (m, 12H, chaine lipophile).

Exemple 2b : synthèse du trimethyl((4-(10-phenyldec-1-yn-1-yl)phenyl)ethynyl)silane

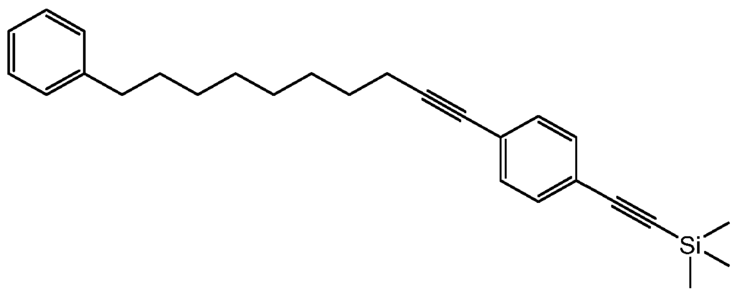

Chemical Formula: $C_{27}H_{34}Si$
Molecular Weight: 386,65

Le ((4-bromophenyl)ethynyl)trimethylsilane (2000mg, 7,90mmol ; Aldrich, référence 494011) est dilué dans 19mL de diisopropylamine, puis sont ajoutés le 1,1'-Bis(diphenyl-phosphino)ferrocene (0.02 équivalent ; Aldrich, référence 697230), l'iodure de cuivre (0.06 équivalent ; Aldrich, référence 03140) et la triphénylphosphine (0.04 équivalent ; Aldrich, référence T84409). La solution est dégazée sous atmosphère inerte puis est chauffée à 90°C. L'alcyne obtenu à l'exemple 2a (1,1 équivalent) est ajoutée à travers un septum puis le milieu réactionnel est agité à chaud pendant 19 heures. La solution est refroidie à température ambiante, filtrée sur papier de verre et les résidus sont rincés avec de l'éther diéthylique. Le filtrat est concentré à sec sous pression réduite. Le résidu est repris avec de l'éther diéthylique, lavé avec une solution de NaCl saturée, séché sur MgSOa, filtré et concentré à sec. Le liquide noir est adsorbé sur gel

de silice 60A (Merck ; référence : 1.09385.2500) et purifié sur une cartouche de silice (40g ; Buchi, référence 14000024) en éluant avec un mélange heptane/dichlorométhane.

On obtient un liquide incolore de masse m = 1418mg
Rendement = 93%
Support : Silice
Eluant : Heptane/Dichlorométhane (80 :20)
Rf = 0,27
Méthode LCMS : 2
Tr = 5,9min
$^{1}H$ RMN (60 MHz, $CDCl_3$) : $\delta$7.31 & 7.18 (m, 9H, phényl), 2.62 & 2.26 (m, 4H, R-$CH_2$-phényl & R-$CH_2$-alcyne), 1,33 (m, 12H, 6$CH_2$ chaine alkyle), 0,22 (s, 9H, 3x$CH_3$ du TMS).

Exemple 2c : 1-ethynyl-4-(10-phenyldec-1-yn-1-yl)benzene

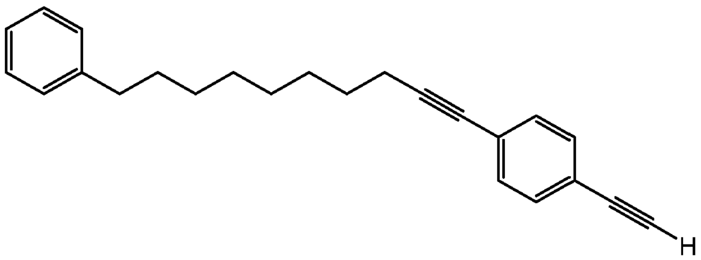

Chemical Formula: $C_{24}H_{26}$
Molecular Weight: 314,47

L'intermédiaire obtenu à l'exemple 2b (1418mg, 3,67mmol) est dilué avec 2,2mL de tétrahydrofurane anhydre. La solution est refroidie dans un bain eau/glace puis 4,4mL (1,2 equiv) de fluorure de tétrabutylammonium 1M dans le THF (Aldrich, référence 216143) sont ajoutés goutte à goutte à travers un septum avec une seringue et une aiguille. Le milieu réactionnel est agité pendant 2 heures à température ambiante. Puis 12mL d'eau sont ajoutés avant de réaliser 3 extractions avec 20mL de diéthyle éther afin d'extraire le produit. Les phases organiques sont réunies, et séchées sur $Na_2SO_4$, filtrées et concentrées à sec. Le liquide jaune ainsi obtenu est purifié sur une cartouche de silice (40g, Buchi, référence 14000024) en éluant avec de l'heptane.

On obtient un liquide incolore de masse m = 773mg
Rendement = 67%
Support : Silice
Eluant : Heptane/Dichlorométhane (80 :20)
Rf = 0,43
Méthode LCMS : 2
Tr = 4,9min
$^{1}H$ NMR (60 MHz, $CDCl_3$) : $\delta$7.47-7.18 (m, 9H, phényl), 3,08 (s, 1H, CH alcyne), 2.70-2.27 (m, 4H, R-$CH_2$-phényl & R-$CH_2$-alcyne), 1,33 (m, 12H, 6x$CH_2$ chaine alkyle).

**EXEMPLE 3** : **synthèse du 1-ethynyl-4-octylbenzene**

Exemple 3a : synthèse du trimethyl((4-octylphenyl)ethynyl)silane

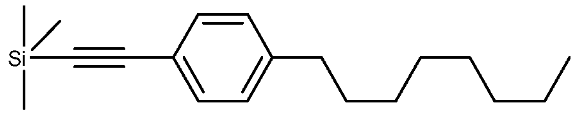

Chemical Formula: $C_{19}H_{30}Si$
Molecular Weight: 286,53

Le 1-bromo-4-n-octylbenzène (3000mg, 11.14mmol, 1 équiv. ; Alfa Aesar, référence A14676.06) est dilué dans 27mL de diisopropylamine, puis sont ajoutés 0.02 équivalent de 1,1'-Bis(diphenylphosphino)ferrocene (Aldrich, référence 697230), 0.06 équivalent d'iodure de cuivre (Aldrich, référence 03140) et 0.04 équivalent de triphénylphosphine (Aldrich, référence T84409). La solution est dégazée sous atmosphère inerte puis est chauffée à 85°C avant d'y ajouter le triméthylsilyléthyne (1,1 équiv ; Aldrich, référence 218170) à travers un septum. Le milieu réactionnel est chauffé pendant 19 heures puis refroidi à température ambiante, filtré sur papier de verre et les sels sont rincés avec de l'éther diéthylique. Le filtrat est concentré à sec sous pression réduite. Le résidu est repris avec de l'éther diéthylique puis lavé avec une solution de NaCl saturée. La phase organique est ensuite séchée sur $MgSO_4$, filtrée et le solvant est évaporé. On obtient un liquide noir qui sera adsorbé sur gel de silice 60 (Merck, référence 1.09385.2500) et purifié sur une cartouche de silice 80g avec un gradient d'éluant heptane/dichlorométhane.
On obtient un liquide jaune m = 2371mg
Rendement = 74%
Support : Silice
Eluant : Heptane
Rf = 0,43
Méthode LCMS : méthode 3
Tr = 9,9min
IR :

| Liaison | Type de liaison | Type de spécifique de liaison | Pic d'absorption $cm^{-1}$ | Apparence |
|---|---|---|---|---|
| $\nu$ C-C | C≡C | Alcyne disubstitué | 2157$cm^{-1}$ | Pic faible |

Exemple 3b : synthèse du 1-ethynyl-4-octylbenzene

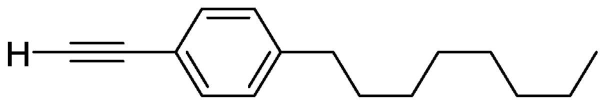

Chemical Formula: $C_{16}H_{22}$
Molecular Weight: 214,35

L'intermédiaire obtenu dans l'exemple 3a (2254mg, 7.87mmol) est dilué dans 11,8mL de THF anhydre puis le milieu est conditionné à l'azote. La solution est refroidie dans un bain eau/glace puis 9,4mL (1,2 équivalents) de fluorure de tétrabutylammonium 1M dans le THF (Aldrich, référence 216143) sont ajoutés goutte à goutte à travers un septum. Le milieu réactionnel est agité pendant 2 heures à température ambiante. En fin de réaction 40mL d'eau sont ajoutés. Le produit est extrait avec 80mL de diéthyle éther. La phase organique est séchée sur $Na_2SO_4$, filtrée et le solvant est évaporé. Obtention d'un liquide jaune brut qui sera purifié avec une cartouche de silice (80g, Buchi, référence 140000025) avec l'éluant heptane.

On obtient un liquide incolore m = 1162mg
Rendement = 68%
Support : Silice
Eluant : Heptane
Rf = 0,42
Méthode LCMS : 3
Tr = 8,2min

Infrarouge :

| Liaison | Type de liaison | Type de spécifique de liaison | Pic d'absorption $cm^{-1}$ | Apparence |
|---|---|---|---|---|
| ν C-H | C≡C | Alcyne vrai | $3297cm^{-1}$ | Pic moyen |

**EXEMPLE 4 : synthèse du 4-ethynyl-4'-octyl-1,1'-biphenyl**

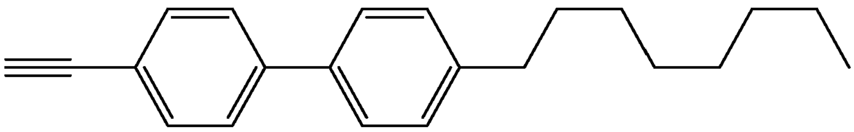

Chemical Formula: $C_{22}H_{26}$
Molecular Weight: 290,45

Le 1-bromo-4-n-octylbenzène (592mg, 2.2 mmol; Alfa Aesar, référence A14676.06), le 4-((trimethylsilyl)ethynyl)benzeneboronic acid pinacol ester (1321mg, 2 équiv. ; Interchim, H51697) et le $Cs_2CO_3$ (3,48 eq ; Alfa Aesar, référence 10924) sont dilués dans 10 mL de tétrahydrofurane et 4mL d'eau puis la solution est purgée à l'azote. L'acétate de palladium II (0.04 équiv., Aldrich référence : 520764), et la triphénylphosphine (0.02 équiv.) sont ajoutés puis le milieu réactionnel est chauffé à 70°C pendant 20 heures à l'abri de la lumière. La solution est refroidie à température ambiante, extraite à l'éther diéthylique puis la phase organique est séchée sur $Na_2SO_4$, filtrée et le solvant est évaporé. L'huile noire est purifiée par chromatographie flash sur une cartouche de silice (40g, Buchi ; référence : 140000024) avec l'éluant heptane/AcOEt. On obtient un solide blanc/jaune après évaporation. L'intermédiaire purifié obtenu (980mg, 2.70mmol) est dilué dans 2,70mL de THF anhydre puis le milieu est conditionné à l'azote. La solution est refroidie dans un bain eau/glace puis 4,59mL (1,7 eq) le TBAF à 1M dans le THF sont ajoutés goutte à goutte à travers un septum. Le milieu réactionnel est agité pendant 2 heures à température ambiante. En fin de réaction 10mL d'eau sont ajoutés. Le produit est extrait avec 40mL de diéthyle éther. La phase organique est séchée sur $Na_2SO_4$, filtrée et le solvant est évaporé. Obtention d'un solide qui sera purifié avec une cartouche de silice (40g, Buchi) avec l'éluant heptane/Acétate d'éthyle.

Obtention d'un solide blanc/jaune. M = 784mg
Rendement =55%

| Liaison | Type de liaison | Type de spécifique de liaison | Pic d'absorption $cm^{-1}$ | Apparence |
|---|---|---|---|---|
| ν C-H | C≡C | Alcyne vrai | $3306cm^{-1}$ | Pic moyen |

Les exemples 5, 6, 7 et 8b suivants illustrent la réaction de Sonogashira et la synthèse des picolinates lipophiles.

**EXEMPLE 5 : methyl 6-(hydroxymethyl)-4-((4-(10-phenyldec-1-yn-1-yl)phenyl)ethynyl)picolinate**

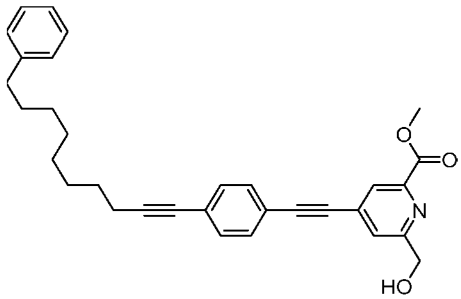

Chemical Formula: $C_{32}H_{33}NO_3$
Molecular Weight: 479,62

Le methyl 4-bromo-6-(hydroxymethyl)picolinate (890mg, 3.621mmol ; 1 équiv.; Interchim, reference 20210326 ; synthèse décrite dans le brevet WO 2017/109217 page 44) est dilué dans 22mL de diméthylformamide . La solution est conditionnée sous atmosphère inerte puis sont ajoutés 7mL de triéthylamine, le $Pd(PPh_3)_2Cl_2$ (0,05 équiv.), la $PPh_3$ (0,1 équiv.) et du CuI (0,1 équiv.). Après quelques minutes d'agitation, l'alcyne obtenu dans l'exemple 2c (1-ethynyl-4-(10-phenyldec-1-yn-1-yl)benzene) (1,2 équiv.) est ajouté au milieu réactionnel et la solution est chauffée à 110°C. La solution refroidie à température ambiante puis sont ajoutés 100mL de diéthyle éther. La phase organique est lavée avec 50mL de solution saturée en $NH_4Cl$ puis par 50mL de solution saturée en NaCl puis elle est séchée avec du $Na_2SO_4$, filtrée et concentrée à sec. Obtention d'une huile noire qui sera adsorbée sur gel de silice 60A et purifiée par chromatographie flash avec une cartouche de silice 80g et le mélange Heptane/AcOEt.

Obtention d'un solide beige m =923mg
Rendement = 53%
CCM : Silice
Eluant : Heptane/AcOEt (4 :6)
Rf = 0.3
HPLC : Méthode 4
Tr = 16.4 min m/z (ES +) = 480,31
RMN : $^1H$ NMR (60 MHz, $CDCl_3$) : $\delta$8.19 et 7.72 (m, 2H, pyridine), 7.67-7.31 (m, 9H, phenyl), 4,97 (s, 2H, $CH_2OH$), 4.10 (s, 3H, $CH_3$ ester), 3.47 (m, 1H, OH alcool primaire), 2.82-2.43 (m, 4H, R-$CH_2$-phényl & R-$CH_2$-alcyne), 1,47 (m, 12H, 6$CH_2$ chaine alkyle).

**EXEMPLE 6** : **synthèse du methyl 6-(hydroxymethyl)-4-((4-octylphenyl)ethynyl)picolinate**

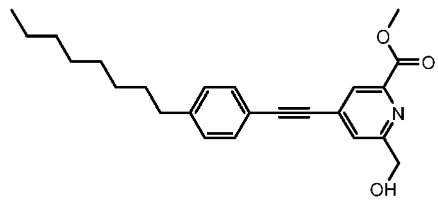

Chemical Formula: $C_{24}H_{29}NO_3$
Molecular Weight: 379,50

Le methyl 4-bromo-6-(hydroxymethyl)picolinate (1500mg, 6,10mmol ; 1 équiv. ; Interchim, reference 20210326 ; synthèse décrite dans la demande de brevet WO 2017/109217 en page 44) est dilué dans 37mL de tétrahydrofurane anhydre. Puis on réalise successivement deux dégazages à l'azote avant d'ajouter 12mL de triéthylamine, du $Pd(PPh_3)_2Cl_2$ (0,05 équiv.), de la $PPh_3$ (0,1 équiv.) et du CuI (0,1 équiv.). Après quelques minutes d'agitation, l'alcyne obtenue dans l'exemple 3b, (1-ethynyl-4-octylbenzene) (1,2 équiv.), est ajouté au milieu réactionnel et la solution est chauffée à 40°C. La solution est refroidie à température ambiante, filtrée sur papier de verre (Whatman) et les résidus sont rincés avec 100mL d'$Et_2O$. Le filtrat est lavé avec 100mL de solution saturée en $NH_4Cl$ et 40mL de solution saturée en NaCl puis la phase organique est séchée sur $Na_2SO_4$, filtrée et concentrée à sec. On obtient une huile noire qui sera adsorbée sur gel de silice 60A et purifiée par chromatographie flash avec une cartouche de silice (Buchi ; 80g) et le

mélange Heptane/AcOEt.

Obtention d'un solide blanc m= 1852mg
Rendement = 80%
CCM : Silice Heptane/AcOEt (8 :2)
Rf = 0.45
HPLC : Méthode 4
Tr = 15.3 min
m/z (ES+) = 380.28

**EXEMPLE 7** : **synthèse du methyl 6-(hydroxymethyl)-4-((4'-octyl-[1,1'-biphenyl]-4-yl)ethynyl)picolinate**

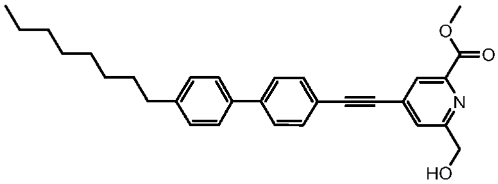

Chemical Formula: $C_{30}H_{33}NO_3$
Molecular Weight: 455,60

Le methyl 4-bromo-6-(hydroxymethyl)picolinate (4,88mmol ; 1 équiv. ; Interchim, reference 20210326 ; synthèse décrite dans la demande de brevet WO 2017/109217 en page 44), le THF anhydre (6,1mUmmol) et après 2 dégazages à l'azote, l'alcyne obtenu dans l'exemple 4 (4-ethynyl-4'-octyl-1,1'-biphenyl) (1,1 équiv.), la $Et_3N$ (2mUmmol), le $Pd(PPh_3)_2Cl_2$ (0,1 équiv.) et le CuI(0,1 équiv) sont mélangés. La solution devient noire et le milieu réactionnel est agité à 40°C en milieu inerte. La solution est refroidie à température ambiante, filtrée sur papier de verre (Whatman), les résidus sont rincés avec 100mL d'$Et_2O$. La phase organique est lavée deux fois avec 100mL de solution saturée en $NH_4Cl$ et 1×100mL de solution saturée en NaCl. Elle est ensuite séchée sur $Na_2SO_4$, filtrée et concentrée à sec. Obtention d'une huile noire qui sera adsorbée sur silice et purifiée par chromatographie flash avec une cartouche de silice et le mélange Heptane/AcOEt.

Obtention d'un solide beige m= 1668mg
Rendement = 75%
m/z (ES +) = 456

L'exemple 8 suivant présente la synthèse du composé obtenu à l'exemple 5, (methyl 6-(hydroxymethyl)-4-((4-(10-phenyldec-1-yn-1-yl)phenyl)ethynyl)picolinate), via une réaction de protection de l'alcool par acétylation.

**EXEMPLE 8** :

**Exemple 8a** : **synthèse du methyl 6-(acetoxymethyl)-4-bromopicolinate**

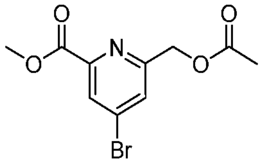

Chemical Formula: $C_{10}H_{10}BrNO_4$
Molecular Weight: 288,10

L'intermédiaire methyl 6-(hydroxymethyl)-4-bromopicolinate (25,409g ; 103,26mmol) est dilué dans 516mL de dichlorométhane puis le milieu est conditionné sous atmosphère d'azote. 26mL de triéthylamine sont ajoutés en une

fois et l'anhydride acétique (7,6 équiv ; Aldrich, 242845) est ajouté goutte à goutte via une ampoule à brome puis le milieu réactionnel est agité pendant 2 heures à température ambiante. La phase organique est lavée avec 50mL d'eau osmosée, séchée sur$Na_2SO_4$, filtrée et concentrée à sec.

On obtient un solide blanc m=33,65g.
Rendement quantitatif
Support : Silice gel
Eluant : Heptane/AcOEt (4 :6)
Rf= 0.6
Méthode HPLC : 5Tr = 3,56min
m/z (ES +) = 288
RMN : $^1H$ NMR (60 MHz, $CDCl_3$) : $\delta$ 8.19 et 7.72 (m, 2H, pyridine), 5,35 (s, 2H, $CH_2Ac$), 4.07 (s, 3H, $CH_3$ ester), 2,25 (s, 3H, $CH_3$ acétate)

**Exemple 8b : synthèse du methyl 6-(acetoxymethyl)-4-((4-(10-phenyldec-1-yn-1-yl)phenyl)ethynyl)picolinate**

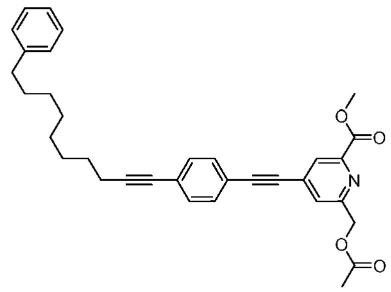

Chemical Formula: $C_{34}H_{35}NO_4$
Molecular Weight: 521,66

Le composé obtenu à l'exemple 8a (20,53g, 71,36mmol) est dilué dans 800mL de tétrahydrofurane puis la solution est conditionnée sous atmosphère inerte. De la triphenylphosphine(0,1 équiv), du $Pd(PPh_3)_2Cl_2$(0,05 équiv), du CuI (0,1 équiv) et 143mL $Et_3N$ sont ajoutés à la solution. Le composé obtenu à l'exemple 2c en solution dans 200mL de tétrahydrofurane est ajouté en une fois au milieu réactionnel puis la solution est chauffée à 60°C pendant 50min. Le milieu réactionnel est refroidi à température ambiante, filtré et rincé avec 250mL de tétrahydrofurane. Le solvant est évaporé sous vide. Le produit brut est alors remis en solution dans 910mL de diéthyle éther, filtré puis_la phase organique est lavée avec 500mL de solution saturée en $NH_4Cl$, 500mL de solution saturée en $Na_2CO_3$ et 250mL de solution saturée en NaCl. La phase organique est séchée sur $Na_2SO_4$, filtrée puis concentrée sous vide. Obtention d'un solide marron qui est recristallisé dans un mélange heptane/AcOEt avec une filtration à chaud.

Le solide est ensuite rincé avec 100mL d'AcOEt froid afin d'obtenir un solide blanc m=35,58g

Rendement = 96%
Support : Silice gel
Eluant : Heptane/AcOEt (4 :6)
Rf = 0.65
Méthode HPLC : Tr = 8.15min
m/z (ES+) = 522
RMN : $^1H$ NMR (60 MHz, $CDCl_3$) : $\delta$ 8.11 et 7.57 (m, 2H, pyridine), 7.42-7.19 (m, 9H, phenyl), 5,30 (s, 2H, $CH_2Ac$), 3.99 (s, 3H, $CH_3$ ester), 2.59-2.30 (m, 4H, R-$CH_2$-phényl & R-$CH_2$-alcyne), 2,17 (s, 3H, $CH_3$ acétate), 1,35 (m, 12H, $6CH_2$ chaine alkyle).

**Exemple 8c : synthèse du composé selon l'exemple 5 (methyl 6-(hydroxymethyl)-4-((4-(10-phenyldec-1-yn-1-yl)phenyl)ethynyl)picolinate) à partir de l'intermédiaire obtenu dans l'exemple 8b**

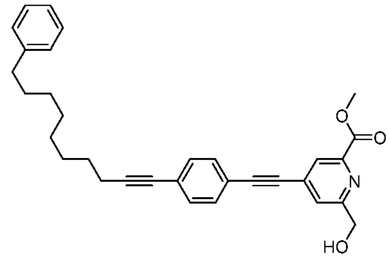

Chemical Formula: $C_{32}H_{33}NO_3$
Exact Mass: 479,25
Molecular Weight: 479,62

L'intermédiaire obtenu à l'exemple 8b (33,36g, 63.95mmol) est dilué dans 255mL de méthanol. Ajout de 27mL de triéthylamine (3 équiv) puis le milieu réactionnel est chauffé au reflux pendant 23 heures. Le milieu réactionnel est filtré puis le filtrat est refroidi dans un bain acétone/carboglace. On obtient après filtration un solide blanc de masse m=22,91g
Rendement = 75%

L'exemple 9 suivant illustre les réactions d'activation de l'alcool sous forme de mésylate.

**EXEMPLE 9** : **synthèse des methyl-4-6-(((methylsulfonyl)oxy)methyl)picolinate substitués**

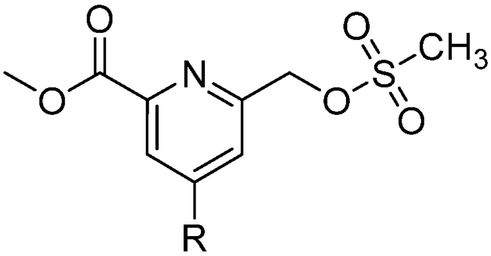

R:

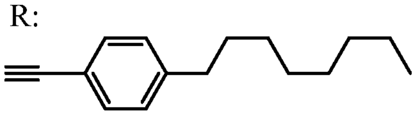

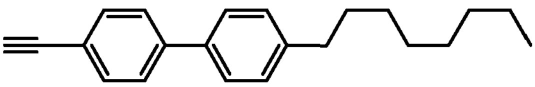

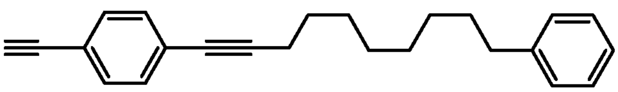

| | Structure | Nomenclature | Formule brute |
|---|---|---|---|
| Exemple 9a | R : 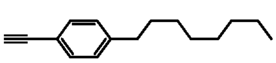 | methyl 6-(((methyl-sulfonyl)oxy)methyl)-4-((4-octylphenyl)ethynyl)picolinate | C25H31NO5S |
| Exemple 9b | R : 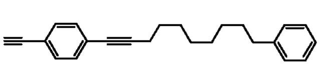 | methyl 6-(((methyl-sulfonyl)oxy)methyl)-4-((4-(10-phenyldec-1-yn-1-yl)phenyl) ethynyl)picolinate | C33H35NO5S |

(suite)

| | Structure | Nomenclature | Formule brute |
|---|---|---|---|
| Exemple 9c | R : 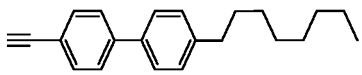 | methyl 6-(((methyl-sulfonyl)oxy)methyl)-4-((4'-octyl-[1,1'-biphenyl]-4-yl)ethynyl) picolinate | C31H35NO5S |

L'intermédiaire obtenu dans l'exemple 5, 6 ou 7 (1,64mmol, 1 équiv.) est dilué dans 18mL de DCM (11mUmmol). La solution est conditionnée sous atmosphère inerte puis est refroidie dans un bain eau/glace. On ajoute de la triéthylamine (3 équiv) et du chlorure de mésyle (1,5 équiv.) au goutte à goutte. Le milieu réactionnel est agité pendant 10min puis 18mL de solution saturée de NaHCOs sont ajoutés pour mettre fin à la réaction. La phase organique est récupérée, séchée sur $Na_2SO_4$ et concentrée à sec. Obtention d'un solide jaune qui sera purifié sur chromatographie flash sur une cartouche de silice avec le mélange Heptane/AcOEt.

Les résultats sont présentés dans le tableau suivant :

| | **Exemple 9a** | **Exemple 9b** | **Exemple 9c** |
|---|---|---|---|
| Masse molaire | 457,59 | 557,71 | 533,68 |
| Rendement | 97% | 97% | 63% |
| Méthode LCMS | Méthode 8 | Méthode 4 | Méthode 9 |
| Tr | 8.1 min | 9.7 min | ND |
| m/z (ES +) | 458 | 558.31 | 534 |
| a : RMN : $^1$H NMR (60 MHz, $CDCl_3$) : $\delta$ 8.20 et 7.76 (m, 2H, pyridine), 7.48-7.24 (m, 9H, phenyl), 5,45 (s, 2H, $CH_2OH$), 4.04 (s, 3H, $CH_3$ ester), 3,19 (s, 3H, $CH_3$ mésyle), 2.75-2.36 (m, 4H, R-$CH_2$-phényl & R-$CH_2$-alcyne), 1,40 (m, 12H, 6$CH_2$ chaine alkyle). | | | |

L'exemple 10 suivant illustre les réactions d'alkylation du composé obtenu à l'exemple 1c avec les réactifs mésylés décrits à l'exemple 9.

**EXEMPLE 10** : **Alkylation de l'intermédiaire obtenu à l'exemple 1c (methyl 2-(3,6,9-triaza-1(2,6)-pyridinacyclodecaphane-3-yl)acetate)**

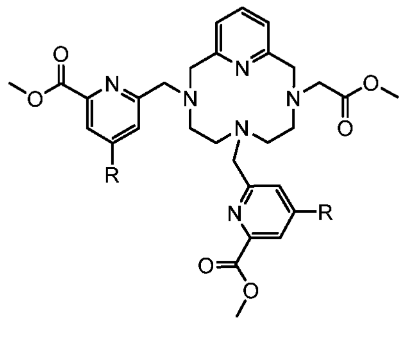

R:

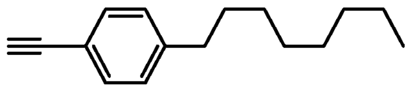

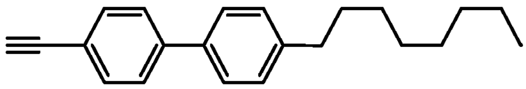

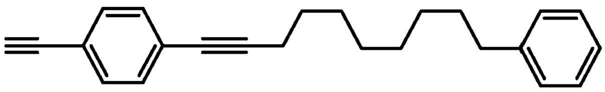

| | Structure | Nom | Formule brute |
|---|---|---|---|
| Exemple 10a | R : 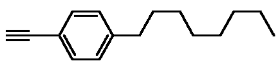 | dimethyl 6,6'-((9-(2-methoxy-2-oxoethyl)-3,6,9-triaza-1(2,6)-pyridina-cyclodecaphane-3,6-diyl)bis(methylene))bis(4-((4-octylphenyl) ethynyl)picolinate) | C62H76N6O6 |
| Exemple 10b | R : 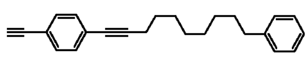 | dimethyl 6,6'-((9-(2-methoxy-2-oxoethyl)-3,6,9-triaza-1(2,6)-pyridina-cyclodecaphane-3,6-diyl)bis(methylene))bis(4-((4-(10-phenyldec-1-yn-1-yl)phenyl)ethynyl) picolinate) | C78H84N6O6 |
| Exemple 10c | R : 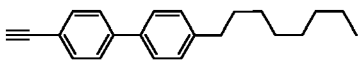 | dimethyl 6,6'-((9-(2-methoxy-2-oxoethyl)-3,6,9-triaza-1(2,6)-pyridina-cyclodecaphane-3,6-diyl)bis(methylene))bis(4-((4'-octyl-[1,1'-biphenyl]-4-yl)ethynyl)picolinate) | C74H84N6O6 |

Le composé obtenu à l'exemple1c (methyl 2-(3,6,9-triaza-1(2,6)-pyridinacyclodecaphane-3-yl)acetate) (462mg, 1,66mmol, 1 équivalent) est dilué dans 30mL d'acétonitrile anhydre puis, sont ajoutés le carbonate de calcium calciné (2,5 équivalents) et l'intermédiaire obtenu à l'exemple 9 (9a ou 9b ou 9c) (2,1 équivalents). Le milieu réactionnel est chauffé à 60°C sous agitation. La solution est ensuite refroidie à température ambiante et filtrée. Les sels sont rincés avec de l'acétonitrile puis le filtrat est concentré à sec. Une huile orange est obtenue qui sera purifiée par flash chromatographie avec une cartouche de silice avec le mélange DCM/MeOH.

| | **Exemple 10a** | **Exemple 10b** | **Exemple 10c** |
|---|---|---|---|
| Masse | 1001,33 | 1201,57 | 1153.52 |
| Rendement | 45% | 50% | 23% |
| CCM éluant | DCM/MeOH 8 :2, Silice Rf=0.78 | DCM/MeOH 9 :1, Silice Rf=0.38 | DCM/MeOH 8:2, Silice |
| Méthode LCMS | Méthode 4 | Méthode 4 | Méthode 4 |
| Tr | 16.1 min | 14.3 min | 14.4min |
| m/z (ES +) | 1002 | 1202 | 1153,7 |

L'exemple 11 suivant illustre l'obtention des ligands lipophiles.

**EXEMPLE 11 : Saponification des intermédiaires obtenus à l'exemple 10, pour obtenir les composés de formule (I)**

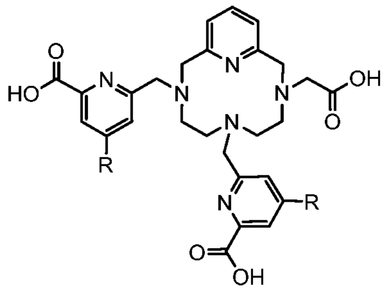

(I)

Les groupements R sont choisis parmi :

R:

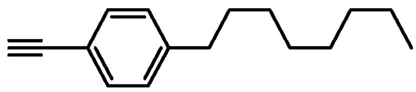

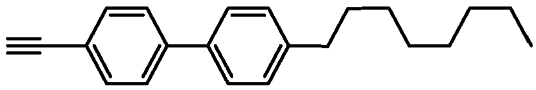

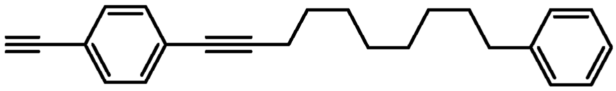

| Composés de formule (I) selon l'invention | Structure | Nomenclature | Formule brute |
|---|---|---|---|
| Exemple 11a | R : 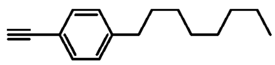 | 6,6'-((9-(carboxymethyl)-3,6,9-triaza-1 (2,6)-pyridina-cyclodecaphane-3,6-diyl) bis(methylene))bis(4-((4-octylphenyl) ethynyl)picolinic acid) | $C_{59}H_{70}N_6O_6$ |
| Exemple 11b | R : 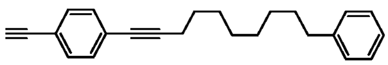 | 6,6'-((9-(carboxymethyl)-3,6,9-triaza-1 (2,6)-pyridina-cyclodecaphane-3,6-diyl) bis(methylene))bis(4-((4-(10-phenyldec-1-yn-1-yl)phenyl)ethynyl)picolinic acid) | $C_{75}H_{78}N_6O_6$ |
| Exemple 11c | R : 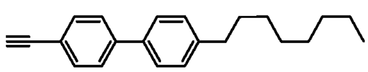 | 6,6'-((9-(carboxymethyl)-3,6,9-triaza-1 (2,6)-pyridina-cyclodecaphane-3,6-diyl) bis(methylene))bis(4-((4'-octyl-[1,1'-biphenyl]-4-yl)ethynyl)picolinic acid) | $C_{71}H_{78}N_6O_6$ |

L'intermédiaire obtenu à l'exemple 10 (10a ou 10b ou 10c) (1.00 mmol, 1 équivalent) est dilué dans 10mL de solution d'hydroxyde de potassium 2M dans l'éthanol. Le milieu réactionnel est agité à température ambiante pendant 15 min. Ajout de 15mL de DCM puis la solution est refroidie dans un bain eau/glace avant d'ajouter de l'acide chlorhydrique à 30% sans métaux jusqu'à obtenir un pH 6-7 et un précipité. Les sels sont filtrés, rincés avec du DCM puis le filtrat est concentré à sec. Il est obtenu un solide jaune brut qui sera purifié sur colonne préparative sauf pour le composé 11c qui sera laissé brut.

Les fractions enrichies en produit d'intérêt sont réunies, concentrées sous vide puis lyophilisées.

| | Exemple 11a | Exemple 11b | Exemple 11c |
|---|---|---|---|
| Masse molaire | 959,25 | 1159,49 | 1111,44 |
| Rendement | 41% (purifié) | 26% (purifié) | 42% (brut) |
| Méthode LCMS | Méthode 4 | Méthode 4 | Méthode 9 |
| Tr | 13.3 min | 13,93 min | 9.0 min |
| m/z (ES +) | 959.6 | 1159.7 | 1111.6 |

Les exemples 12, 13, 14 et 15 illustrent des réactions de complexations avec différents métaux d'intérêt.

**EXEMPLE 12** : **synthèses de complexes de formule (III) selon l'invention**

**Exemple 12a : synthèse du complexe métal(III) 6,6'-((9-(carboxylatomethyl)-3,6,9-triaza-1(2,6)-pyridinacyclodecaphane-3,6-diyl)bis(methylene))bis(4-((4-(10-phenyldec-1 -yn-1 -yl)phenyl)ethynyl)picolinate)**

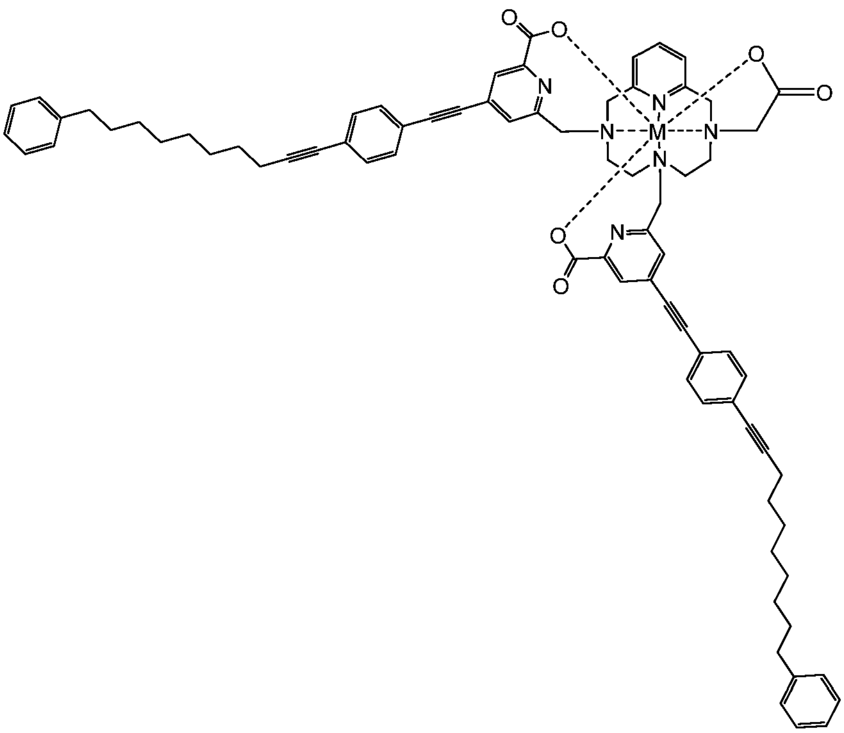

| Complexes de formule (III) selon l'invention | Réactif | M | Nomenclature | Formule brute |
|---|---|---|---|---|
| Exemple 12a | $YCl_3.6 (H_2O)$ Strem Chemicals Ref : 93-3903 | $Y^{89}$ | Yttrium(III) 6,6'-((9-(carboxylatomethyl)-3,6,9-triaza-1(2,6)-pyridina-cyclodecaphane-3,6-diyl)bis (methylene))bis(4-((4-(10-phenyldec-1-yn-1-yl) phenyl)ethynyl)picolinate) | C75H75N6O6Y |

(suite)

| Complexes de formule (III) selon l'invention | Réactif | M | Nomenclature | Formule brute |
|---|---|---|---|---|
| Exemple 12b | $LuCl_3$.6 ($H_2O$) Strem Chemicals Ref : 93-7111 | Nat. Lu | Lutetium(III) 6,6'-((9-(carboxylatomethyl)-3,6,9-triaza-1(2,6)-pyridinacyclodecaphane-3,6-diyl)bis (methylene))bis(4-((4-(10-phenyldec-1-yn-1-yl) phenyl)ethynyl)picolinate) | C75H75LuN6O6 |
| Exemple 12c | $TbCl_3$.6 ($H_2O$) Alfa Aesar Ref : 11210 | $Tb^{159}$ | Terbium(III) 6,6'-((9-(carboxylatomethyl)-3,6,9-triaza-1(2,6)-pyridinacyclodecaphane-3,6-diyl)bis (methylene))bis(4-((4-(10-phenyldec-1-yn-1-yl) phenyl)ethynyl)picolinate) | C75H75N6O6Tb |

50mg de ligand obtenu à l'exemple 11b sont dissous dans 13,3 mL de méthanol et le métal chlorhydrate hexa hydraté (1,5équiv) est ajouté au milieu. Le pH de la solution est ajusté à 6 en utilisant une solution de méthanolate de sodium (0,12M) puis le milieu réactionnel est agité à température ambiante 30min. La solution est concentrée à sec puis le solide brut est traité avec 4 mL de dichlorométhane. Le solvant est alors évaporé sous pression réduite afin d'obtenir un solide jaune.

| | Exemple 12a | Exemple 12b | Exemple 12c |
|---|---|---|---|
| Masse molaire | 1245,37 | 1331,43 | 1315,39 |
| Rendement | Quantitatif | Quantitatif | Quantitatif |
| Méthode HPLC | Méthode 7 | Infusion | Infusion |
| Tr | 7.05min | NA | NA |
| m/z (ES +) | 1246.6 | 1331.6 | 1315.5 |

**EXEMPLE 13** : **synthèse des complexes radiomarqués**

**Exemple 13a** : **synthèse du complexe d'Yttrium 90 du ligand préparé à l'étape 11b (6,6'-((9-(carboxymethyl)-3,6,9-triaza-1 (2,6)-pyridinacyclodecaphane-3,6-diyl)bis(methylene))bis(4-((4-(10-phenyldec-1-yn-1-yl)phenyl)ethynyl)picolinic acid))**

500 μL de solution du composé obtenu à l'exemple 11b (C = $10^{-3}$ mol/L) sont prélevés et déposés dans une fiole en verre SF8, 500μL d'une solution tamponnée d'acétate de sodium 3M pH5,2 sont rajoutés à un lot d'environ 629MBq (17mCi) de chlorure de [90Y] ([90Y]Cl) (Perkin Elmer). La solution radioactive (-500 μl) est alors récupérée et déposée dans la fiole contenant les 500μ! de solution de ligand. Le mélange est agité légèrement avant d'être incubé à 80°C durant 20 min. A la fin de la réaction, un prélèvement est effectué à l'aide d'une seringue à insuline et déposé sur une plaque de chromatographie sur couche mince (Chromatography paper 1CHR, GE). La quantité de radioactivité dans le flacon est mesurée à l'aide d'une chambre d'ionisation (activimètre VDC-405, model VIK202, utilisé avec le logiciel v3.29; Comecer Netherlands, Pays-Bas) préalablement calibrée pour la mesure de la radioactivité émise par la désintégration d'[90Y] conditionnée en flacon en verre et re-suspendue dans un milieu eau/éthanol.

Il est ensuite procédé à l'extraction dans le Lipiodol®.

1 mL de sérum physiologique (Mini-Plasco NaCl 0,9%, BBraun), puis 2 ml de Lipiodol® Ultra Fluide (Guerbet), sont ajoutés successivement au milieu réactionnel issu du radiomarquage contenu dans la fiole et cette dernière est agitée vigoureusement manuellement à l'aide d'une pince pour émulsifier. L'émulsion est ensuite brisée par centrifugation (2600g, 20 min) (Sigma 2-6, Fisher Bioblock Scientific) permettant l'obtention d'une phase inférieure huileuse, constituée de Lipiodol® (dite « phase Lipiodolique ») contenant le complexe radiomarqué et d'une phase supérieure contenant le mélange eau/éthanol. La quantité de radioactivité contenue dans le flacon est mesurée à l'aide de la même chambre d'ionisation préalablement calibrée pour la mesure de l'[$^{90}$Y] dans le Lipiodol®. La majorité de la phase supérieure est

aspirée à la seringue. La phase Lipidolique est ensuite récupérée à l'aide d'une aiguille 23G0, 60X25mm (Sterican, BBraun), attachée à une seringue de 1,0ml (1ml Syringe Luer BD Plastipak, BD) dans une fiole en verre SF8 pré-pesée.

La quantité de radioactivité contenue dans les flacons contenant respectivement la phase éthanol/ligand et la phase Lipiodolique est mesurée à l'aide de la chambre d'ionisation préalablement calibrée pour chacune des conditions de mesure. Le flacon contenant la phase Lipiodolique est pesé à l'aide d'une balance de précision (modèle TE64-0CE, Sartorius) permettant le calcul de l'activité volumique du radio traceur.

Calculs des rendements de synthèse du radio traceur, de sa pureté radiochimique et de son activité volumique :

Rendement de synthèse

Le rendement de synthèse du radio traceur ciblé a été établi comme devant être supérieur ou égal à 50%. Il a été calculé selon la formule suivante :

$$R=\frac{\textit{activité de la phase lipiodolique récupérée}}{\textit{activité engagée mesurée post-incubatio à 80°C)}}\ X\ 100\%$$

Pureté radiochimique (PRC) par chromatographie sur couche mince

Les plaques (Chromatography paper 1CHR, GE) sont éluées avec une solution de triéthylamine ($Et_3N$) 0,1 % en volume dans du méthanol (MeOH) jusqu'à ce que le front de migration atteigne le sommet de la feuille. La radioactivité présente sur les feuilles séchées est ensuite détectée à l'aide d'un phosphorimager FLA-7000 fonctionnant avec le logiciel d'acquisition du même nom (version 1.1, FujiFilm). La PRC du ligand radio marqué a été calculée à l'aide du programme Multi Gauge v3.1 (FujiFilm), sur base de la quantification de la radioactivité détectée sur la feuille où a migré le milieu réactionnel. En pratique cette quantification est basée sur la création de régions d'intérêt tracées sur chacune des zones associées à la radioactivité détectée. La PRC a été calculée comme suit :

$$PRC=\frac{\textit{radioactivité associée au ligand (PSL)}}{\textit{radioactivité correspondant à l'[90 ]libre présent dans le milieu réactionnel (PSL)}}\ X\ 100\%$$

Calcul de l'activité volumique

L'activité volumique est déterminée par pesée du flacon dans lequel a été récupérée la phase Lipiodolique contenant le radio traceur et par mesure de l'activité en [90Y] au moyen d'une chambre d'ionisation (l'activité mesurée est corrigée pour le bruit de fond cosmique ainsi que pour la décroissance physique de l'[$^{90}$Y] (« bruit de fond »). Elle sera calculée comme suit, en prenant en compte comme densité du Lipiodol® la valeur de 1,28g/mL :

$$\text{activité volumique (MBq/ml)} = \textit{activité massique}\ \left(\frac{MBq}{g}\right) X\ \textit{densité(lipiodol)}\left(\frac{g}{ml}\right)$$

avec $$\text{activité massique (MBq/g)} = \frac{\textit{activité (phase lipiodolique) (MBq)}}{\textit{masse lipiodol (g)}}$$ et

$$\text{masse Lipiodol (g)} = \textit{masse (fiole pleine) – masse (fiole vide, pré-pesée)}$$

et

masse Lipiodol® (g) = masse (fiole pleine) – masse (fiole vide, pré-pesée).

**Exemple 13b** : **complexe d'indium 111 du ligand obtenu à l'exemple 11b (6,6'-((9-(carboxymethyl)-3,6,9-triaza-1(2,6)-pyridinacyclodecaphane-3,6-diyl)bis(methylene))bis(4-((4-(10-phenyldec-1-yn-1-yl)phenyl)ethynyl)picolinic acid))**

Le même protocole que celui décrit à l'exemple 13a en utilisant une solution d'indium 111 (Curium, 4.71 mCi, 174 MBq) conduit à la formation du complexe d'indium 111.

**Exemple 13c** : **complexe de luthétium 177 du ligand obtenu à l'exemple 11b (6,6'-((9-(carboxymethyl)-3,6,9-triaza-1(2,6)-pyridinacyclodecaphane-3,6-diyl)bis(methylene))bis(4-((4-(10-phenyldec-1-yn-1-yl)phenyl)ethynyl)picolinic acid))**

Le même protocole que celui décrit à l'exemple 13a en utilisant une solution de lutétium 177(673 MBq) conduit à la formation du complexe de lutétium 177.

Résultats de marquage du Lipiodol®

| | Exemple 13a | Exemple 13b | Exemple 13c |
|---|---|---|---|
| Pureté Radio Chimique (%) | 99,2 | 95,5 | 94,1 |
| Rendement (%) | 99 | 94,5 | 87,4 |

**EXEMPLE 14 : complexe d'Yttrium 90 du composé obtenu à l'exemple 11c (6,6'-((9-(carboxymethyl)-3,6,9-triaza-1(2,6)-pyridinacyclodecaphane-3,6-diyl)bis(methylene))bis(4-((4'-octyl-[1,1'-biphenyl]-4-yl)ethynyl)picolinic acid))**

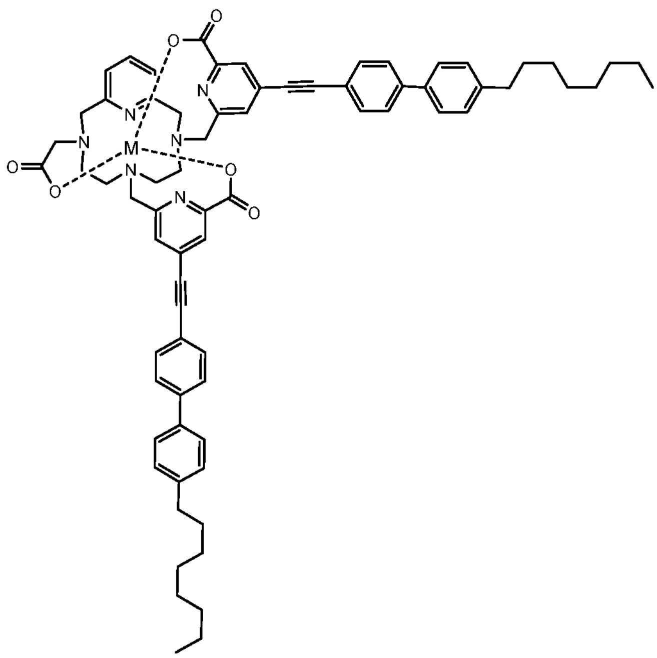

0,5 mL de chlorure d'yttrium-90 en solution tampon acétate pH = 5,2 sont ajoutés à 0,5 mL de ligand obtenu à l'exemple 11c en solution dans du DMSO à une concentration de $10^{-3}$ mol/L. La solution est chauffée 15 min à 80°C. 1 mL de sérum physiologique + 2 mL de Lipiodol® sont ajoutés et le mélange est agité vigoureusement. Les phases sont ensuite séparées par centrifugation (4500 tours/min, 20 min) et la phase huileuse est collectée pour obtenir le radiotraceur attendu.

| | |
|---|---|
| **PRC (%)** | 92,7 |
| **Rendement (%)** | 89,5 |

**EXEMPLE 15 : complexe d'Yttrium 90 du composé obtenu à l'exemple 11a (6,6'-((9-(carboxymethyl)-3,6,9-triaza-1(2,6)-pyridinacyclodecaphane-3,6-diyl)bis(methylene))bis(4-((4-octylphenyl)ethynyl)picolinic acid))**

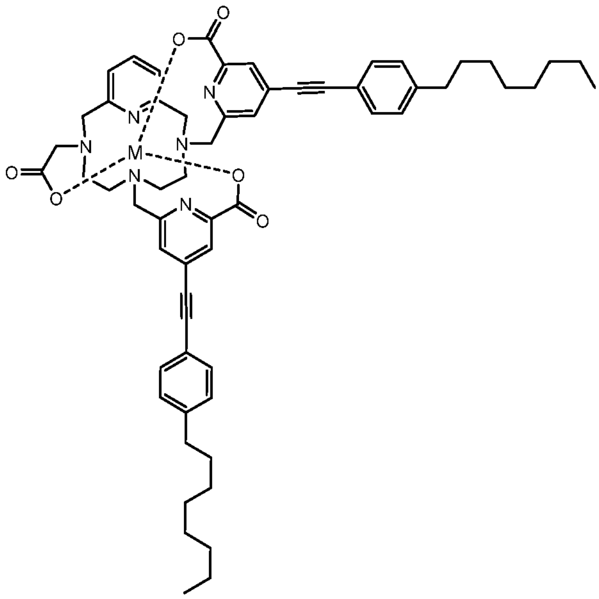

0,5 mL de chlorure d'yttrium-90 en solution tampon acétate pH = 5,2 sont ajoutés à 0,5 mL de ligand obtenu à l'exemple 11a en solution dans l'éthanol à une concentration de $10^{-3}$ mol/L. La solution est chauffée 15 min à 80°C. 1 mL de sérum physiologique + 2 mL de Lipiodol® sont ajoutés et le mélange est agité vigoureusement. Les phases sont ensuite séparées par centrifugation (3500 tours/min, 15 min) et la phase huileuse est collectée pour obtenir le radiotraceur attendu.

| | |
|---|---|
| **PRC (%)** | 99,7 |
| **Rendement (%)** | 92,9 |

**EXEMPLE 16** : **Etudes de stabilité**

**1/ Avec les composés selon l'invention** :

Les prototypes radiomarqués (Lipiodol® marqué) sont ensuite engagés dans des tests de stabilité, consistant en une période d'incubation à 37°C en présence de sérum (physiologique ou humain) et suivi cinétique du passage de la radioactivité de la phase huileuse vers la phase aqueuse. Ces résultats sont présentés sous forme de courbes de relargage indiquant sur l'axe des abscisses la durée d'incubation (en heures) et sur l'axe des ordonnées le taux de relargage (% de la radioactivité transféré vers la phase aqueuse).

1 mL de radiotraceur fraichement préparé (exemples 13a, 13b, 13c, 14, 15) est prélevé puis déposé dans un flacon en verre à fond plat de 12 mL. La radioactivité est mesurée à l'activimètre, et l'heure notée. 10 mL de sérum (physiologique ou humain) sont ajoutés avant agitation du mélange. Le flacon est ensuite placé dans l'incubateur à 37°C, avec un agitateur réglé à 30 rpm.

On laisse l'agitation pendant plusieurs jours. La phase aqueuse est prélevée à différents temps pour doser l'yttrium-90 (complexe radiomarqué de l'exemple 13a, de l'exemple 14 et de l'exemple 15) ; l'indium-111 (complexe radiomarqué de l'exemple 13b) ou le lutétium 177 (complexe radiomarqué de l'exemple 13c) relargué.

Les résultats de relargage d'yttrium 90 ou d'indium 111 pour les composés des exemples 13a et 13b respectivement dans la phase aqueuse (sérum physiologique) sont présentés dans la figure 1.
Les résultats de relargage d'yttrium 90 dans le sérum humain (phase aqueuse) pour les composés des exemples 13a, 14 et 15, sont présentés dans la figure 2.
Les résultats de relargage de lutétium 177 dans le sérum physiologique et dans le sérum humain pour le composé de l'exemple 13c, sont présentés dans la figure 7. Ces résultats montrent que le complexe de lutétium 177 de l'exemple 13c présente une stabilité dans les sérums équivalente à celle observée pour les composés des exemples 13a et 13b (complexes d'yttrium 90 et complexe d'indium 111.
Les figures 1, 2 et 7 montrent que les composés objets de l'invention sont stables dans le sérum physiologique et humain, c'est-à-dire que la radioactivité reste dans la phase Lipiodolée et ne fuit pas dans la phase aqueuse (moins de 30% de relargage pour les composés des exemples 14 et 15 et moins d'environ 10% de relargage pour les composés des exemples 13a, 13b et 13c), sur une durée d'au moins 350 h ce qui permet d'envisager leur utilisation en radiothérapie des tumeurs du foie.

**2/ Avec des composés comparatifs** :

Les composés comparatifs suivants ont été étudiés dans les mêmes conditions afin de pouvoir comparer leurs propriétés avec les composés de l'invention.

a) Structure des picolinates comparateurs:

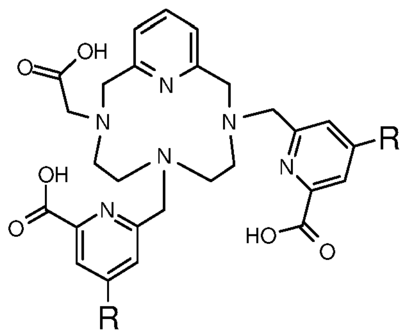

| | Comparateur 1 | Comparateur 2 |
|---|---|---|
| **R** | H | $C\equiv CH-(CH_2)_9CH_3$ |

| | Comparateur 1 | Comparateur 2 |
|---|---|---|
| **Radiomarquage 1 PRC (%)** | 98 | 98 |
| **Rendement Extraction 1 (%)** | 0 | 89,8 |
| **Radiomarquage 2 PRC (%)** | - | 98,9 |
| **Rendement Extraction 2 (%)** | - | 14,6 |
| **Radiomarquage 3 PRC (%)** | - | 69,7 |
| **Rendement Extraction 3 (%)** | - | 17 |

Le produit comparateur 1 dépourvu de résidu lipophile sur les motifs picolinates n'est pas extrait dans le Lipiodol®.
Les résultats obtenus avec le produit comparateur 2 indiquent que le radiomarquage et l'extraction dans du Lipiodol® ne sont pas reproductibles pour cette substance.
Ces résultats contrastent avec ceux décrits à l'exemple 17 avec des radiomarquages parfaitement reproductibles pour le composé décrit à l'exemple 11b.

De plus, la stabilité du comparateur 2 dans le sérum humain dans les conditions expérimentales décrites ci-dessus est montrée en figure 3 ; elle est significativement moins bonne que celle observée pour les produits selon l'invention (figure 2).

En effet sur la figure 3 on constate que plus de 50% de la radioactivité a déjà fui vers la phase aqueuse à T0 + 200 Heures.

| Composé | Comparateur 2 | Exemple 13a | Exemple 14 | Exemple 15 |
|---|---|---|---|---|
| Fuite de la radioactivité dans le sérum à 216H (%) | 58 | 3 | 21 | 12,5 |

Ceci indique que la structure du résidu lipophile R installé sur les motifs picolinates est critique pour obtenir des propriétés équivalentes (radiomarquage, reproductible et stabilité) aux composés de l'invention.

Lors de l'optimisation des résidus lipophiles des prototypes de structure apparentée ont été préparés et testés. Il est apparu que la structure du groupement R devait être réglée très finement afin d'obtenir des composés stables lors du test réalisé dans le sérum physiologique.

b) Structures des composés comparateurs 3, 4, 5 :

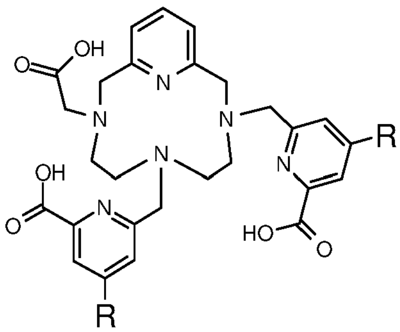

R=

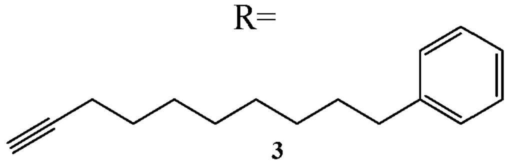

3

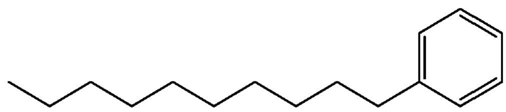

4

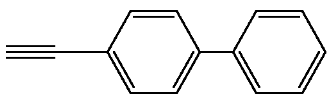

5

Les données d'extraction dans le Lipiodol® pour les différents composés préparés sont présentées dans le tableau suivant :

| | **Comparateur 3** | **Exemple 15** | **Comparateur 4** | **Comparateur 5** | **Exemple 14** | **Exemple 13a** |
|---|---|---|---|---|---|---|
| Extraction Lipiodol® (%) | 51,0 | 92,9 | 93,4 | 84,5 | 89,5 | 90,0 |

Les données de stabilité sont présentées sur la figure 4.

Les courbes de stabilité des composés de l'invention montrent un transfert de la radioactivité vers le sérum moins rapide et une amplitude plus faible que les trois composés comparateurs. En effet, aucun de ces trois composés comparateurs n'est stable dans le sérum, c'est à dire qu'ils présentent une fuite rapide et importante de radioactivité vers la phase aqueuse.

Cela indique que la structure de ces composés ne permet pas d'obtenir des phases huileuses radiomarquées stables.

**EXEMPLE 17** : **Etude de biodistribution du composé obtenu à l'exemple 13a**

Le radiomarquage est réalisé selon le mode opératoire décrit à l'exemple 13a.

L'yttrium 90 ($^{90}YCl$ dans HCl) est fourni par Perkin Elmer. La pureté radiochimique est supérieure à 95%.

| Temps | Lot | Rdt radiochimique (%) | Activité (MBq) | Activité Spécifique (MBq/g) | Activité volumique (MBq/ml) |
|---|---|---|---|---|---|
| 1H | [90Y] T1H | 96 | 226.6 | 157.7 | 201.9 |
| 24H | 90Y] T3H | 96 | 362.5 | 191.0 | 244.4 |
| J3 | 90Y] T3J | 96 | 350.6 | 159.5 | 204.2 |
| J6 | [90Y] T6J | 98 | 343.6 | 172.1 | 220.2 |

La qualité du radiomarquage obtenue pour chaque essai indique que le protocole est reproductible. La robustesse de ce radio-marquage a permis de produire les lots de radio-pharmaceutique pour les études *in vivo.* 56 rats femelles (Sprague-Dawley, agées de 8 à10 semaines, pesant entre 220 et 225g, Janvier France) sont acclimatés 5 jours (23°C, humidité 22%) avec accès libre à la nourriture et à l'eau de boisson.

Des cellules Novikoff N1S1 (ATCC, UK) sont utilisées pour induire un carcinome hepato-cellulaire chez les rats.

L'induction tumorale consiste à injecter les cellules N1S1 aux rats selon le protocole décrit dans la littérature *(*Garin E, Denizot B, Roux J et al. Description and technical pitfalls of a hepatoma model and of intra-arterial injection of radiolabeled Lipiodol in the rat. Lab Animals 2005; 39: 314-320*).*

Le complexe radiomarqué de l'exemple 13a est injecté via une canule (26G) préalablement insérée dans l'artère hépatique du rat. La dose injectée est d'environ 3.5 Mbq.

Les animaux sont sacrifiés à 1 heure, 24 heures 3 jours et 6 jours, le sang et les différents organes sont prélevés et pesés après dissection.

Les tubes contenant les organes sont comptés à l'aide d'un compteur gamma (Perkin Elmer, USA) calibré pour l'yttrium 90.

Les résultats figurent dans le tableau ci-dessous et en figure 5.

| | **1H** | **24H** | **3J** | **6jours** |
|---|---|---|---|---|
| % dose injectée à la tumeur | 29 ± 10 | 17 ± 10 | 23 ± 14 | 35 ± 18 |
| Rapport dose tumeur/foie sain | 5.4 ±2.6 | 3.3 ±1.9 | 4.9 ±3.4 | 8.5± 4.8 |

Distribution de la radioactivité dans le foie

L'étude de la biodistribution montre que le produit selon l'invention est bien capté par la tumeur avec un ratio tumeur/foie sain d'au moins 3 (voir Figure 5), et que la dose captée par le foie sain est faible car elle est d'environ 5% de la dose injectée. Cette sélectivité de la distribution pour la tumeur du composé radioactif permet d'envisager son utilisation en radiothérapie des tumeurs du foie.

Les mêmes expériences ont été réalisées avec le composé de l'exemple 13b (111 Indium). Les résultats montrent qu'on retrouve un rapport dose injectée de tumeur / foie sain supérieur à 5 aux délais de 1h et 6 jours, ce qui est cohérent avec les résultats observés pour le composé de l'exemple 13a. Cette sélectivité de la distribution pour la tumeur du composé radioactif de l'exemple 13b, permet d'envisager son utilisation en radiothérapie des tumeurs du foie.

Les pourcentages de dose injectée au fémur et à la moelle osseuse pour les composés de l'exemple 13a et de l'exemple 13b, à 1h et à 6 jours après injection sont en Figure 6. Ces résultats montrent que la dose résiduelle de radioactivité dans ces organes est extrêmement faible (inférieure à 0,05%). Ces résultats de faibles doses captées par ces organes sensibles permettent d'envisager l'utilisation de ce composé en radiothérapie des tumeurs du foie.

## Revendications

1. Composé de formule générale (I) suivante :

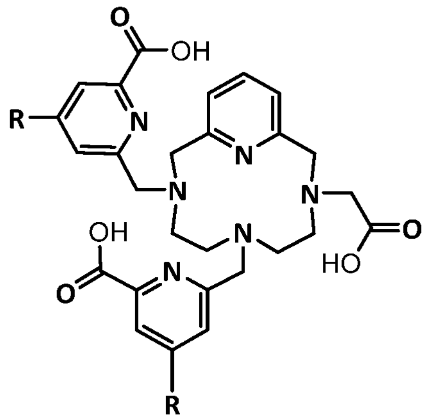

(I)

dans laquelle R est un groupement de formule (II) suivante:

-CΞC-Ph-L1-(CH2)n-L2 (II)

avec

- L1 représentant Ph ou -CΞC- ou CH2,
- L2 représentant H ou Ph ou alkylphényle,
- n compris entre 4 et 12,

ou l'un de ses sels pharmaceutiquement acceptables.

2. Composé de formule (I) selon la revendication 1, choisi parmi le groupe constitué des composés suivants :

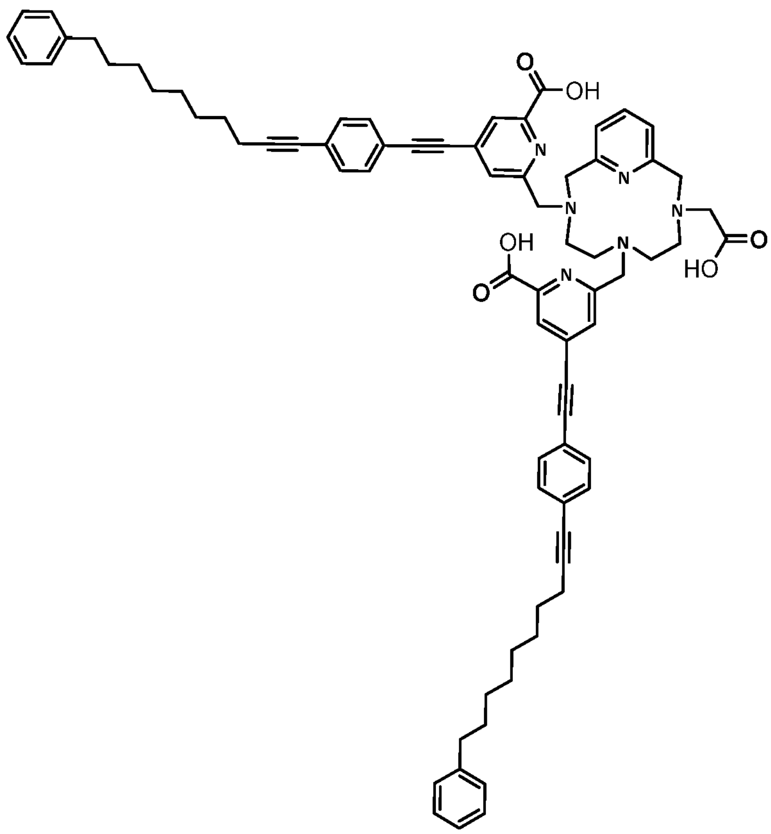

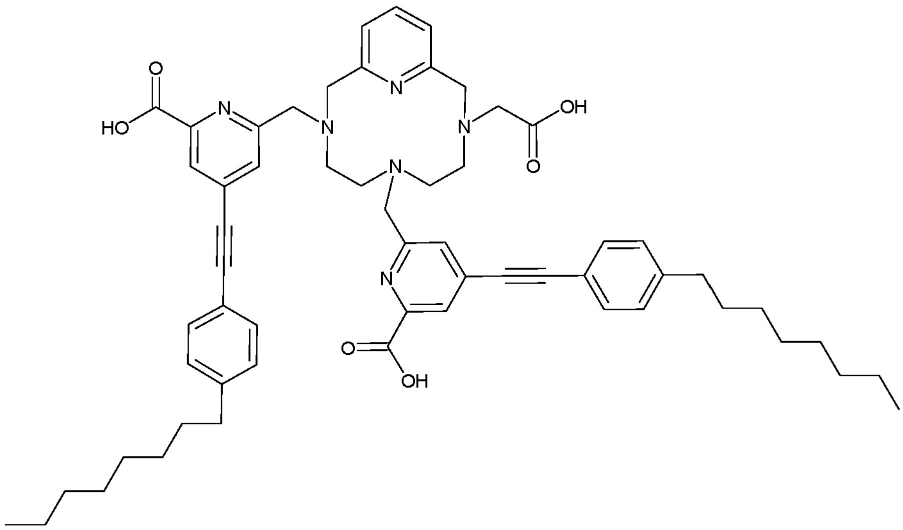

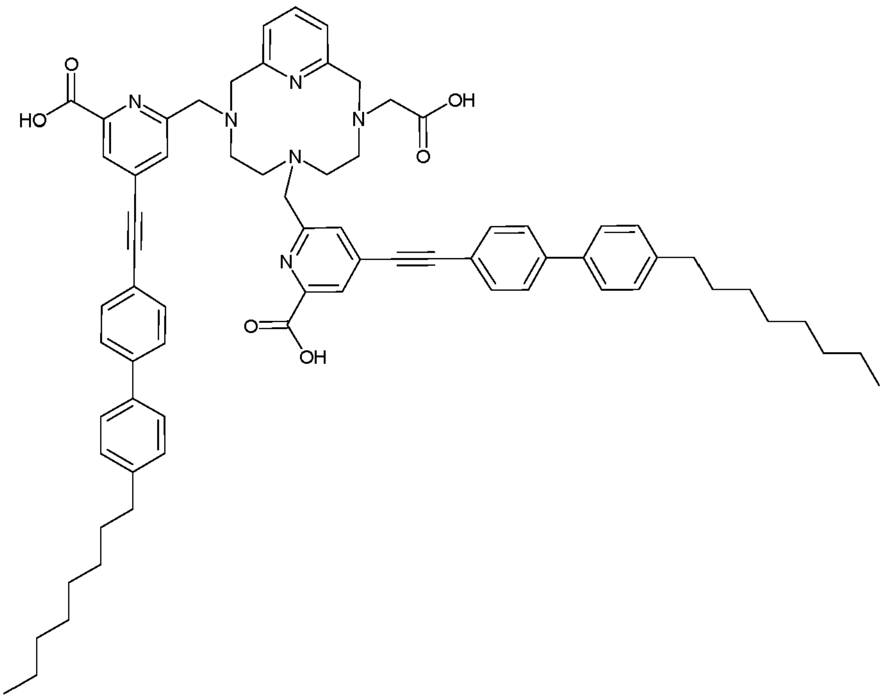

3. Complexe d'un composé de formule (I) selon l'une quelconque des revendications précédentes, ou d'un de ses sels pharmaceutiquement acceptables, avec un élément chimique M ; M étant un métal.

4. Complexe selon la revendication 3 dans lequel M est un radioélément choisi parmi le groupe constitué de $^{44}Sc(III)$, $^{47}Sc(III)$, $^{111}In(III)$, $^{152}Tb(III)$, $^{155}Tb(III)$, $^{149}Tb(III)$, $^{161}Tb(III)$, $^{64}Cu(III)$,$^{61}Cu(III)$, $^{67}Cu(II)$ ,$^{68}Ga(III)$ , $^{90}Y(III)$, $^{153}Sm(III)$, $^{166}Ho(III)$, $^{177}Lu(III)$, $^{52}Mn$ et $^{225}Ac(III)$, de préférence $^{177}Lu(III)$, $^{90}Y(III)$, $^{225}Ac(III)$, $^{67}Cu(II)$, $^{111}In(III)$, $^{152}Tb(III)$, $^{155}Tb(III)$, $^{149}Tb(III)$, $^{161}Tb(III)$ et $^{68}Ga(III)$.

5. Complexe selon la revendication 3 ou 4, choisi parmi le groupe constitué des composés suivants :

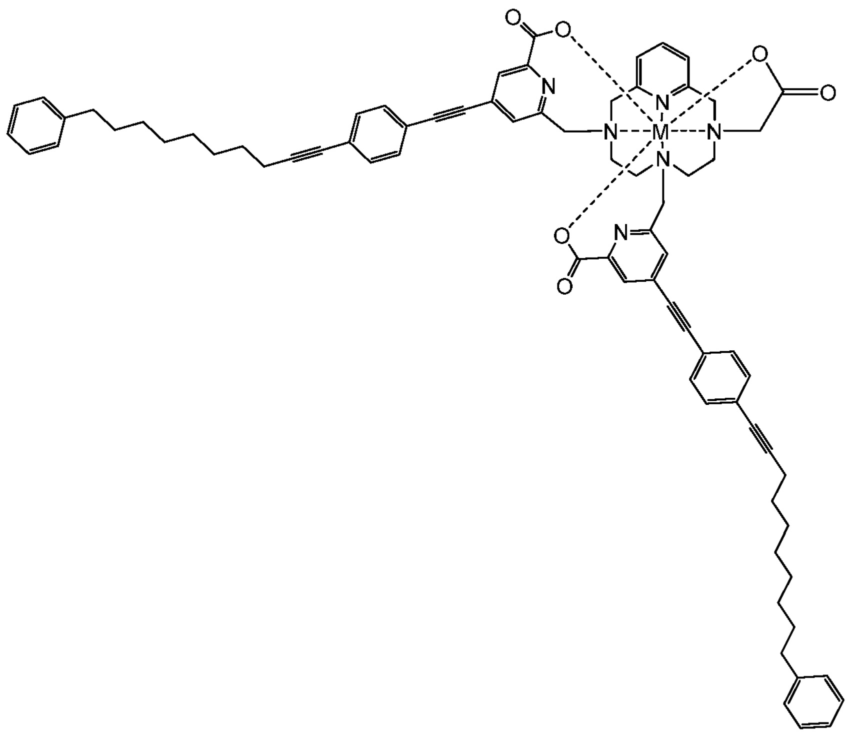

dans lequel M est choisi parmi $^{177}Lu(III)$, $^{90}Y(III)$ et $^{111}In(III)$,

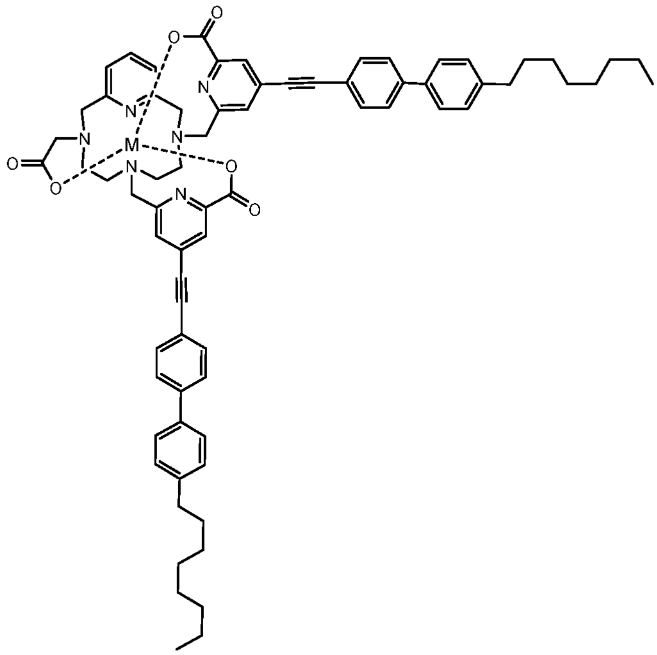

dans lequel M est $^{90}Y(III)$,
et

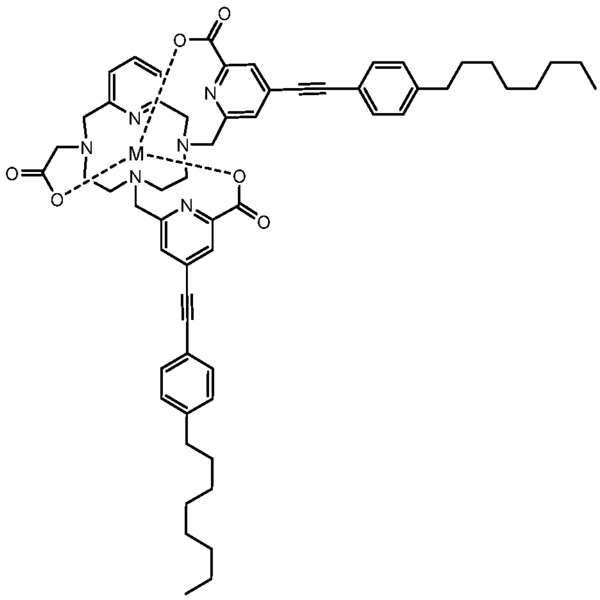

dans lequel M est $^{90}Y(III)$.

6. Composition pharmaceutique comprenant un composé selon l'une quelconque des revendications 1 ou 2, et éventuellement un ou plusieurs excipient(s) pharmaceutiquement acceptable(s), notamment l'excipient est un radioprotecteur.

7. Composition pharmaceutique comprenant un complexe selon l'une quelconque des revendications 3 à 5, et éventuellement un ou plusieurs excipient(s) pharmaceutiquement acceptable(s), notamment l'excipient est un radioprotecteur.

**8.** Composition pharmaceutique selon la revendication 6 ou 7, comprenant en outre une huile iodée, notamment une huile iodée comprenant des esters éthyliques d'acides gras iodés de l'huile d'oeillette.

**9.** Composition pharmaceutique selon la revendication 8, qui comprend le composé suivant :

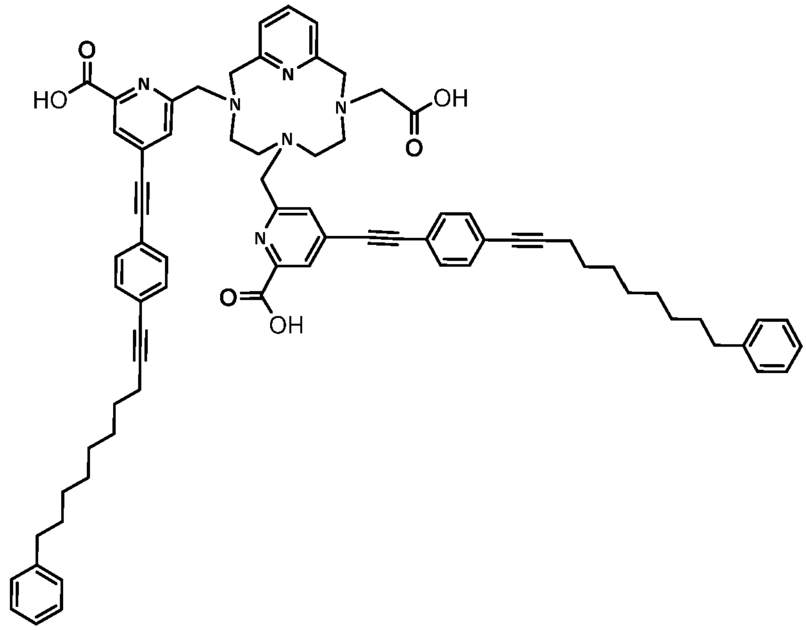

ou bien qui comprend le complexe suivant :

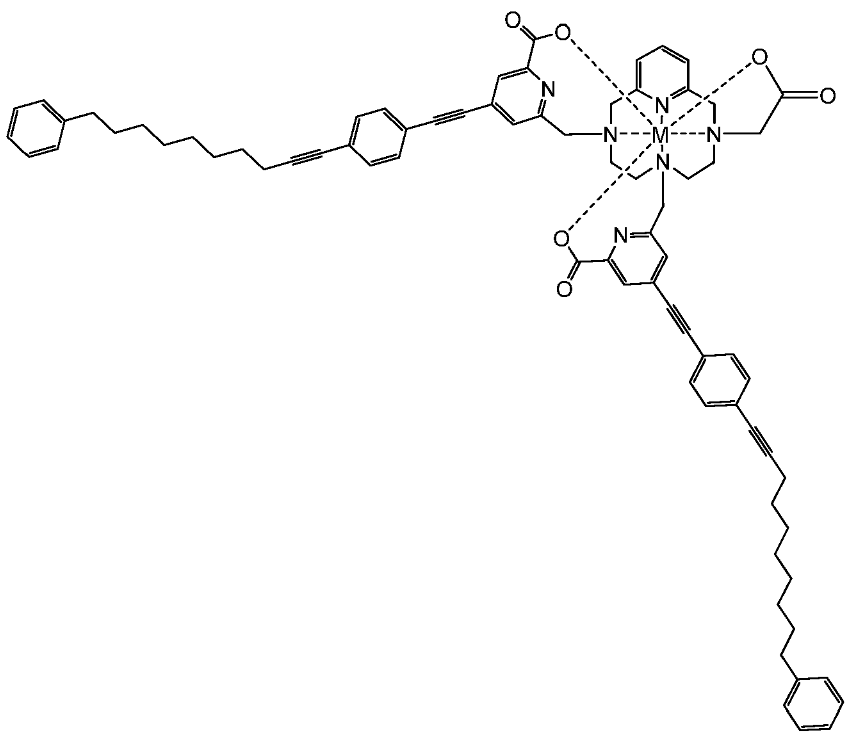

dans lequel M est de préférence choisi parmi $^{177}Lu(III)$ et $^{90}Y(III)$.

**10.** Complexe selon l'une quelconque des revendications 3 à 5, pour son utilisation dans le traitement des cancers, en particulier des cancers du foie.

**11.** Composition pharmaceutique selon l'une des revendications 6 à 9, pour son utilisation dans le traitement des cancers, en particulier des cancers du foie.

**12.** Utilisation d'un complexe selon l'une quelconque des revendications 3 à 5, ou d'une composition pharmaceutique selon l'une des revendications 6 à 9, en imagerie médicale.

**13.** Procédé de préparation des composés de formule générale (I) suivante :

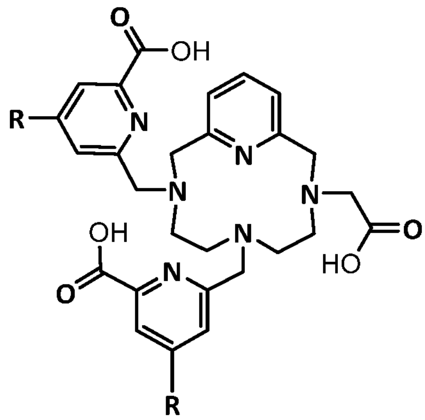

(I)

R étant tel que défini dans l'une des revendications 1 à 3, comprenant une étape de fonctionnalisation d'un composé de formule générale (IX) suivante :

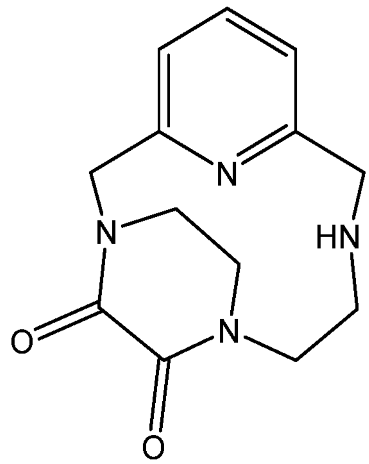

(IX),

pour former un composé de formule générale (X) suivante :

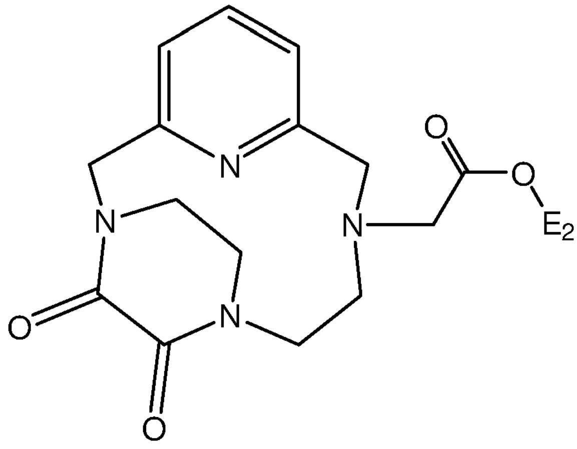

(X),

dans laquelle E2 est un groupe protecteur alkyle en C1-C4 pouvant être choisi dans le groupe constitué de méthyl, éthyle, isopropyle et terbutyle.

**14.** Procédé selon la revendication 13, comprenant en outre :

- une étape de déprotection du composé de formule générale (X), pour obtenir un composé de formule générale (XI) suivante :

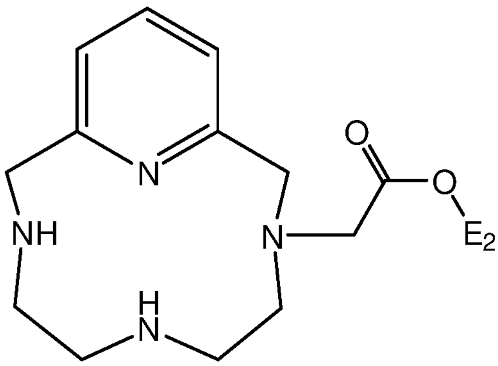

(XI)

et

- une étape de fonctionnalisation du composé de formule générale (XI) pour obtenir un composé de formule générale (I):

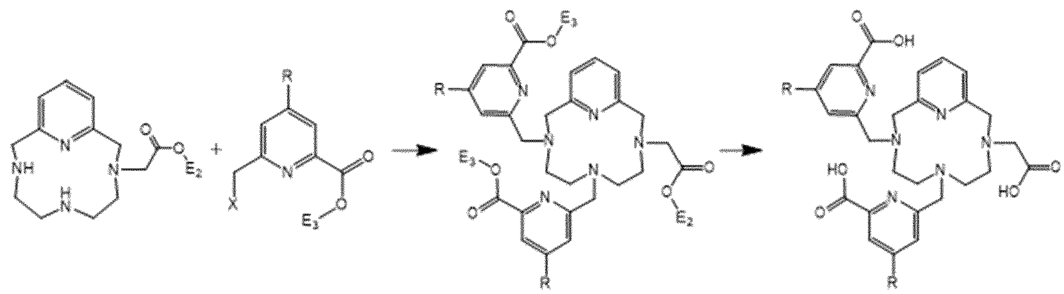

(XI) (XII) (XIII) (I)

E1, E2, E3 étant des groupes protecteurs alkyle en C1-C4 pouvant par exemple être indépendamment choisis dans le groupe constitué de méthyl, éthyle, isopropyle et terbutyle.

## Patentansprüche

**1.** Verbindung der folgenden allgemeinen Formel (I):

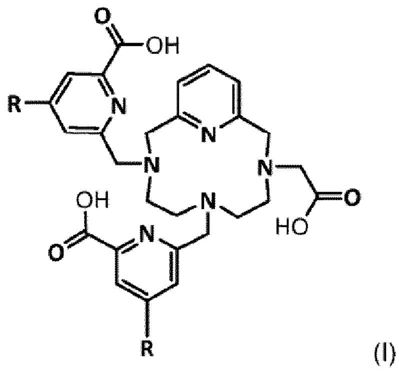

(I)

in der R für eine Gruppe der folgenden Formel (II) steht:

-C≡C-Ph-L1-(CH2)n-L2 (II)

wobei

- L1 für Ph oder -C=C- oder CH2 steht,
- L2 für H oder Ph oder Alkylphenyl steht,
- n zwischen 4 und 12 liegt,

oder ein pharmazeutisch unbedenkliches Salz davon.

**2.** Verbindung der Formel (I) nach Anspruch 1, ausgewählt aus der Gruppe bestehend aus den folgenden Verbindungen:

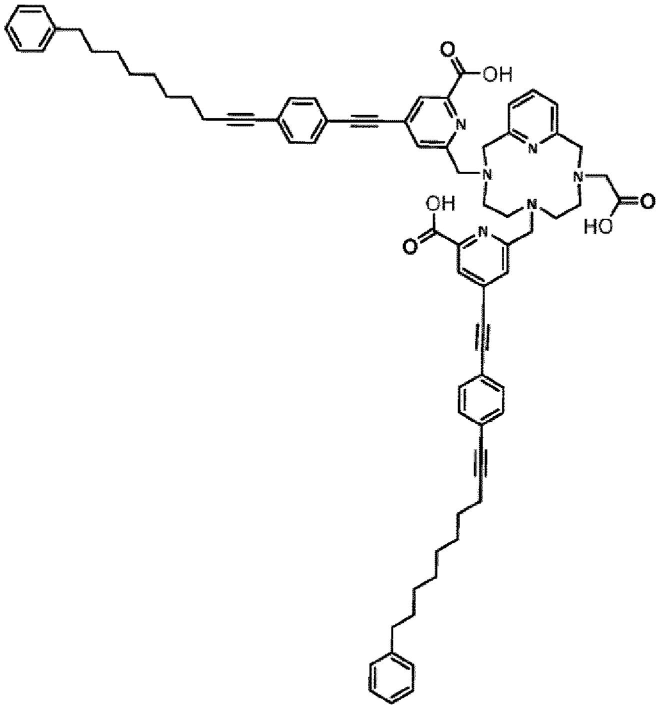

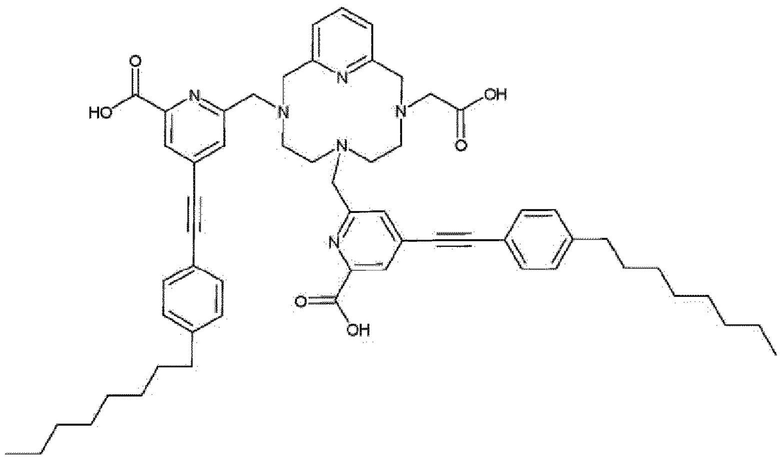

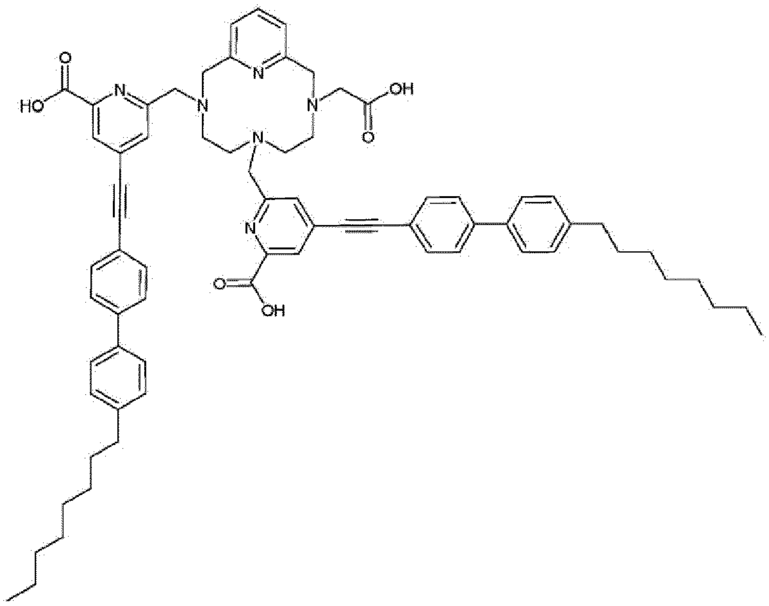

3. Komplex einer Verbindung der Formel (I) nach einem der vorhergehenden Ansprüche oder eines pharmazeutisch unbedenklichen Salzes davon mit einem chemischen Element M; wobei M für ein Metall steht.

4. Komplex nach Anspruch 3, wobei M für ein Radioelement steht, das aus der Gruppe bestehend aus $^{44}$Sc(III), $^{47}$Sc(III), $^{111}$In(III), $^{152}$Tb(III), $^{155}$Tb(III), $^{149}$Tb(III), $^{161}$Tb(III), $^{64}$Cu(III), $^{61}$Cu(III), $^{67}$Cu(II), $^{68}$Ga(III), $^{90}$Y(III), $^{153}$Sm(III), $^{166}$Ho(III), $^{177}$Lu(III), $^{52}$Mn und $^{225}$Ac(III), vorzugsweise $^{177}$Lu(III), $^{90}$Y(III), $^{225}$Ac(III), $^{67}$Cu(II), $^{111}$In(III), $^{152}$Tb(III), $^{155}$Tb(III), $^{149}$Tb(III), $^{161}$Tb(III) und $^{68}$Ga(III) ausgewählt ist.

5. Komplex nach Anspruch 3 oder 4, ausgewählt aus der Gruppe bestehend aus den folgenden Verbindungen:

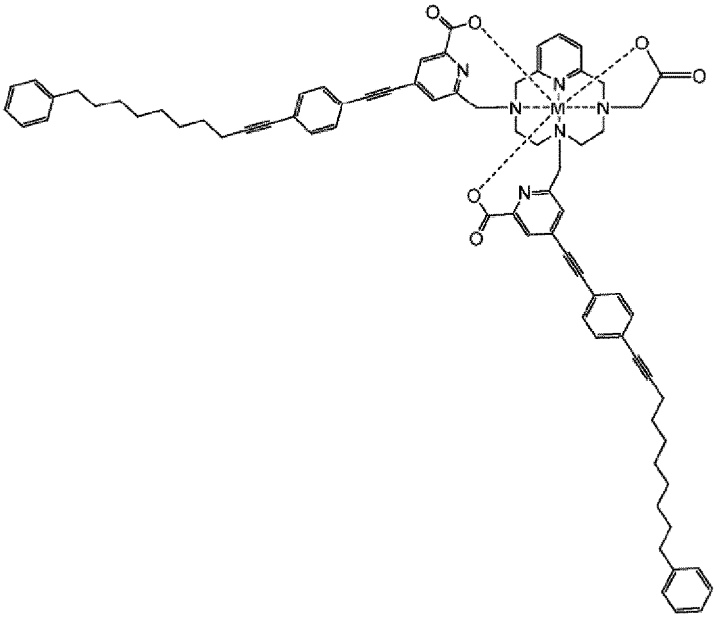

wobei M aus $^{177}Lu(III)$, $^{90}Y(III)$ und $^{111}In(III)$ ausgewählt ist,

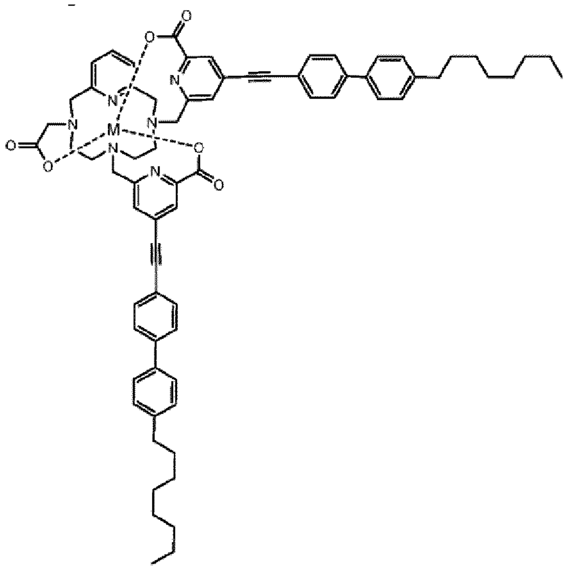

wobei M für $^{90}Y(III)$ steht,
und

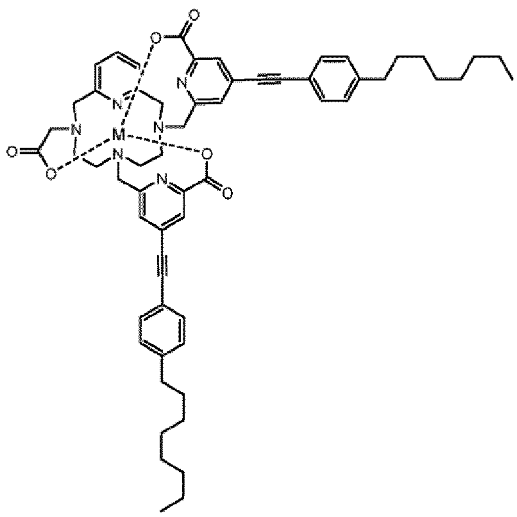

wobei M für $^{90}Y(III)$ steht.

6. Pharmazeutische Zusammensetzung, umfassend eine Verbindung nach einem der Ansprüche 1 oder 2 und gegebenenfalls einen oder mehrere pharmazeutisch unbedenkliche Hilfsstoffe, wobei es sich bei dem Hilfsstoff insbesondere um ein Strahlenschutzmittel handelt.

7. Pharmazeutische Zusammensetzung, umfassend einen Komplex nach einem der Ansprüche 3 bis 5 und gegebenenfalls einen oder mehrere pharmazeutisch unbedenkliche Hilfsstoffe, wobei es sich bei dem Hilfsstoff insbesondere um ein Strahlenschutzmittel handelt.

8. Pharmazeutische Zusammensetzung nach Anspruch 6 oder 7, außerdem umfassend ein iodiertes Öl, insbesondere ein iodiertes Öl, das Ethylester von iodierten Mohnölfettsäureestern umfasst.

9. Pharmazeutische Zusammensetzung nach Anspruch 8, die die folgende Verbindung umfasst:

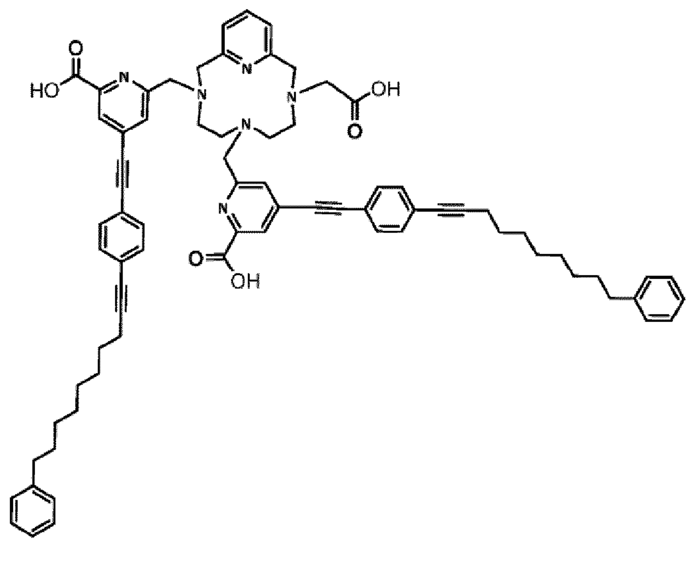

oder die die folgende Verbindung umfasst:

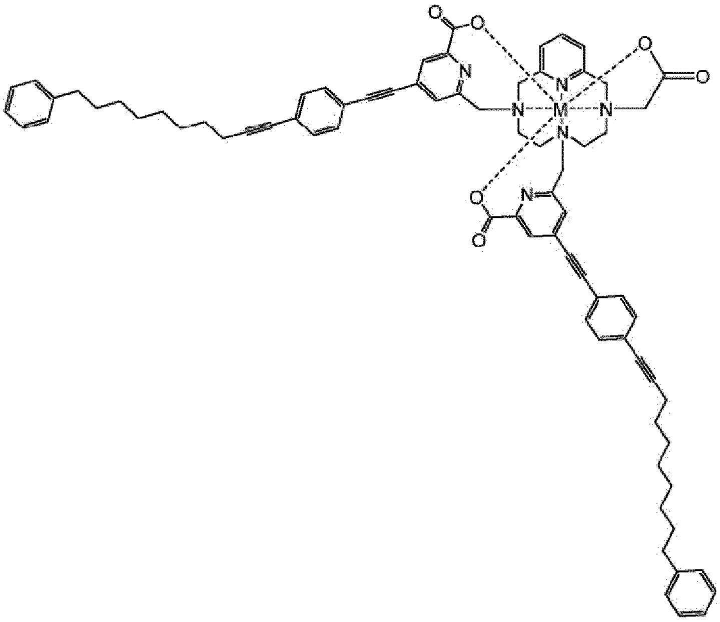

wobei M vorzugsweise aus $^{177}Lu(III)$ und $^{90}Y(III)$ ausgewählt ist.

**10.** Komplex nach einem der Ansprüche 3 bis 5 zur Verwendung bei der Behandlung von Krebserkrankungen, insbesondere Leberkrebserkrankungen.

**11.** Pharmazeutische Zusammensetzung nach einem der Ansprüche 6 bis 9 zur Verwendung bei der Behandlung von Krebserkrankungen, insbesondere Leberkrebserkrankungen.

**12.** Verwendung eines Komplexes nach einem der Ansprüche 3 bis 5 oder einer pharmazeutischen Zusammensetzung nach einem der Ansprüche 6 bis 9 bei der medizinischen Bildgebung.

**13.** Verfahren zur Herstellung der Verbindungen der folgenden allgemeinen Formel (I):

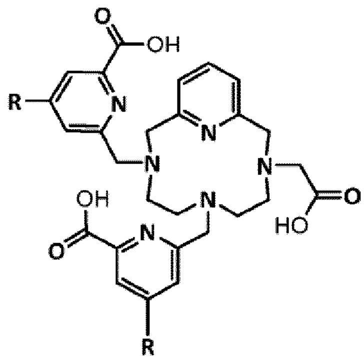

(I)

wobei R wie in einem der Ansprüche 1 bis 3 definiert ist, umfassend einen Schritt der Funktionalisierung einer Verbindung der folgenden allgemeinen Formel (IX) :

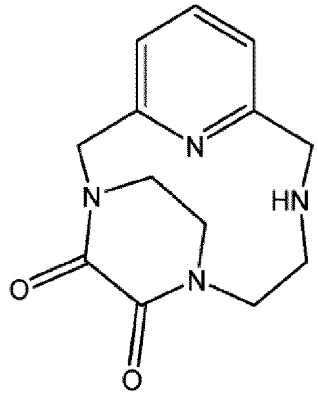

(IX)

zur Bildung einer Verbindung der folgenden allgemeinen Formel (X):

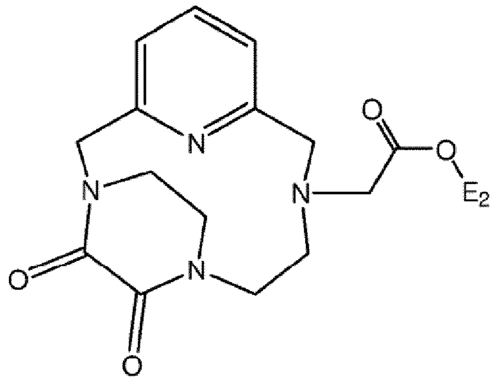

(X),

in der E2 für eine C1-C4-Alkylschutzgruppe steht, die aus der Gruppe bestehend aus Methyl, Ethyl, Isopropyl und tert-Butyl ausgewählt werden kann.

**14.** Verfahren nach Anspruch 13, außerdem umfassend:

- einen Schritt der Entschützung der Verbindung der allgemeinen Formel (X) zum Erhalt einer Verbindung der folgenden allgemeinen Formel (XI)

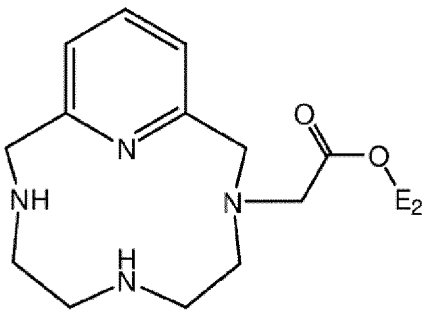

(XI)

und

- einen Schritt der Funktionalisierung der Verbindung der allgemeinen Formel (XI) zum Erhalt einer Verbindung der allgemeinen Formel (I):

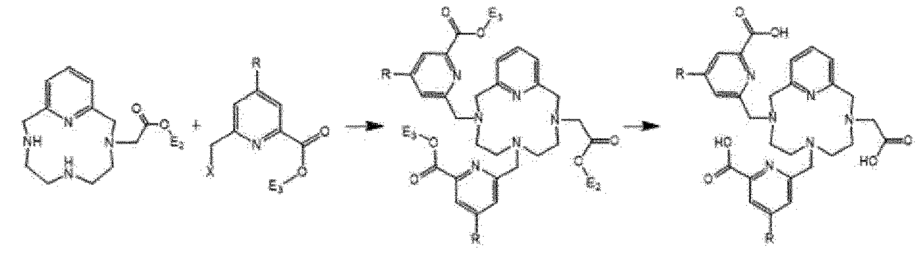

(XI) (XII) (XIII) (I)

wobei E1, E2 und E3 für C1-C4-Alkylschutzgruppen stehen, die beispielsweise unabhängig aus der Gruppe bestehend aus Methyl, Ethyl, Isopropyl und tert-Butyl ausgewählt werden können.

## Claims

**1.** Compound of the following general formula (I):

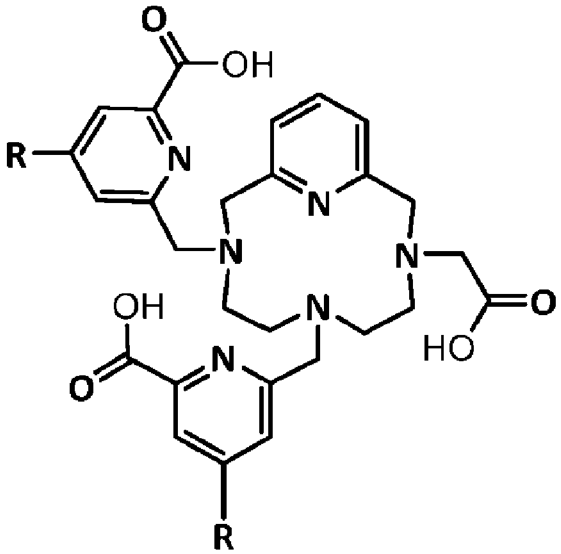

(I)

in which R is a group of the following formula (II):

-C≡C-Ph-L1-(CH2)n-L2 (II)

with

- L1 representing Ph or -C=C- or CH2,
- L2 representing H or Ph or alkylphenyl,
- n being between 4 and 12,

or one of the pharmaceutically acceptable salts thereof.

**2.** Compound of formula (I) according to Claim 1, chosen from the group consisting of the following compounds:

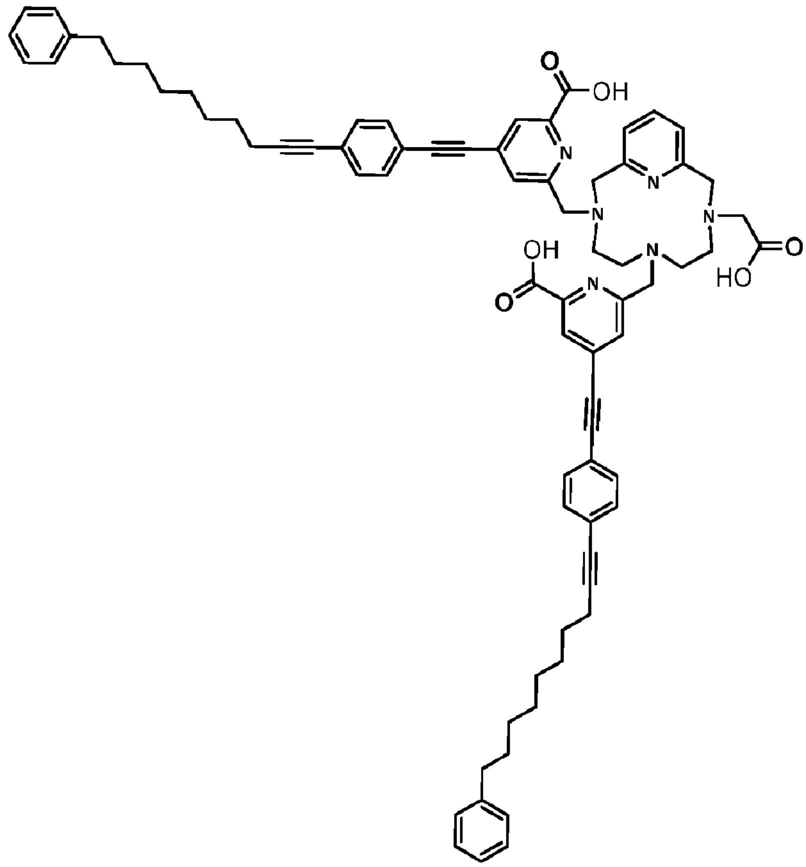

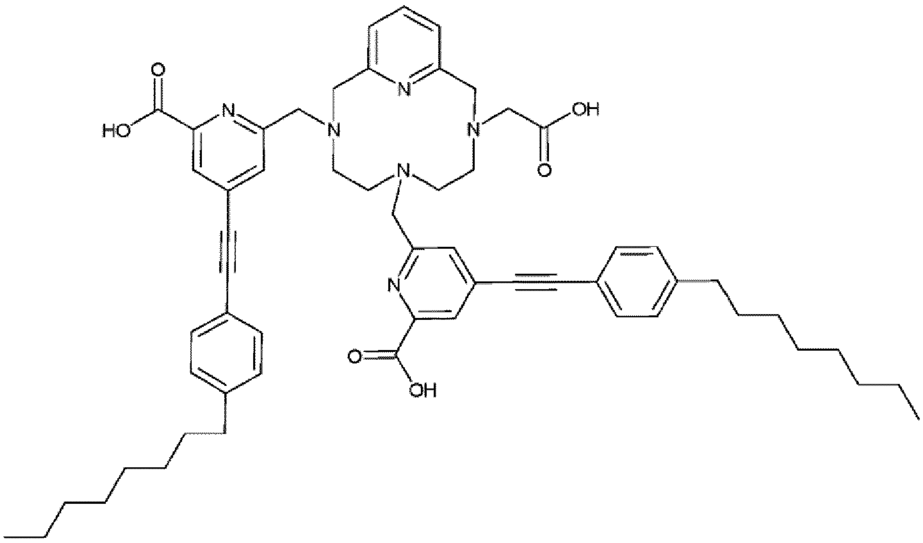

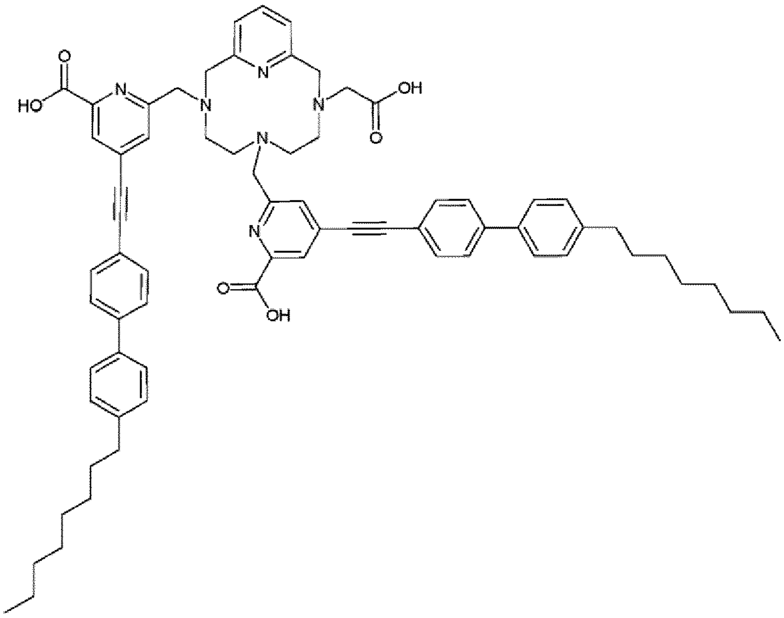

3. Complex of a compound of formula (I) according to either one of the preceding claims, or one of the pharmaceutically acceptable salts thereof, with a chemical element M, M being a metal.

4. Complex according to Claim 3, wherein M is a radioelement chosen from the group consisting of $^{44}$Sc(III), $^{47}$Sc(III), $^{111}$In(III), $^{152}$Tb(III), $^{155}$Tb(III), $^{149}$Tb(III), $^{161}$Tb(III), $^{64}$Cu(III), $^{61}$Cu(III), $^{67}$Cu(II), $^{68}$Ga(III), $^{90}$Y(III), $^{153}$Sm(III), $^{166}$Ho(III), $^{177}$Lu(III), $^{52}$Mn and $^{225}$Ac(III), preferably $^{177}$Lu(III), $^{90}$Y(III), $^{225}$Ac(III), $^{67}$Cu(II), $^{111}$In(III), $^{152}$Tb(III), $^{155}$Tb(III), $^{149}$Tb(III), $^{161}$Tb(III) and $^{68}$Ga(III) .

5. Complex according to Claim 3 or 4, chosen from the group consisting of the following compounds:

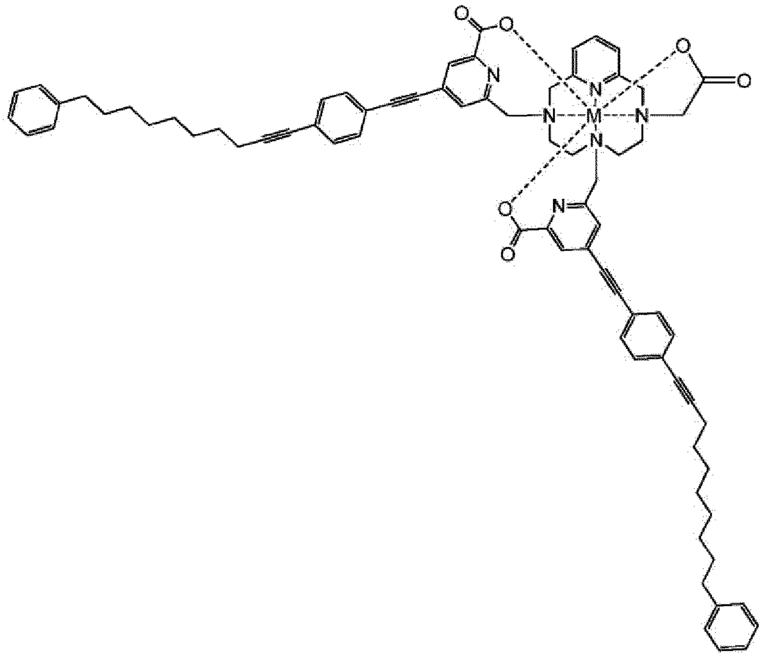

in which M is chosen from $^{177}$Lu(III), $^{90}$Y(III) and $^{111}$In(III),

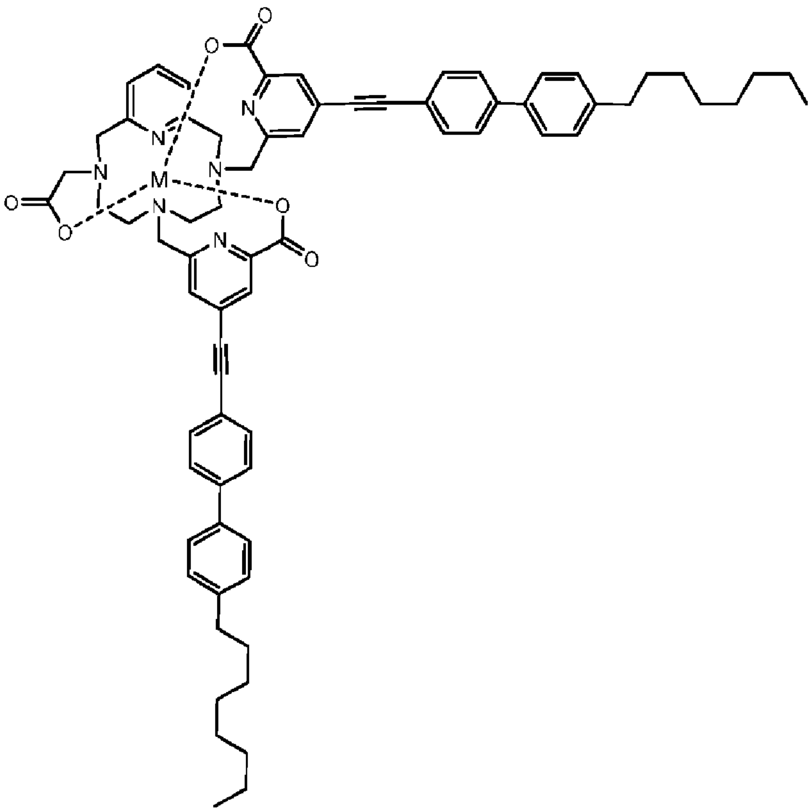

in which M is $^{90}Y(III)$,
and

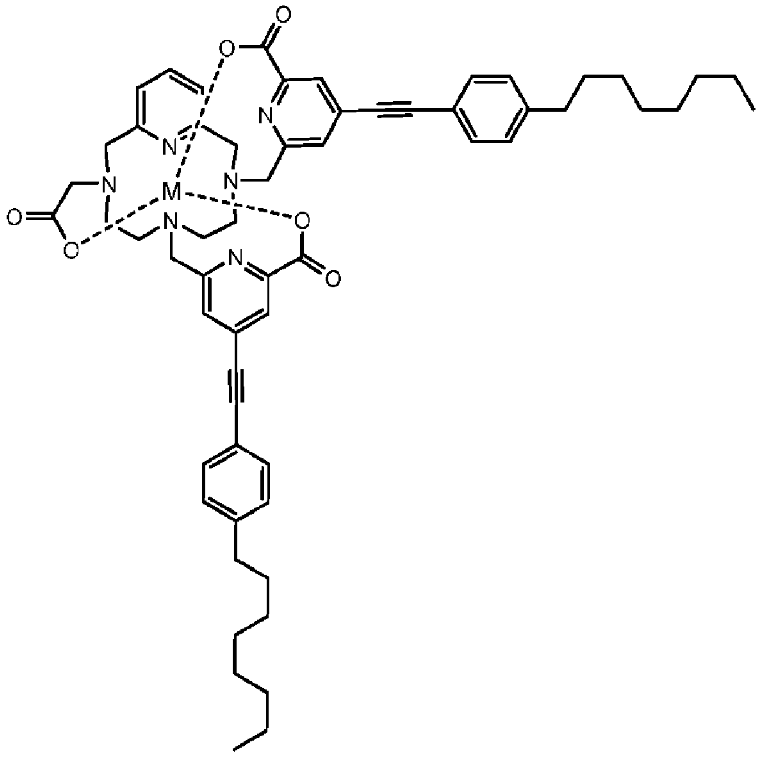

in which M is $^{90}Y(III)$.

6. Pharmaceutical composition comprising a compound according to either one of Claims 1 and 2, and optionally one or more pharmaceutically acceptable excipients; in particular the excipient is a radioprotector.

7. Pharmaceutical composition comprising a complex according to any one of Claims 3 to 5, and optionally one or more pharmaceutically acceptable excipients; in particular the excipient is a radioprotector.

8. Pharmaceutical composition according to Claim 6 or 7, further comprising an iodized oil, in particular an iodized oil comprising iodized ethyl esters of fatty acids of poppyseed oil.

9. Pharmaceutical composition according to Claim 8, comprising the following compound:

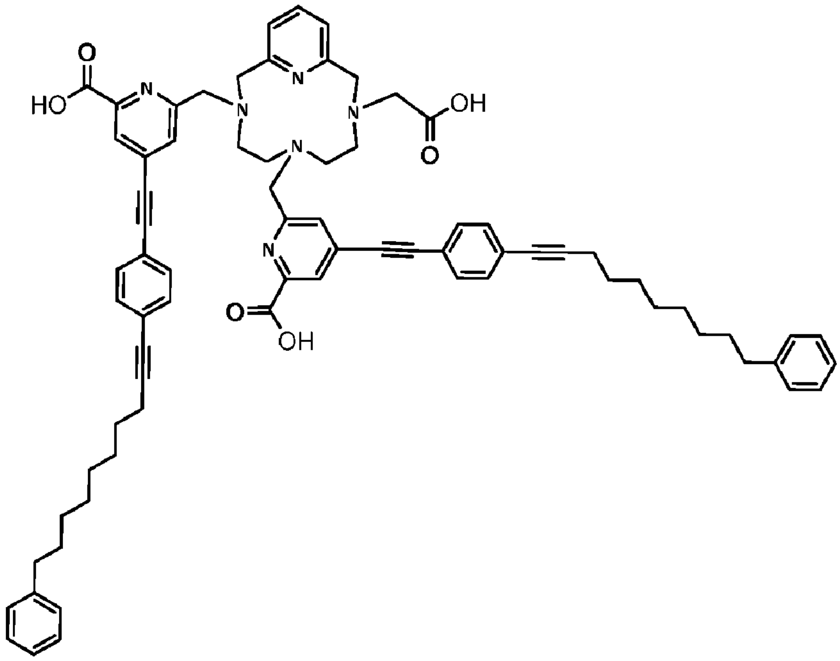

or else comprising the following complex:

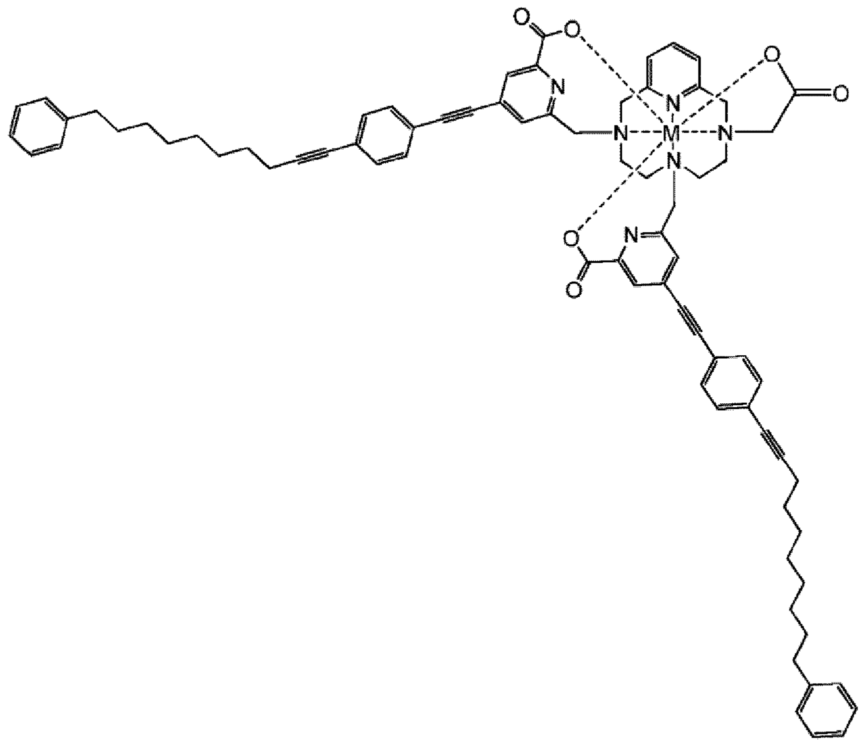

in which M is preferably chosen from $^{177}Lu(III)$ and $^{90}Y(III)$.

**10.** Complex according to any one of Claims 3 to 5, for the use thereof in the treatment of cancers, in particular cancers of the liver.

**11.** Pharmaceutical composition according to one of Claims 6 to 9, for the use thereof in the treatment of cancers, in particular cancers of the liver.

**12.** Use of a complex according to any one of Claims 3 to 5, or of a pharmaceutical composition according to one of Claims 6 to 9, in medical imaging.

**13.** Process for preparing compounds of the following general formula (I):

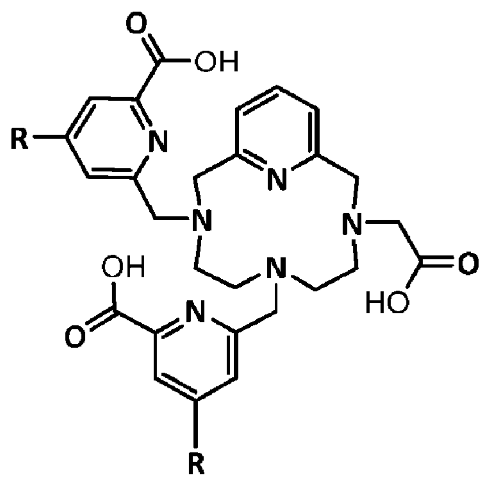

(I)

R being as defined in one of Claims 1 to 3, comprising a step of functionalizing a compound of the following general formula (IX):

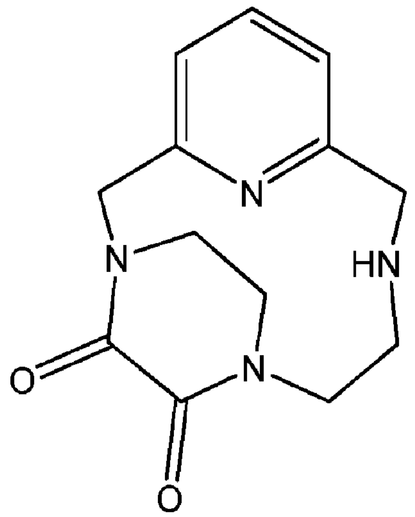

(IX),

to form a compound of the following general formula (X):

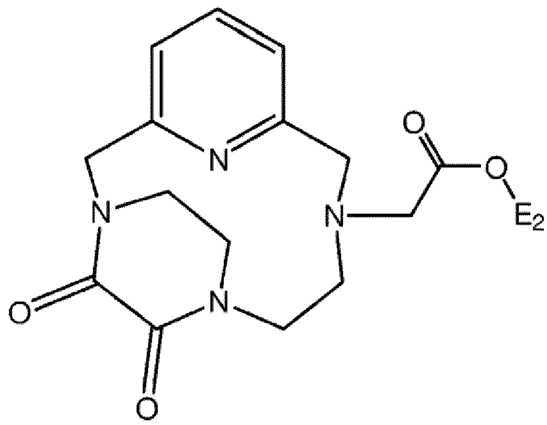

(X),

in which E2 is a C1-C4 alkyl protective group that can be chosen from the group consisting of methyl, ethyl, isopropyl and tert-butyl.

**14.** Process according to Claim 13, further comprising:

- a step of deprotecting the compound of general formula (X) in order to obtain a compound of the following general formula (XI):

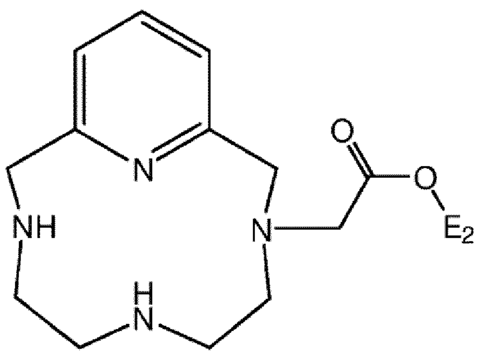

(XI)

and

- a step of functionalizing the compound of general formula (XI) in order to obtain a compound of general formula

(I):

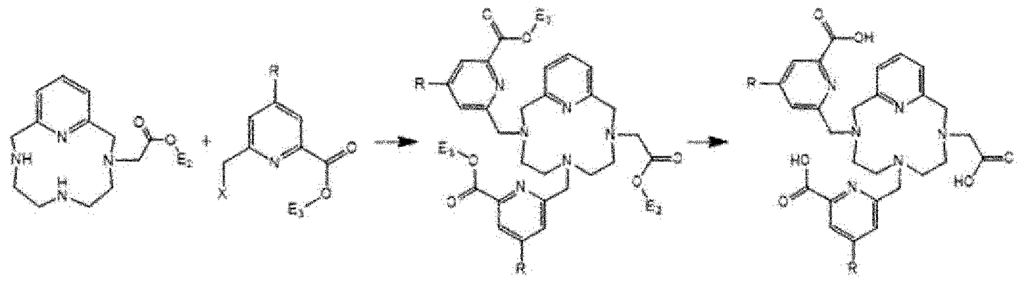

(XI) (XII) (XIII) (I)

E1, E2 and E3 being C1-C4 alkyl protective groups that can for example be independently chosen from the group consisting of methyl, ethyl, isopropyl and tert-butyl.

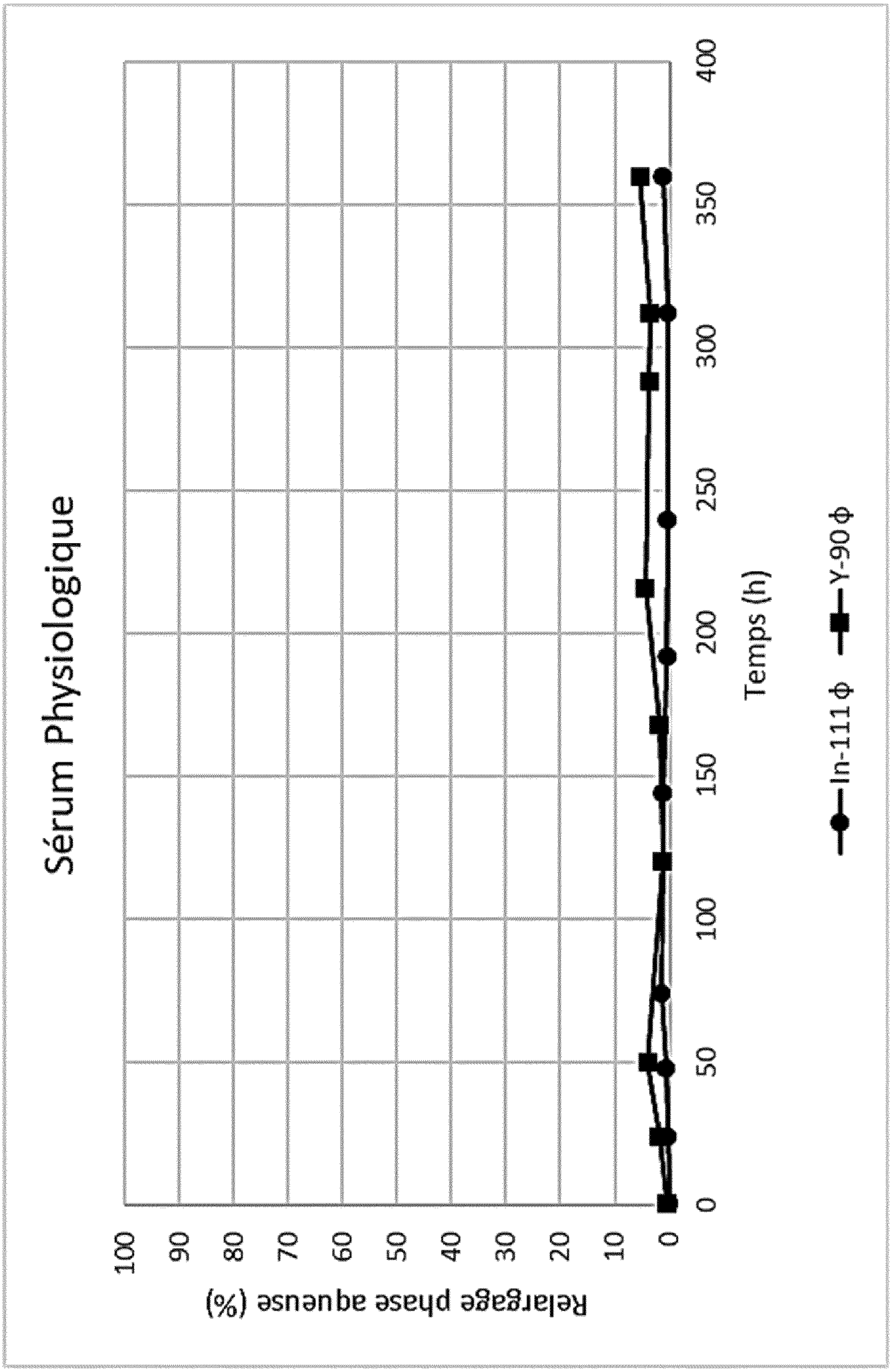
Sérum Physiologique
Relargage phase aqueuse (%)
100
90
80
70
60
50
40
30
20
10
0
0
50
100
150
200
250
300
350
400
Temps (h)
In-111 Φ
Y-90 Φ

FIG.1

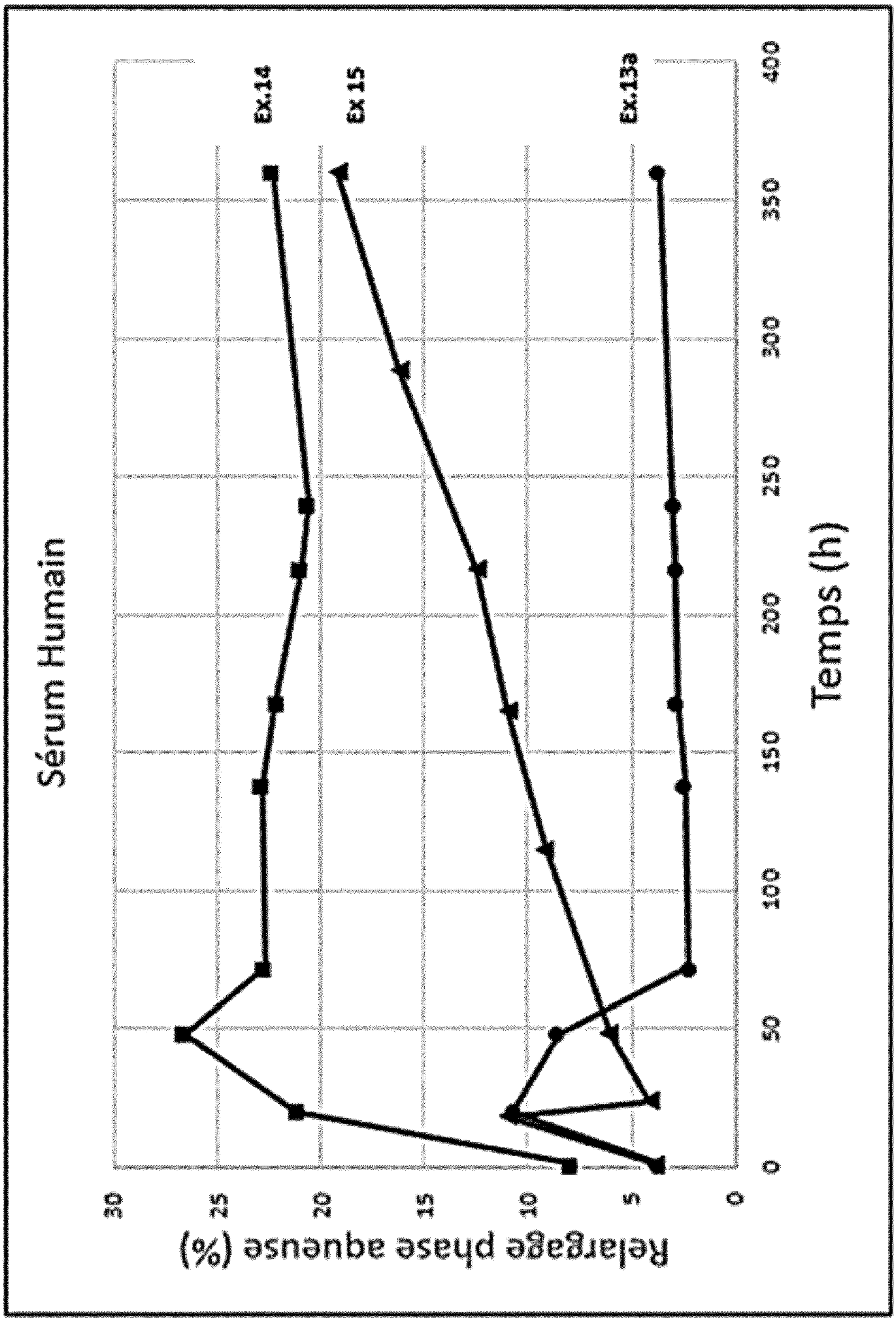
Sérum Humain
Relargage phase aqueuse (%)
Temps (h)
Ex.14
Ex 15
Ex.13a
0
5
10
15
20
25
30
0
50
100
150
200
250
300
350
400

FIG.2

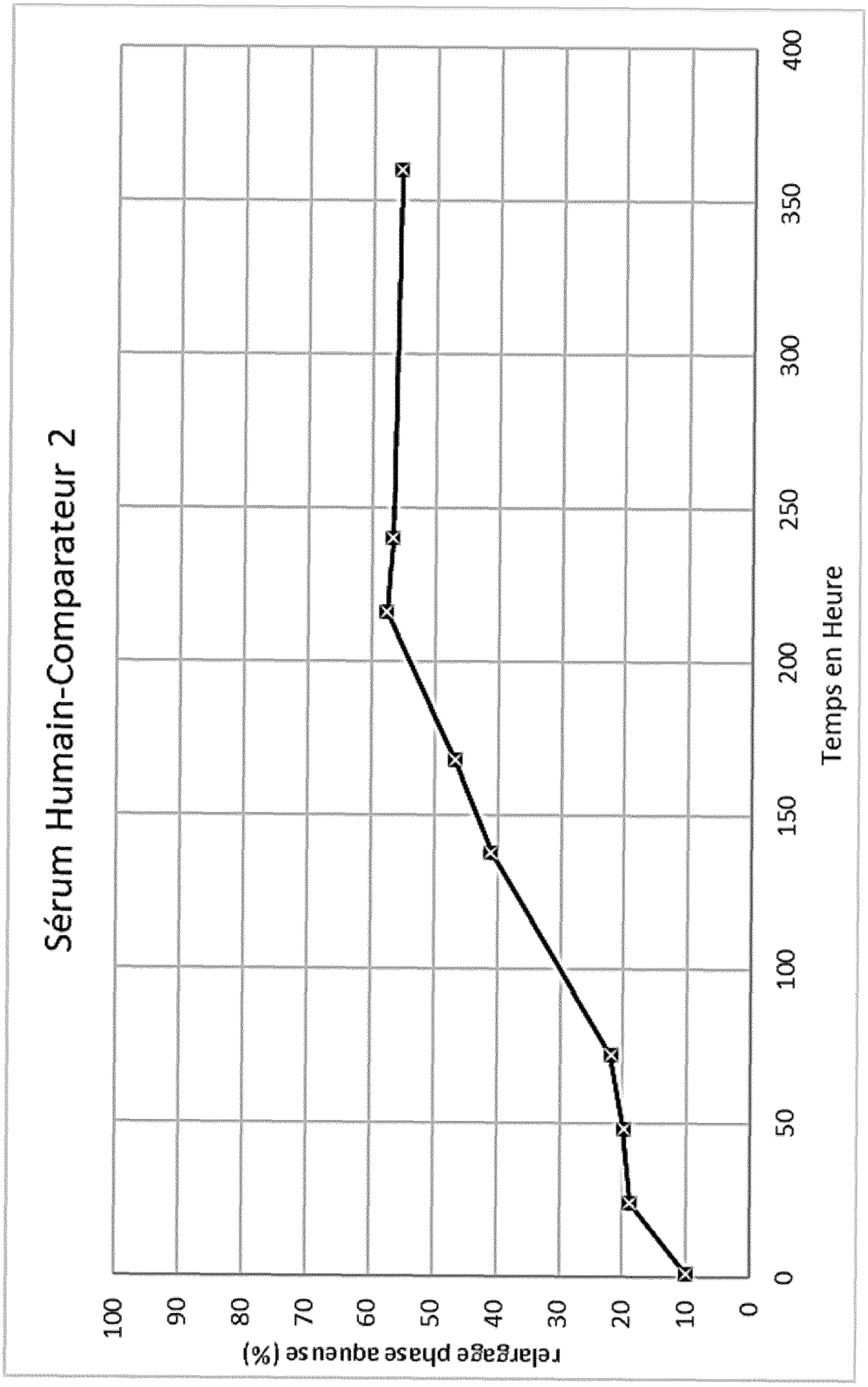
Sérum Humain-Comparateur 2
relargage phase aqueuse (%)
0
10
20
30
40
50
60
70
80
90
100
Temps en Heure
0
50
100
150
200
250
300
350
400

FIG.3

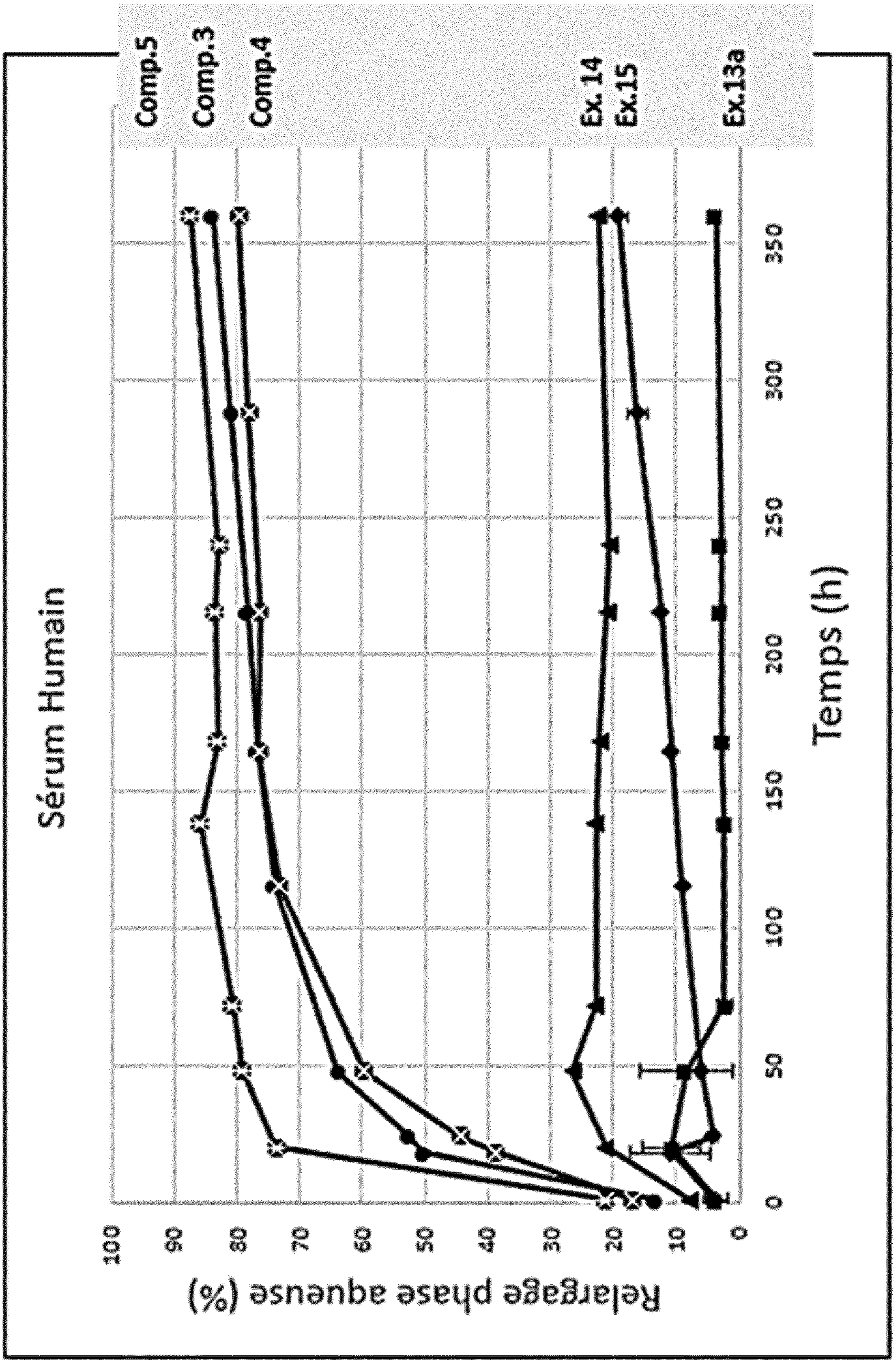
Sérum Humain
Relargage phase aqueuse (%)
Temps (h)
0
10
20
30
40
50
60
70
80
90
100
0
50
100
150
200
250
300
350
Comp.5
Comp.3
Comp.4
Ex. 14
Ex.15
Ex.13a

FIG.4

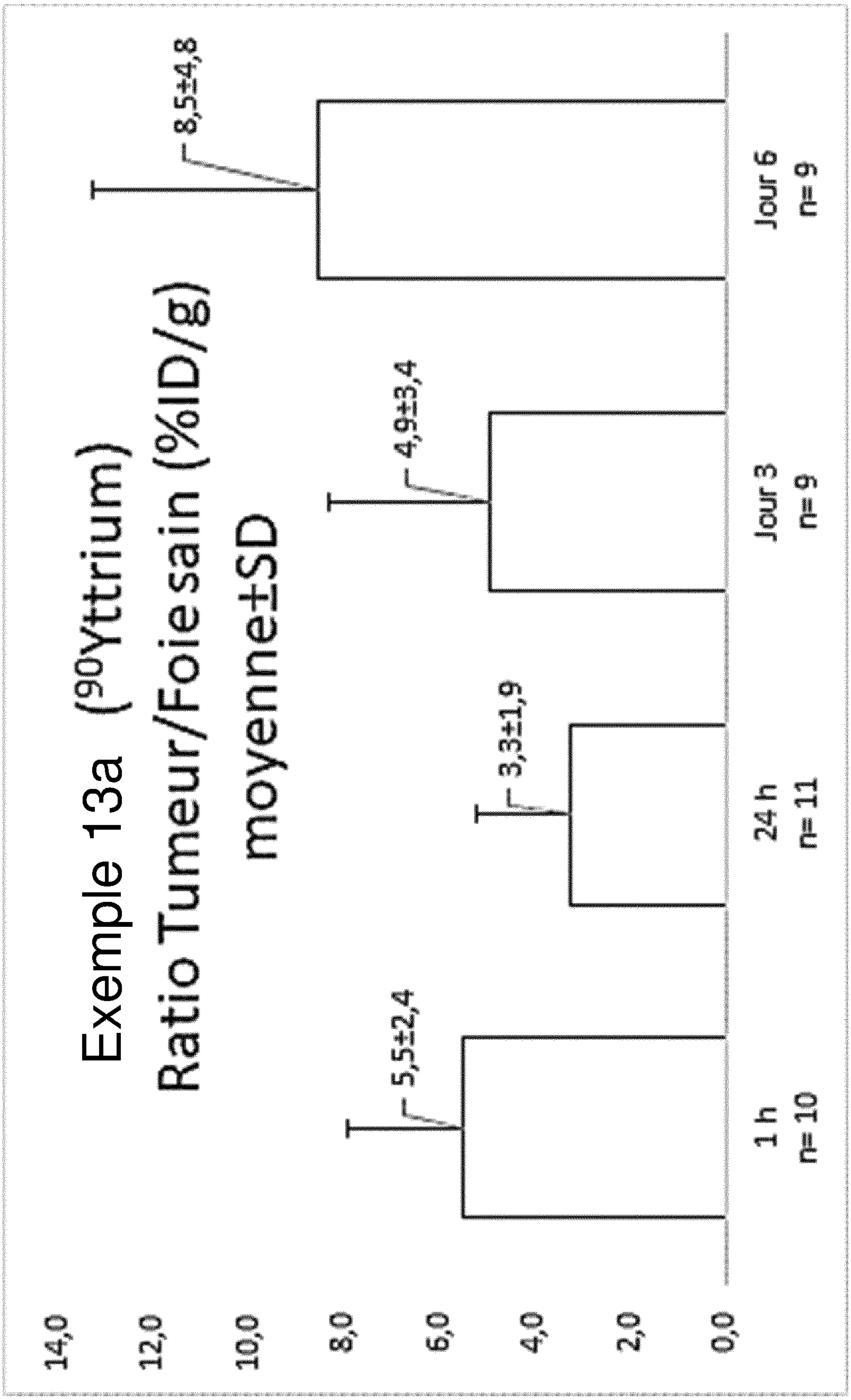
Exemple 13a ($^{90}$Yttrium)
Ratio Tumeur/Foie sain (%ID/g)
moyenne±SD
14,0
12,0
10,0
8,0
6,0
4,0
2,0
0,0
5,5±2,4
3,3±1,9
4,9±3,4
8,5±4,8
1 h
n= 10
24 h
n= 11
Jour 3
n= 9
Jour 6
n= 9

FIG.5

**Biodistribution: Exemple 13a ($90_Y$) vs Exemple 13b ($111_{In}$)**
**% Dose injectée / organe - Moyenne**

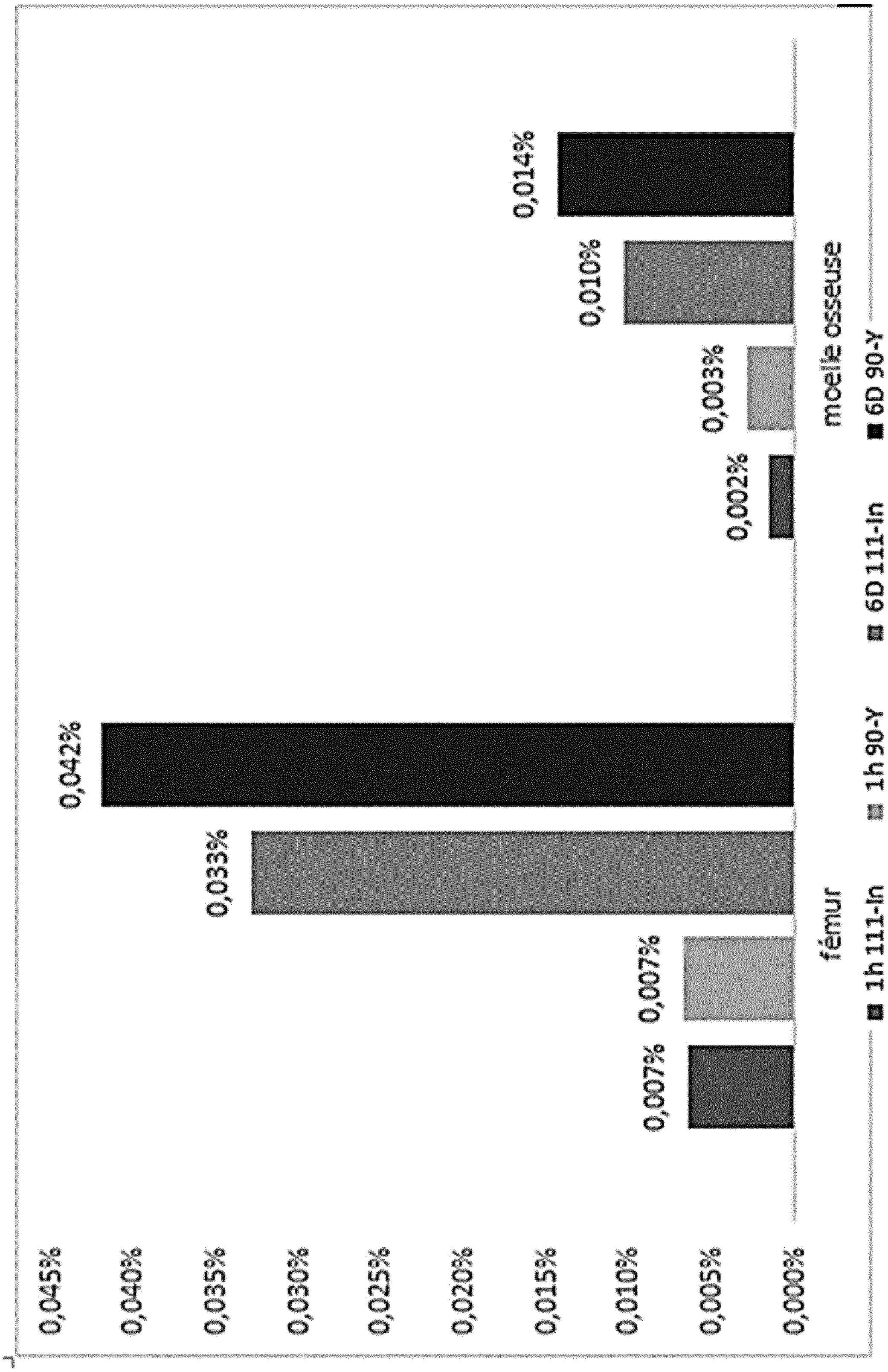


FIG.6

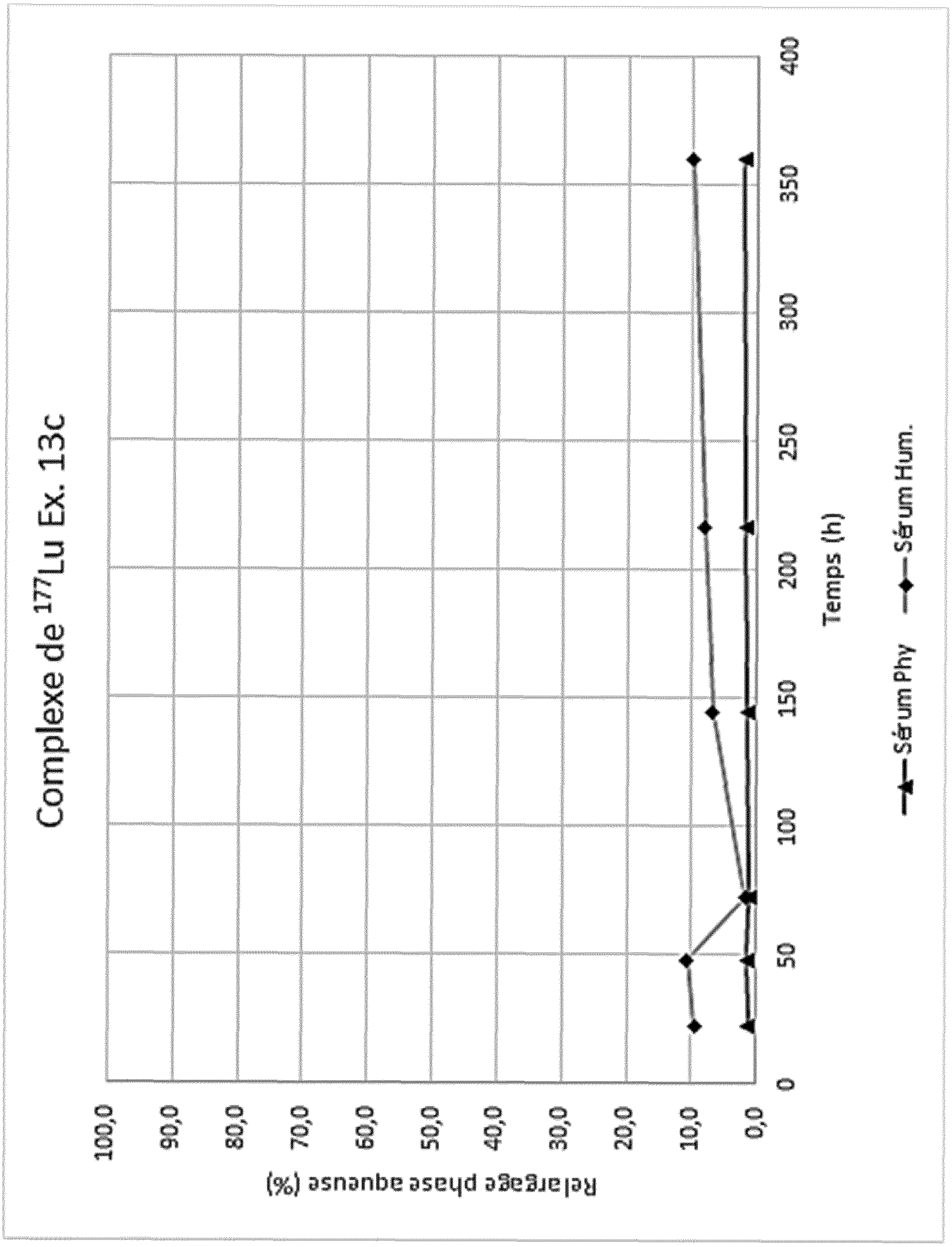
Complexe de 177Lu Ex. 13c
Relargage phase aqueuse (%)
100,0
90,0
80,0
70,0
60,0
50,0
40,0
30,0
20,0
10,0
0,0
0
50
100
150
200
250
300
350
400
Temps (h)
Sérum Phy
Sérum Hum.

FIG.7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2017109217 A


### Littérature non-brevet citée dans la description


- **LE FUR et al.** *Inorganic Chemistry,* 2018, vol. 57 (4), 2051-2063
- **WOLFF et al.** *Medicine,* 2001, vol. 80, 20-36
- **BERGE et al.** *J. Pharm. Sd,* 1977, vol. 66, 1
- **LOIC BELLOUARD.** *J CHEM S Perkin,* 1999, vol. 1 (23), 3499-3505
- **SONOGASHIRA.** *Comprehensive Chirality,* 2012, vol. 4, 18-32
- *Inorganic Chemistry,* vol. 36 (14), 2992-3000
- **GARIN E ; DENIZOT B ; ROUX J et al.** Description and technical pitfalls of a hepatoma model and of intra-arterial injection of radiolabeled Lipiodol in the rat. *Lab Animals,* 2005, vol. 39, 314-320